# EUROPÄISCHE PATENTSCHRIFT

(11) **EP 3 524 341 B1**
(45) Veröffentlichungstag und Bekanntmachung des Hinweises auf die Patenterteilung: **22.07.2020**
(21) Anmeldenummer: 18211265.6
(22) Anmeldetag: 10.12.2018
(51) Int. Cl.: B01F 13/00, B01F 15/00, A61B 17/88

(54) **KNOCHENZEMENTAPPLIKATOR MIT VERSCHLIESSBARER BEGASUNGSÖFFNUNG**
BONE CEMENT APPLICATOR WITH A SEALABLE FUMIGATION OPENING
APPLICATEUR DE CIMENT OSSEUX POURVU D'OUVERTURE DE CHARGEMENT DE GAZ POUVANT ÊTRE FERMÉE

(30) Priorität: 18.01.2018 DE 102018101041
(43) Veröffentlichungstag der Anmeldung: 14.08.2019
(73) Patentinhaber: Heraeus Medical GmbH, 61273 Wehrheim (DE)
(72) Erfinder: Kluge, Thomas, 56179 Vallendar (DE); Vogt, Sebastian, 99092 Erfurt (DE)
(74) Vertreter: Heraeus IP

(56) Entgegenhaltungen:
- EP-A1- 2 974 681
- DE-A1- 4 030 832

## Beschreibung

Die Erfindung betrifft eine Vorrichtung zum Herstellen eines Knochenzementteigs aus einer Monomerflüssigkeit und einem Zementpulver als Ausgangskomponenten des Knochenzementteigs und zum Austragen des gemischten Knochenzementteigs.

Die Erfindung betrifft auch ein Verfahren zur Herstellung eines Knochenzementteigs, insbesondere eines niedrigviskosen pastenförmigen Polymethylmethacrylat-Knochenzementteigs für die Augmentation von Wirbelkörpern.

Gegenstand der Erfindung ist insbesondere eine Vorrichtung zum separaten Lagern des Zementpulvers und der Monomerflüssigkeit von Polymethylmethacrylat-Knochenzement (PMMA-Knochenzement), zum anschließenden Vermischen des Zementpulvers mit der Monomerflüssigkeit zur Bildung eines Knochenzementteigs und zum Austragen des gemischten Knochenzementteigs. Der mit der Vorrichtung hergestellte dünnflüssige Knochenzementteig ist für die Augmentation von frakturierten Wirbelkörpern und zur Befüllung von Pedikelschrauben beziehungsweise für die Spondylodese bestimmt. Es handelt sich bei der erfindungsgemäßen Vorrichtung bevorzugt um ein Full-Prepacked-Zementiersystem.

Polymethylmethacrylat-(PMMA)-Knochenzemente gehen auf die grundlegenden Arbeiten von Sir Charnley zurück (Charnley, J.: Anchorage of the femoral head prosthesis of the shaft of the femur. J. Bone Joint Surg. 42 (1960) 28-30). Konventionelle PMMA-Knochenzemente bestehen aus einer flüssigen Monomerkomponente und einer Pulverkomponente. Die Monomerkomponente enthält im Allgemeinen das Monomer Methylmethacrylat und einen darin gelösten Aktivator (N,N-Dimethyl-p-toluidin). Die Pulverkomponente, auch als Zementpulver oder Knochenzementpulver bezeichnet, weist ein oder mehrere Polymere auf, die auf Basis von Methylmethacrylat und Comonomeren, wie Styren, Methylacrylat oder ähnlichen Monomeren durch Polymerisation, vorzugsweise Suspensionspolymerisation, hergestellt sind, einen Röntgenopaker und den Initiator Dibenzoylperoxid auf. Beim Vermischen der Pulverkomponente mit der Monomerkomponente entsteht durch Quellung der Polymere der Pulverkomponente im Methylmethacrylat ein plastisch verformbarer Teig, der eigentliche Knochenzement beziehungsweise Knochenzementteig. Beim Vermischen der Pulverkomponente mit der Monomerkomponente reagiert der Aktivator N,N-Dimethyl-p-toluidin mit Dibenzoylperoxid unter Bildung von Radikalen. Die gebildeten Radikale initiieren die radikalische Polymerisation des Methylmethacrylats. Mit fortschreitender Polymerisation des Methylmethacrylats erhöht sich die Viskosität des Knochenzementteigs, bis dieser erstarrt.

PMMA-Knochenzemente können in geeigneten Mischbechern mit Hilfe von Spateln durch Vermischen des Zementpulvers mit der Monomerflüssigkeit vermischt werden.

Weiterhin ist es Stand der Technik, während einer Operation (OP) Polymethylmethacrylat-Knochenzementpulver mit Monomerflüssigkeit in Kartuschensysteme zu füllen und die beiden Ausgangskomponenten durch manuelle Betätigung von Mischvorrichtungen, wie axial beweglichen Mischstäben oder rotierenden Mischern, zu vermischen und anschließend den gebildeten Polymethylmethacrylat-Knochenzementteig aus den Kartuschen herauszupressen.

Eine Weiterentwicklung in der Zementiertechnik stellen Zementiersysteme dar, in denen sowohl das Zementpulver als auch die Monomerflüssigkeit bereits in separaten Kompartimenten der Mischvorrichtungen verpackt sind und die erst unmittelbar vor der Zementapplikation im Zementiersystem miteinander vermischt werden. Solche geschlossenen Full-Prepacked-Mischvorrichtungen wurden mit der EP 0 692 229 A1, der DE 10 2009 031 178 B3, der US 5 997 544 A, der US 6 709 149 B1, der WO 00/35506 A1, der EP 0 796 653 A2 und der US 5 588 745 A vorgeschlagen.

Das Patent DE 10 2009 031 178 B3 offenbart eine Lager- und Mischvorrichtung als Full-Prepacked-Zementiersystem, in dem die zur Herstellung des Knochenzementteigs notwendigen Ausgangskomponenten bereits in der Lager- und Mischvorrichtung gelagert sind und in dieser zusammengeführt und gemischt werden können.

In der WO 00/035506 A1 wird eine Vorrichtung vorgeschlagen, bei der Polymethylmethacrylat-Zementpulver in einer Kartusche gelagert wird, wobei das Zementpulver das gesamte Volumen der Kartusche ausfüllt und die Zwischenräume zwischen den Partikeln des Zementpulvers ein solches Volumen hat, das dem Volumen der Monomerflüssigkeit entspricht, das zur Herstellung von Knochenzementteig mit dem in der Kartusche gelagerten Zementpulver notwendig ist.

In den Patenten DE 40 30 832 C2 und US 5 435 645 wird ein Full-Prepacked-Zementiersystem beschrieben, bei dem Monomerflüssigkeit nach Öffnung einer Ampulle in einen Pulverbehälter einer Kartusche enthaltend ein Zementpulver gezogen wird. Die Vermischung des Zementpulvers mit der Monomerflüssigkeit erfolgt durch Schütteln der Kartusche. Das Öffnen der Ampulle wird durch axiales Einschieben eines von außen zugänglichen Kolbens in die Kartusche bewirkt. Der Kolben schiebt die Ampulle gegen eine Halterung und bricht dadurch einen Ampullenkopf, der in der Halterung gelagert ist, von der Ampulle ab, um die Monomerflüssigkeit aus der Ampulle freizusetzen.

Ein ähnliches Mischsystem wird in dem Patent EP 1 883 379 B1 offenbart, bei dem die Mischung der Zementkomponenten ebenfalls durch Schütteln der Kartusche bewirkt wird. Die Austragsöffnung der Kartusche ist mit einer Membran verschlossen.

Im Patent EP 1 912 597 B1 wird ein Mischsystem vorgeschlagen, bei dem ein ringförmiges, entlang einer Führung bewegliches Mischelement zum Vermischen von Zementpulver mit der Monomerflüssigkeit eingesetzt wird. Das Mischelement ist dabei koaxial zu einer Führung angeordnet. Ein zylindrisches Element schiebt eine gelagerte Ampulle enthaltend eine Monomerflüssigkeit gegen einen Dorn und bricht dadurch die Ampulle.

Die Patentanmeldung EP 2 974 681 A1 beschreibt ein Vakuummischsystem zum Mischen von Polymethylmethacrylat-Knochenzement mit einem Austragskolben, wobei der Austragskolben eine gasdurchlässige und für Partikel undurchlässige Durchführung aufweist um eine Sterilisation mit Ethylenoxid durchzuführen.

Diese Knochenzement-Misch-Systeme sind komplex und dadurch kostenaufwendig im Aufbau. Gleichzeitig kann der Innenraum mit dem Zementpulver nur im zumindest teilweise zerlegten Zustand mit einem sterilisierenden Gas sterilisiert werden. Hierdurch ist nach dem Sterilisieren des Inhalts der Vorrichtungen ein weiterer Montageschritt notwendig, der der bereits erfolgten Sterilisation entgegenwirken kann und der die Fertigung der Vorrichtungen weiter erschwert und aufwendiger gestaltet.

Die Aufgabe der vorliegenden Erfindung besteht also darin, die Nachteile des Stands der Technik zu überwinden. Insbesondere besteht die Aufgabe der Erfindung in der Entwicklung einer Vorrichtung die zum Mischen des Knochenzementteigs aus den Ausgangskomponenten vorgesehen und geeignet ist sowie eines Verfahrens zur Herstellung eines Knochenzementteigs, insbesondere eines niedrigviskosen pastenförmigen Polymethylmethacrylat-Knochenzementteigs, wobei der Knochenzementteig aus einem Zementpulver und einer Monomerflüssigkeit hergestellt wird, mit denen die Nachteile der bisherigen Vorrichtungen und Verfahren überwunden werden.

Die Erfindung hat die Aufgabe eine solche Vorrichtung derart zu verbessern, dass die Vorrichtung kostengünstig und möglichst weitgehend aus Kunststoff zu fertigen ist und die Anwendung einfach und für Fehlbedienungen unanfällig ist. Der Aufbau soll dabei auch deshalb kostengünstig sein, damit die Vorrichtung aus hygienischen Gründen nur einmalig verwendet werden kann. Gleichzeitig soll die Anwendung der Vorrichtung möglichst einfach sein. Ferner soll ein Sterilisieren der fertig zusammengesetzten Vorrichtung ermöglicht werden, ohne dass bei der Anwendung der Vorrichtung beziehungsweise des Verfahrens Monomerdämpfe der Monomerflüssigkeit oder die Monomerflüssigkeit selbst freigesetzt werden können. Die Mischung des Knochenzementteigs soll also trotz einer Erreichbarkeit des Inneren der Vorrichtung für ein sterilisierendes Gas in einem vollständig geschlossenen System erfolgen, ohne das die Gefahr eines Austritts der Ausgangskomponenten besteht.

Mit der Vorrichtung und mit dem Verfahren soll ein PMMA-Knochenzementteig herstellbar und zu applizieren sein, der für die Vertebroplastie und die Kyphoplastie einsetzbar ist. Derartige PMMA-Knochenzementteige sind relativ zu Knochenzementteigen zur Verankerung von Prothesen besonders dünnflüssig (niedrigviskos), da sie auch in kleine Zwischenräume von frakturierten Wirbeln und in enge Hohlräume von Pedikelschrauben noch gut einpressbar sein müssen. Die Ausbildung von Luftblasen beziehungsweise Gasblasen ist in einem dünnflüssigeren Knochenzementteig weniger kritisch, weil Gasblasen leichter aus einem niedrigviskosen Knochenzementteig entweichen können.

Es sollen möglichst viele oder alle in der Vorrichtung ablaufenden Prozesse, wie die Vermischung der Ausgangskomponenten, das Öffnen des Monomerflüssigkeitsbehälters und möglichst auch das Austragen des Knochenzementteigs mit möglichst wenigen Arbeitsschritten ablaufen und dabei einfach manuell zu bedienen sein. Bevorzugt soll keine weitere Apparatur außer der Vorrichtung und kein elektrischer oder anderer motorgetriebener Antrieb notwendig sein, um die Vorrichtung und das Verfahren anzuwenden. Die Vorrichtung und das Verfahren sollen also auch unter schwierigen Bedingungen problemlos einsetzbar sein.

Die Handhabung der Vorrichtung soll dabei maximal vereinfacht sein, um Anwendungsfehler infolge von fehlerhaft durchgeführten Montageschritten grundsätzlich zu vermeiden. Der medizinische Anwender soll die Vorrichtung nach der Entnahme aus einer Verpackung unmittelbar betätigen können. Weitere Montage- und Arbeitsschritte sollen durch den Aufbau der Vorrichtung und das Verfahren vermieden werden. Die Vorrichtung soll vorzugsweise auch eine sichere Lagerung von Zementpulver und Monomerflüssigkeit in voneinander getrennten Kompartimenten ermöglichen, so dass während der Lagerung der Vorrichtung eine unbeabsichtigte Vermischung der Zementkomponenten ausgeschlossen ist. Die Vorrichtung soll eine Sterilisation mit dem Gas Ethylenoxid ermöglichen. Das in der Vorrichtung gelagerte Zementpulver muss dazu für Ethylenoxid zugänglich sein.

Die Aufgabe der Erfindung besteht somit in der Entwicklung einer Vorrichtung zum Lagern und Vermischen von Zementpulver und Monomerflüssigkeit, wobei der durch Vermischung der Zementkomponenten gebildete Polymethylmethacrylat-Knochenzementteig insbesondere zur Augmentation von frakturierten Wirbelkörpern und zur Befüllung von Pedikelschrauben bestimmt ist. Die Vorrichtung soll weiterhin ein Auspressen des gebildeten Polymethylmethacrylat-Knochenzementteigs ermöglichen, ohne dass eine separate Auspressvorrichtung notwendig ist. Es soll ein Full-Prepacked-Zementiersystem entwickelt werden, bei dem der medizinische Anwender keinen Kontakt zur Monomerflüssigkeit und zum Zementpulver hat oder bekommen kann. Es soll ein geschlossenes Mischsystem bereitgestellt werden, das während des Mischvorgangs hermetisch gegenüber der Umgebung abgeschlossen ist, so dass keine Monomerdämpfe in die umgebende Atmosphäre austreten können. Gleichzeitig soll das Innere der Vorrichtung aber für Ethylenoxid zugänglich sein. Dabei ist es wichtig, dass das in der Vorrichtung gelagerte Zementpulver und alle im Inneren der Vorrichtung vorhandenen Oberflächen für Ethylenoxid zugänglich sein müssen. Die Handhabung der Vorrichtung soll maximal vereinfacht sein, um Anwendungsfehler infolge von fehlerhaft durchgeführten Montageschritten grundsätzlich zu vermeiden.

Die Aufgaben der Erfindung werden gelöst durch eine Vorrichtung zum Herstellen eines Knochenzementteigs aus einer Monomerflüssigkeit und einem Zementpulver als Ausgangskomponenten des Knochenzementteigs und zum Austragen des gemischten Knochenzementteigs, die Vorrichtung aufweisend
eine Kartusche mit einem zylindrischen Innenraum, wobei das Zementpulver im Innenraum der Kartusche angeordnet ist, die Kartusche an einer geschlossenen Vorderseite eine Austragsöffnung aufweist, die Austragsöffnung durch einen entfernbaren Verschluss verschlossen ist und wobei die Kartusche an einer Rückseite ein Gewinde aufweist,
eine Monomeraufnahme aufweisend ein zum Gewinde an der Rückseite der Kartusche passendes Gegengewinde, wobei die Monomeraufnahme in ihrem Inneren eine Kammer bildet, die Monomeraufnahme über ihr Gegengewinde an das Gewinde der Kartusche geschraubt ist, die Monomeraufnahme gegen die Kartusche in Längsrichtung schraubbar beweglich ist und wobei die Monomeraufnahme an einer Vorderseite einen zylindrischen Kolben bildet, wobei in dem Kolben ein für Gase und die Monomerflüssigkeit durchlässiger aber für das Zementpulver undurchlässiger Durchgang vorgesehen ist, wobei der Durchgang den Innenraum der Kartusche mit der Kammer der Monomeraufnahme verbindet, wobei der Kolben den Innenraum der Kartusche an seiner Rückseite bis auf den Durchgang dicht verschließt und wobei in der Wandung der Monomeraufnahme wenigstens eine Begasungsöffnung angeordnet ist, die die Kammer mit der Umgebung der Vorrichtung verbindet,
einen Monomerflüssigkeitsbehälter enthaltend die Monomerflüssigkeit, wobei der Monomerflüssigkeitsbehälter in der Kammer der Monomeraufnahme angeordnet ist, und
eine Öffnungseinrichtung zum Öffnen des Monomerflüssigkeitsbehälters innerhalb der Kammer der Monomeraufnahme, wobei die wenigstens eine Begasungsöffnung durch die Bewegung der Öffnungseinrichtung oder durch eine mit der Bedienung der Öffnungseinrichtung synchronisierte Bewegung eines Verschlussmittels verschließbar ist, bevor der Monomerflüssigkeitsbehälter durch die Öffnungseinrichtung geöffnet ist.

Der zylindrische Innenraum und der zylindrische Kolben haben eine Zylindergeometrie mit kreisförmiger Grundfläche. Dadurch kann sich der zylindrische Kolben im zylindrischen Innenraum bei einer Schraubbewegung der Monomeraufnahme drehen und den zylindrischen Innenraum gleichzeitig an seiner Rückseite abdichten.

Der Durchgang kann vorzugsweise durch mehrere Kanäle gebildet werden, die von einem für die Monomerflüssigkeit und Gase durchlässigen aber für das Zementpulver undurchlässigen Porenfilter abgedeckt sind. Vorzugsweise kann der Durchgang zusätzlich mit einem Gitter und/oder einem Sieb abgedeckt sein, mit dem Splitter oder Bruchstücke des Monomerflüssigkeitsbehälters zurückgehalten werden.

Zur Abdichtung des Kolbens gegen die Innenwand des Innenraums der Kartusche kann zumindest eine umlaufende Dichtung am Kolben vorgesehen sein, vorzugsweise sind zwei in Längsrichtung voneinander beabstandete umlaufende Dichtungen vorgesehen. Die Dichtungen können aus Gummi bestehen. Hierdurch kann beim Vergrößern des Innenraums der Kartusche durch Herausschrauben der Monomeraufnahme aus dem Innenraum der Kartusche ein Unterdruck im Innenraum der Kartusche erzeugt werden, mit dem die Monomerflüssigkeit aus der Kammer in den Innenraum der Kartusche gesaugt werden kann. Zudem kann auch ein Gas, das in dem Knochenzementteig vorhanden beziehungsweise verteilt ist, durch einen durch Einschrauben des Kolbens beziehungsweise der Monomeraufnahme in den Innenraum erzeugten Druck auf das Knochenzementteig-Gas-Gemisch durch den Durchgang heraus und in die Monomeraufnahme hineingetrieben werden oder durch die Austragsöffnung herausgetrieben werden, ohne dass Knochenzementteig zwischen dem Kolben und der Innenwand der Kartusche aus der Vorrichtung nach draußen dringen kann. Dadurch kann verhindert werden, dass der Anwender oder der OP-Saal mit Knochenzementteig verschmutzt wird und dass Knochenzementteig ungenutzt verloren geht.

Besonders bevorzugt kann vorgesehen sein, dass der Kolben gasdicht axial in dem Innenraum der Kartusche bewegbar ist.

Ebenso kann an der Öffnungseinrichtung zumindest eine Dichtung, vorzugsweise zwei Dichtungen, vorgesehen sein, mit der oder mit denen die wenigstens eine Begasungsöffnung von der Öffnungseinrichtung verschließbar ist. Besonders bevorzugt kann dabei vorgesehen sein, dass die wenigstens eine Begasungsöffnung gasdicht und ganz besonders bevorzugt auch druckdicht verschließbar ist.

Der Verschluss ist vorzugsweise ein Verschlussstopfen, der die Austragsöffnung im geschlossenen Zustand gasdicht verschließt. Es kann alternativ auch vorgesehen sein, dass der Verschluss für Gase durchlässig aber für das Zementpulver und den Knochenzementteig undurchlässig ist, wobei diese Variante weniger bevorzugt wird, weil dies einen Transfer der Monomerflüssigkeit mit Hilfe eines Unterdrucks im Innenraum der Kartusche unmöglich macht.

Durch die wenigstens eine Begasungsöffnung kann die Vorrichtung auch in ihrem Inneren, und insbesondere auch das Zementpulver im Innenraum der Kartusche, mit Hilfe eines sterilisierenden Gases, wie beispielsweise Ethylenoxid, sterilisiert werden. Durch die Verschließbarkeit der wenigstens einen Begasungsöffnung kann verhindert werden, dass Monomerflüssigkeit aus der Monomeraufnahme nach außen dringt. Wenn die wenigstens eine Begasungsöffnung gasdicht und bevorzugt auch druckdicht verschließbar ist, kann in der Kammer der Monomeraufnahme ein Unterdruck erzeugt werden, der zum Absaugen von Gasen aus dem Knochenzementteig verwendet werden kann, oder ein Überdruck erzeugt werden, mit dem die Monomerflüssigkeit aus der Kammer in den Innenraum der Kartusche überführt beziehungsweise gesaugt werden kann.

Bei erfindungsgemäßen Vorrichtungen kann vorgesehen sein, dass die Öffnungseinrichtung eine Sicherungseinrichtung aufweist, die ein erneutes Öffnen der wenigstens einen Begasungsöffnung nach deren Verschließen verhindert, wobei vorzugsweise die Öffnungseinrichtung über ein Gewinde mit der Monomeraufnahme verbunden ist und beim Einschrauben der Öffnungseinrichtung in die Kammer die wenigstens eine Begasungsöffnung verschließbar ist, wobei die Sicherungseinrichtung eine Rückdrehsicherung ist.

Hiermit kann verhindert werden, dass die wenigstens eine Begasungsöffnung nach dem Öffnen des Monomerflüssigkeitsbehälters aus Versehen wieder geöffnet wird und dann Monomerflüssigkeit aus der Vorrichtung austreten kann.

Ferner kann vorgesehen sein, dass ein erstes lösbares Sicherungselement vorgesehen ist, das eine Bedienung der Öffnungseinrichtung verhindert, und/oder ein zweites lösbares Sicherungselement vorgesehen ist, das ein Schrauben der Monomeraufnahme in die Kartusche verhindert.

Hiermit kann sichergestellt werden, dass die Vorrichtung nicht aus Versehen beim Transport oder beim Bereitstellen der Vorrichtung aktiviert beziehungsweise bedient wird.

Des Weiteren kann vorgesehen sein, dass an der in die Kammer der Monomeraufnahme weisenden Seite des Kolbens ein Dorn oder eine Schneidkante zum Aufbrechen des Monomerflüssigkeitsbehälters angeordnet ist, wobei vorzugsweise zwischen dem Kolben und dem Monomerflüssigkeitsbehälter ein zusammenpressbares Lagerungselement, insbesondere eine Feder oder ein Schaumstoff oder ein elastischer Hohlkörper, angeordnet ist, wobei das Lagerungselement den Monomerflüssigkeitsbehälter von dem Dorn oder der Schneidkante beabstandet.

Hierdurch kann der Monomerflüssigkeitsbehälter mit dem Dorn oder der Schneidkante an einer definierten Stelle im Bereich des Durchgangs geöffnet werden, so dass die Monomerflüssigkeit aus dem in diesem Bereich geöffneten Monomerflüssigkeitsbehälter unmittelbar durch den Durchgang in den Innenraum der Kartusche fließen kann. Mit dem Lagerungselement kann verhindert werden, dass der Monomerflüssigkeitsbehälter aus Versehen beim Transport, beispielsweise durch Stöße, versehentlich geöffnet wird.

Es kann bevorzugt auch vorgesehen sein, dass zumindest ein loses Mischelement, insbesondere zumindest eine lose Kugel, frei beweglich im Innenraum der Kartusche angeordnet ist.

Hierdurch kann der Inhalt des Innenraums der Kartusche beziehungsweise das Zementpulver und die eingeleitete Monomerflüssigkeit durch Schütteln der Kartusche effizienter gemischt werden, wobei das sich dabei im Innenraum der Kartusche bewegende beziehungsweise hin- und herfliegende oder geschleuderte zumindest eine Mischelement den Mischvorgang unterstützt.

Dabei kann vorgesehen sein, dass an der in den Innenraum der Kartusche weisenden Vorderseite der Kartusche, die den Innenraum der Kartusche an der Vorderseite begrenzt, wenigstens ein Vorsprung neben der Austragsöffnung und/oder wenigstens ein bis zur Austragsöffnung laufenden Steg angeordnet ist, der sich in den Innenraum der Kartusche erstreckt.

Hiermit kann verhindert werden, dass sich das zumindest eine Mischelement, insbesondere die zumindest eine Kugel, vor die Austragsöffnung setzt und diese verschließt. Der wenigstens eine Steg erstreckt sich vorzugsweise radial in dem Innenraum der Kartusche ausgehend von der seitlichen Wandung der Kartusche zu der auf der Zylinderachse des zylindrischen Innenraums liegenden Austragsöffnung. Besonders bevorzugt nimmt die Höhe des wenigstens einen Stegs dabei in Richtung der Austragsöffnung hin zu.

Bei erfindungsgemäßen Vorrichtungen mit zumindest einem Mischelement kann vorgesehen sein, dass im Innenraum der Kartusche an dessen Vorderseite ein verformbares Aufnahmeelement angeordnet ist, insbesondere eine vorformbare Ringscheibe angeordnet ist, wobei vorzugsweise die Höhe des verformbaren Aufnahmeelements in radialer Richtung nach außen zur Seitenwand des Innenraums hin zunimmt.

Das zumindest eine Mischelement kann von dem in den Innenraum der Kartusche hineingeschraubten Kolben in das verformbare Aufnahmeelement hineingedrückt werden, wobei sich das Aufnahmeelement dabei verformt. Das Aufnahmeelement kann das zumindest eine Mischelement dadurch aufnehmen. Gleichzeitig bleibt an den anderen Stellen das verformbare Aufnahmeelement unverformt, so dass weniger ungenutztes Volumen in dem Innenraum der Kartusche verbleibt, in dem Knochenzementteig zurückbleiben kann. Hierdurch kann ein größerer Anteil des gemischten Knochenzementteigs aus dem Innenraum der Kartusche ausgepresst und genutzt werden.

Das Aufnahmeelement kann mit einem elastischen Material ausgeführt werden, wobei ein Hohlkörper mit elastischen Wandungen, insbesondere aus einem Gummi, bevorzugt wird. Alternativ kann das Aufnahmeelement auch durch ein geschlossen-poriges Material, wie einen geschlossen-porigen Schaustoff geformt werden. Die geschlossenen Poren verhindern ein Eindringen von Zementpulver und eine großflächige Adsorption der Monomerflüssigkeit an der zugänglichen Oberfläche des Materials des Aufnahmeelements.

Das Aufnahmeelement kann erfindungsgemäß bevorzugt an einem Kartuschendeckel befestigt sein, mit dem das vordere Ende der Kartusche und damit der Innenraum der Kartusche an seiner Vorderseite gasdicht verschließbar ist.

Ferner kann vorgesehen sein, dass das zumindest eine lose Mischelement eine größere Dichte aufweist als Polymethylmethacrylat (PMMA), vorzugsweise eine zumindest doppelt so hohe Dichte oder eine zumindest dreimal so hohe Dichte als Polymethylmethacrylat aufweist, wobei bevorzugt das zumindest eine Mischelement aus einem Korund, aus α-Korund, aus einem Zirkonoxid, aus tetragonalem ZrO₂ oder aus mit Y₂O₃ kubisch stabilisiertem ZrO₂ besteht.

Durch die höhere Dichte kann das zumindest eine Mischelement beim Schütteln gut gegen den Knochenzementteig bewegt werden.

Es kann erfindungsgemäß vorgesehen sein, dass am Durchgang ein Porenfilter angeordnet ist, der für Gase und die Monomerflüssigkeit durchlässig aber für das Zementpulver und den Knochenzementteig undurchlässig ist.

Hiermit kann auf einfache Weise verhindert werden, dass das Zementpulver in die Kammer der Monomeraufnahme und vorzugsweise auch in den Durchgang eindringt und dort vorzeitig mit der Monomerflüssigkeit reagiert und der Durchgang durch anquellenden Knochenzementteig verschlossen wird.

Es kann auch vorgesehen sein, dass der Monomerflüssigkeitsbehälter eine Ampulle aus Glas oder Kunststoff ist und zwischen dem Durchgang und der Kammer der Monomeraufnahme ein Splitterschutz, ein Gitter oder ein Sieb zum Zurückhalten von Splittern oder Bruchtücken der Ampulle vorgesehen ist. Hiermit kann verhindert werden, dass der Durchgang durch die Splitter oder Bruchstücke verschlossen wird und dass diese ungewollt in den Innenraum der Kartusche vordringen können.

Gemäß einer bevorzugten Weiterbildung der vorliegenden Erfindung kann vorgesehen sein, dass der Monomerflüssigkeitsbehälter eine Ampulle aus Glas oder Kunststoff ist, wobei die Ampulle einen Ampullenkörper mit einer zylindrischen Wandung aufweist und wobei die Öffnungseinrichtung einen in der Monomeraufnahme in einer Längsrichtung der zylindrischen Kammer der Monomeraufnahme beweglichen Hohlzylinder aufweist, der mit der zylindrischen Wandung der Ampulle fluchtet, so dass die Ampulle mit dem Hohlzylinder in Richtung des Innenraums der Kartusche drückbar ist.

Hierdurch kann die Ampulle als Monomerflüssigkeitsbehälter in Richtung der Kartusche gedrückt werden, ohne dass die Ampulle an der von der Kartusche abgewandten Seite von dem Hohlzylinder aufgebrochen wird. Dadurch kann sichergestellt werden, dass die Ampulle an der zum Innenraum der Kartusche weisenden Seite aufgebrochen wird.

Der Hohlzylinder kann Durchbrechungen aufweisen oder auch in Längsrichtung geschlitzt sein. Es wird jedoch erfindungsgemäß bevorzugt, dass der Hohlzylinder zumindest an der der Ampulle zugewandten Vorderseite durchgehend ist, damit beim Eindrücken des Hohlzylinders keine Druckspitzen an der Wandung des Ampullenkörpers auftreten, die zu einem ungewollten Brechen der Wandung des Ampullenkörpers führen könnten.

Alternativ zu einer Ampulle kann der Monomerflüssigkeitsbehälter auch durch einen Folienbeutel realisiert werden, der in der Kammer der Monomeraufnahme mit der Öffnungseinrichtung aufgerissen, aufgestochen oder aufgeschnitten wird, um die Monomerflüssigkeit in der Kammer der Monomeraufnahme freizusetzen.

Wenn die Ampulle aus Kunststoff besteht, so muss sie aus einem gegenüber der Monomerflüssigkeit chemisch stabilen Kunststoff bestehen. Glas ist als Material für die Ampulle bevorzugt.

Bei erfindungsgemäßen Vorrichtungen mit Ampullen mit zylindrischem Körper kann vorgesehen sein, dass die wenigstens eine Begasungsöffnung neben dem Hohlzylinder in die Kammer der Monomeraufnahme mündet, so dass eine Bewegung des Hohlzylinders in die Kammer hinein die wenigstens eine Begasungsöffnung mit einer seitlichen Wandung des Hohlzylinders flüssigkeitsdicht oder gasdicht verschließt, wobei bevorzugt der Hohlzylinder hierzu zumindest einen umlaufenden Dichtungsring aufweist, besonders bevorzugt zwei umlaufende Dichtungsringe aufweist, der oder die die wenigstens eine Begasungsöffnung bei einer Bewegung des Hohlzylinders in die Kammer hinein überfahren und gegen die Kammer abdichten. Bevorzugt wird die wenigstens eine Begasungsöffnung mit einer seitlichen Wandung des Hohlzylinders gasdicht verschlossen.

Hierdurch kann auf konstruktiv einfache Wiese sichergestellt werden, dass die wenigstens eine Begasungsöffnung verschlossen wird und dann keine Monomerflüssigkeit mehr austreten kann. Bevorzugt kann durch eine Bewegung der Monomeraufnahme in dem Innenraum der Kartusche ein Unterdruck erzeugt werden, wenn die Begasungsöffnung gasdicht verschlossen wird.

Des Weiteren kann vorgesehen sein, dass die den Innenraum begrenzende Vorderseite des Kolbens und/oder die die Vorderseite des Innenraums begrenzende Oberfläche sich in radialer Richtung zunehmend in den Innenraum der Kartusche hinein erstreckt, so dass eine vordere Grundfläche und/oder eine rückseitige Grundfläche des Innenraums keine Kante oder keine Kante mit einem Winkel von weniger als 60° aufweist, wobei vorzugsweise die vordere Grundfläche und/oder die rückseitige Grundfläche des Innenraums eine abgerundete Form aufweisen.

Hiermit wird verhindert, dass ein Pulver in einer spitzen Kante des Innenraums nicht oder nur schlecht von der Monomerflüssigkeit erreicht werden kann und mit der Monomerflüssigkeit durchmischt werden kann und deshalb die Konsistenz des Knochenzementteigs inhomogen werden kann oder nicht das gewünschte Mischungsverhältnis erhält.

Es kann zu dem gleichen Zweck auch vorgesehen sein, dass die den Innenraum begrenzende Vorderseite des Kolbens und/oder die die Vorderseite des Innenraums begrenzende Oberfläche eine abgerundete Form mit in Richtung der seitlichen Wandungen des zylindrischen Innenraums (der Zylindermantelflächen) ansteigenden Flanken aufweist oder aufweisen.

Um eine vollständige Durchmischung des Knochenzementteigs zu erreichen, kann auch vorgesehen sein, dass zumindest eine Kugel als Mischelement frei beweglich im Innenraum der Kartusche angeordnet ist und der Radius der zumindest einen Kugel genauso groß oder kleiner ist als der Krümmungsradius der vorderen Grundfläche und/oder der rückseitigen Grundfläche des Innenraums der Kartusche.

Durch den angepassten Krümmungsradius der zumindest einen Kugel als Mischelement an den Krümmungsradius der Grundflächen wird erreicht, dass die wenigstens eine in dem Innenraum der Kartusche geschleuderte Kugel jeden Bereich des Innenraums der Kartusche erreichen kann, so dass keine nicht oder schlecht durchmischten Bereiche in dem Innenraum der Kartusche verbleiben, die nicht von der zumindest einen Kugel erreicht werden können.

Es kann auch vorgesehen sein, dass die Öffnungseinrichtung mit der Monomeraufnahme über ein Gewinde und ein Gegengewinde verbunden sind, so dass die Öffnungseinrichtung in die Kammer der Monomeraufnahme schraubbar ist und der Monomerflüssigkeitsbehälter durch Hineinschrauben der Öffnungseinrichtung in die Kammer der Monomeraufnahme aufbrechbar, aufschneidbar oder aufstechbar ist.

Hiermit kann der Monomerflüssigkeitsbehälter auf einfache Weise in der Monomeraufnahme geöffnet werden.

Dabei kann vorgesehen sein, dass das Gegengewinde der Monomeraufnahme sowohl zu dem Gewinde der Kartusche als auch zu einem Gewinde der Öffnungseinrichtung passt. Die Monomeraufnahme kann dann mit einem gleichförmigen Gewinde gestaltet werden, besonders bevorzugt mit einem durchgehenden Außengewinde.

Ferner kann vorgesehen sein, dass das Gewinde an der Rückseite der Kartusche ein Innengewinde ist und das Gegengewinde der Monomeraufnahme ein an den seitlichen Außenflächen vorgesehenes Außengewinde.

Hierdurch lässt sich die Vorrichtung besonders kompakt aufbauen.

Dabei kann vorgesehen sein, dass der Kolben einen größeren Durchmesser hat als das Innengewinde an der Rückseite der Kartusche.

Dadurch kann der Kolben die Rückseite der Kartusche gegenüber der Innenwand der Kartusche gut abdichten und den Knochenzementteig in voller Breite aus dem Innenraum der Kartusche austreiben. Zudem kann so die Monomeraufnahme nicht vollständig aus der Kartusche herausgeschraubt werden. Der Kolben bildet an seiner Rückseite zusammen mit dem Beginn des Innengewindes der Kartusche einen Anschlag, über den hinaus die Monomeraufnahme nicht aus der Kartusche herausgeschraubt werden kann, so dass ein versehentliches Öffnen des Innenraums der Kartusche ausgeschlossen ist.

Des Weiteren kann vorgesehen sein, dass die Vorderseite der Kartusche mit einem Kartuschendeckel verschlossen ist, wobei die Austragsöffnung in dem Kartuschendeckel angeordnet ist und der Kartuschendeckel gasdicht und flüssigkeitsdicht mit den seitlichen Wandungen der Kartusche verbunden ist, wobei bevorzugt der Kartuschendeckel auf ein Außengewinde an der Vorderseite der Kartusche aufgeschraubt ist.

Hierdurch wird die Montage der Vorrichtung vereinfacht. Die Monomeraufnahme kann so von vorne in die Kartusche eingeführt werden und in ein Innengewinde als das Gewinde an der Rückseite der Kartusche geschraubt werden, auch wenn der Kolben einen größeren Radius aufweist als das Innengewinde (das Gegengewinde) an der Monomeraufnahme. Anschließend kann das Zementpulver eingefüllt werden und der Innenraum der Kartusche wird an der Vorderseite mit dem Kartuschendeckel verschlossen.

Gemäß einer Weiterbildung der vorliegenden Erfindung kann vorgesehen sein, dass die Öffnungseinrichtung eine auf die Rückseite der Monomeraufnahme schraubbare Verschlusskappe aufweist, die die Kammer an der Rückseite gasdicht verschließt. Vorzugsweise ist ein Anschlag vorgesehen, der ein weiteres Aufschrauben der Verschlusskappe auf die Monomeraufnahme verhindert.

Hierdurch kann die Öffnungseinrichtung ebenso wie die Monomeraufnahme durch Schrauben bedient werden. Zudem kann ein Gas aus dem Innenraum der Kartusche in die Kammer gepresst werden, ohne dass dieses nach außen treten kann.

Dabei kann vorgesehen sein, dass an der Verschlusskappe eine Hülse angeordnet ist, die derart ins Innere der Kammer steckbar oder schraubbar ist, dass sie die Kammer an deren Rückseite gasdicht verschließt.

Die vorspringende Hülse kann zum Öffnen des Monomerflüssigkeitsbehälters verwendet werden.

Die Hülse kann erfindungsgemäße bevorzugt auch zum gasdichten Verschließen der wenigstens einen Begasungsöffnung in der Wandung der Monomeraufnahme verwendet werden. Hierzu weist die Hülse besonders bevorzugt wenigstens eine umlaufende Dichtung auf, ganz besonders bevorzugt zwei umlaufende Dichtungen auf. Es kann also vorgesehen sein, dass die Hülse gasdicht in der Kammer verschiebbar ist.

Wenn die Verschlusskappe ein Innengewinde aufweist, das auf ein Außengewinde an der Rückseite der Monomeraufnahme schraubbar ist, bildet die der Kammer zugewandte Rückwand der Verschlusskappe zusammen mit dem hinteren Ende der Wandung der Monomeraufnahme den Anschlag. Dabei kann bevorzugt vorgesehen sein, dass das Innengewinde der Kappe eine geringere Höhe in Längsrichtung hat als die Hülse.

Besonders bevorzugt ragt die Hülse zumindest derart weit in die Kammer hinein, dass ein in der Kammer angeordneter Monomerflüssigkeitsbehälter mit der Hülse in der Kammer aufbrechbar, aufschneidbar oder aufreißbar ist, wenn die Verschlusskappe bis zum Anschlag auf die Monomeraufnahme aufgeschraubt ist oder wenn die Verschlusskappe auf die Monomeraufnahme aufgeschraubt wird. Wenn der Monomerflüssigkeitsbehälter eine Ampulle aus Glas oder aus einem Kunststoff ist und die Hülse auf die seitlichen Wandungen des Ampullenkörpers drückt, muss der Abstand der Vorderkante der Hülse bis zu dem der Kammer zugewandten Ende des Kolbens oder der Spitze eines daran angeordneten Dorns oder einer daran angeordneten Schneidkante bei bis zum Anschlag aufgeschraubter Verschlusskappe kleiner sein als die Höhe des Ampullenkörpers.

Ferner kann vorgesehen sein, dass in einem Zustand der Vorrichtung, bei dem die Monomeraufnahme bis zu einem Anschlag maximal aus dem Innenraum der Kartusche herausgeschraubt ist, in dem derart maximal vergrößerten Innenraum der Kartusche ein freies Volumen über dem Zementpulver vorhanden ist, das mindestens genausgroß ist wie das Volumen der Monomerflüssigkeit in dem Monomerflüssigkeitsbehälter, vorzugsweise mindestens doppelt so groß ist wie das Volumen der Monomerflüssigkeit in dem Monomerflüssigkeitsbehälter.

Hierdurch wird sichergestellt, dass in dem Innenraum der Kartusche nach dem Einfüllen der Monomerflüssigkeit noch ein ausreichend großer freier Raum enthalten ist, so dass der Inhalt durch Schütteln gut gemischt werden kann. Wenn Mischelemente vorhanden sind, wird durch die Luft beziehungsweise das Gas eine ausreichende Beweglichkeit der Mischelemente in dem Innenraum der Kartusche gewährleistet, so dass diese die Ausgangskomponenten des Knochenzementteigs gut durchmischen können. Die Monomerflüssigkeit fließt bei Überführen der Monomerflüssigkeit in den Innenraum der Kartusche in die Zwischenräume zwischen den Pulverpartikeln des Zementpulvers. Wenn die wenigstens eine Begasungsöffnung gasdicht verschlossen ist, wird dabei der Druck in dem Innenraum der Kartusche verringert, also kein zusätzliches Gas, das nicht schon zuvor in dem Innenraum der Kartusche und in der Kammer vorhanden war, in den Innenraum der Kartusche eingetragen. Dadurch wird die Menge an Gas in dem Gemisch nicht erhöht, so dass später weniger Gas aus dem gemischten Knochenzementteig entweichen muss.

Die der vorliegenden Erfindung zugrundeliegenden Aufgaben werden auch gelöst durch ein Verfahren zur Herstellung eines Knochenzementteigs, insbesondere eines niedrigviskosen Polymethylmethacrylat-Knochenzementteigs zur Augmentation von Wirbelkörpern, wobei der Knochenzementteig aus einem Zementpulver und einer Monomerflüssigkeit hergestellt wird und das Verfahren mit einer Vorrichtung zum Herstellen des Knochenzementteigs und zum Austragen des gemischten Knochenzementteigs durchgeführt wird, gekennzeichnet durch die folgenden Schritte:
A) Bedienen einer Öffnungseinrichtung der Vorrichtung, wobei durch das Bedienen der Öffnungseinrichtung wenigstens eine Begasungsöffnung in einer Wandung einer Monomeraufnahme der Vorrichtung, die die Umgebung der Vorrichtung mit einer Kammer im Inneren der Monomeraufnahme gasdurchlässig verbindet, gasdicht verschlossen wird und anschließend ein Monomerflüssigkeitsbehälter enthaltend die Monomerflüssigkeit innerhalb der Kammer der Monomeraufnahme geöffnet wird,
B) Überführen der Monomerflüssigkeit aus der Monomeraufnahme durch einen für Gase und die Monomerflüssigkeit durchlässigen aber für das Zementpulver undurchlässigen Durchgang, der in einem Kolben angeordnet ist, welcher an der Vorderseite der Monomeraufnahme ausgebildet ist, in einen Innenraum einer Kartusche, in dem das Zementpulver enthalten ist,
C) Mischen der Monomerflüssigkeit und des Zementpulvers in dem Innenraum der Kartusche zu einem Knochenzementteig,
D) Herausdrücken von Gas, das in dem Knochenzementteig verteilt ist, aus dem Innenraum der Kartusche, durch Hineinschrauben der Monomeraufnahme mit dem Kolben an der Vorderseite der Monomeraufnahme von der Rückseite des Innenraums der Kartusche in die Kartusche, und
E) Auspressen des fertig gemischten Knochenzementteigs aus dem Innenraum der Kartusche durch eine dem Kolben gegenüberliegende Austragsöffnung der Kartusche durch weiteres Hineinschrauben der Monomeraufnahme in die Kartusche.

Das Überführen der Monomerflüssigkeit in Schritt B) in die Kartusche erfolgt erfindungsgemäß bevorzugt durch Herausfließen, Heraussaugen, Herausdrücken oder eine Kombination mehrerer oder aller dieser Maßnahmen aus der Kammer der Monomeraufnahme in den Innenraum der Kartusche.

Das Gas kann in Schritt D) aus dem Innenraum der Kartusche durch den Durchgang in die Kammer der Monomeraufnahme herausgedrückt werden oder durch die zuvor geöffnete Austragsöffnung herausgedrückt werden.

Unmittelbar vor Schritt D) oder unmittelbar vor Schritt E) kann ein Öffnen der dem Kolben gegenüberliegende Austragsöffnung erfolgen.

Bei erfindungsgemäßen Verfahren kann vorgesehen sein, dass das Verfahren mit einer erfindungsgemäßen Vorrichtung durchgeführt wird.

Das Verfahren weist dann die mit der erfindungsgemäßen Vorrichtung erzielten Vorteile auf.

Des Weiteren kann vorgesehen sein, dass in Schritt C) das Mischen der Monomerflüssigkeit und des Zementpulvers durch ein Schütteln der Kartusche erfolgt, wobei in dem Innenraum der Kartusche zusätzlich zum Zementpulver und der Monomerflüssigkeit ein Gas und zumindest ein loses und im Innenraum der Kartusche frei bewegliches Mischelement, insbesondere zumindest eine lose Kugel, enthalten ist, wobei das zumindest eine Mischelement in dem Innenraum der Kartusche durch das Schütteln geschleudert wird und dadurch eine Durchmischung des Zementpulvers mit der Monomerflüssigkeit erfolgt.

Hiermit kann eine Durchmischung des Zementpulvers mit der Monomerflüssigkeit erreicht werden, ohne dass ein extern bedienbarer Mischer verwendet werden muss, wie beispielsweise ein Mischpaddel, das über eine Stange bewegt wird, die durch eine abgedichtete Durchführung in der Kartuschenwand geführt werden müsste.

Es kann vorgesehen sein, dass das Öffnen des Monomerflüssigkeitsbehälters in Schritt A) durch Bedienen der Öffnungseinrichtung erfolgt, wobei beim Bedienen der Öffnungseinrichtung die wenigstens eine Begasungsöffnung verschlossen wird, die die Kammer mit der Umgebung der Vorrichtung gasdurchlässig verbindet.

Des Weiteren kann vorgesehen sein, dass die Öffnungseinrichtung in die Monomeraufnahme hineingeschraubt wird und durch die Bewegung der Öffnungseinrichtung der Monomerflüssigkeitsbehälter geöffnet und die wenigstens eine Begasungsöffnung, die in einer Wandung der Monomeraufnahme angeordnet ist, zuvor verschlossen wird.

Hierdurch kann die Kammer und der Innenraum der Kartusche mit Hilfe eines sterilisierenden Gases durch die Belüftungsöffnung zuvor sterilisiert werden, wobei die Kammer beim Öffnen des Monomerflüssigkeitsbehälters geschlossen ist, so dass keine Monomerflüssigkeit aus der Kammer austreten kann.

Bevorzugt kann auch vorgesehen sein, dass der Monomerflüssigkeitsbehälter eine Ampulle aus Glas oder aus einem Kunststoff ist und beim Öffnen der Ampulle in Schritt A) die Ampulle auf einen Dorn oder eine Schneidkante an der in die Kammer der Monomeraufnahme weisenden Seite des Kolbens gedrückt wird, so dass die Ampulle an dieser Seite aufgebrochen wird und die Monomerflüssigkeit im Bereich des Durchgangs aus der geöffneten Ampulle austritt.

Hierdurch kann die Ampulle mit dem Dorn oder der Schneidkante an einer definierten Stelle im Bereich des Durchgangs geöffnet werden, so dass die Monomerflüssigkeit aus der in diesem Bereich geöffneten Ampulle unmittelbar durch den Durchgang in den Innenraum der Kartusche fließen kann.

Gemäß einer Weiterbildung des erfindungsgemäßen Verfahrens kann vorgesehen sein, dass während des Schritts B), bevorzugt während der Schritte A) und B), die Monomeraufnahme oberhalb der Kartusche gehalten wird, so dass die Monomerflüssigkeit angetrieben von der Schwerkraft aus der Kammer durch den Durchgang in den Innenraum der Kartusche fließt.

Hiermit kann auf ein separates Bauteil zum Überführen der Monomerflüssigkeit in den Innenraum der Kartusche verzichtet werden. Bevorzugt wird zusätzlich durch Herausschrauben der Monomeraufnahme aus der Kartusche ein Unterdruck in dem Innenraum der Kartusche erzeugt, mit dem die Monomerflüssigkeit zusätzlich aus der Kammer in den Innenraum der Kartusche gesaugt wird.

Ferner kann vorgesehen sein, dass vor Schritt A) ein Schritt erfolgt, bei dem ein erstes Sicherungselement gelöst wird, das eine Bedienung der Öffnungseinrichtung verhindert, und/oder ein zweites Sicherungselement gelöst wird, das ein Schrauben der Monomeraufnahme in der Kartusche verhindert.

Hiermit kann sichergestellt werden, dass der Monomerflüssigkeitsbehälter nicht aus Versehen beim Transport oder beim Bereitstellen der gesamten Vorrichtung geöffnet und die Vorrichtung nicht versehentlich aktiviert beziehungsweise bedient wird.

Es kann auch vorgesehen sein, dass zum Überführen der Monomerflüssigkeit von der Kammer in den Innenraum der Kartusche in Schritt B) die Monomeraufnahme aus dem Innenraum der Kartusche herausgeschraubt wird, so dass sich der Innenraum der Kartusche vergrößert und in dem Innenraum der Kartusche ein Unterdruck entsteht, mit dem die Monomerflüssigkeit aus der Kammer in den Innenraum der Kartusche gesaugt wird.

Hierdurch kann der Transfer der Monomerflüssigkeit aus der Kammer in den Innenraum der Kartusche erreicht oder zumindest beschleunigt werden.

Der Erfindung liegt die überraschende Erkenntnis zugrunde, dass das Innere der Vorrichtung mit wenigstens einer Begasungsöffnung für ein sterilisierendes Gas erreichbar gestaltet werden kann, wobei gleichzeitig eine Öffnungseinrichtung, die zum Öffnen eines Monomerflüssigkeitsbehälters innerhalb der Vorrichtung verwendet wird, die wenigstens eine Begasungsöffnung entweder selbst oder mit einem separaten Verschlussmittel verschließt, bevor der Monomerflüssigkeitsbehälter geöffnet und die Monomerflüssigkeit innerhalb der Vorrichtung freigesetzt ist, so dass die Monomerflüssigkeit bei der Anwendung der Vorrichtung nicht aus der Vorrichtung freigesetzt werden kann. Gleichzeitig kann durch die Verwendung eines für das Zementpulver undurchlässigen aber für die Monomerflüssigkeit und Gase durchlässigen Durchgangs zwischen der Kammer der Monomeraufnahme und dem Innenraum der Kartusche verhindert werden, dass das lose in dem Innenraum der Kartusche gelagerte Zementpulver aus der Vorrichtung austreten kann. Durch beide Maßnahmen wird eine Vorrichtung geschaffen, deren Inneres mit einem sterilisierenden Gas, wie Ethylenoxid, sterilisiert werden kann und gleichzeitig die Ausgangskomponenten nicht aus der Vorrichtung austreten können. Wenn die wenigstens eine Begasungsöffnung gasdicht verschlossen wird, kann im Inneren der Vorrichtung zudem ein Unterdruck erzeugt werden, mit dem die Monomerflüssigkeit in den Innenraum der Kartusche gesaugt werden kann und der beim Mischen der Ausgangskomponenten ein größeres freies Volumen bereitstellt, so dass durch schütteln der Vorrichtung ein besseres Mischresultat erzielt werden kann, ohne dass die Gasmenge in dem Innenraum der Kartusche erhöht werden muss, die vor der Anwendung des Knochenzementteigs aus diesem herausgedrückt werden müsste.

Eine beispielhafte erfindungsgemäße Vorrichtung kann zusammengesetzt sein aus
a) einer hohlzylinderförmigen Kartusche, wobei an einem vorderen Ende der Kartusche ein Befestigungsmittel für einen Kartuschendeckel angeordnet ist und wobei am entgegengesetzten rückseitigen Ende der Kartusche an der Kartuscheninnenwand ein Innengewinde angeordnet ist,
b) einem Kartuschendeckel, der mit dem Befestigungsmittel am vorderen Ende der Kartusche gasdicht und flüssigkeitsdicht verbunden wird, wobei der Kartuschendeckel mindestens eine Austragsöffnung besitzt,
c) einen Verschlussstopfen, der in der Austragsöffnung des Kartuschendeckels gasdicht und lösbar angeordnet ist,
d) eine kolbenförmige hohlzylinderförmige Monomeraufnahme, die an einer Vorderseite einen Kolben bildet, wobei die Monomeraufnahme an ihrer Mantelfläche mindestens ein Schraubgewinde aufweist,
e) einem für Gase und Flüssigkeiten permeablen, aber nicht für Pulver permeablen Durchgang in einer ansonsten geschlossenen vorderen Grundfläche der Monomeraufnahme, der eine Kammer im Inneren der Monomeraufnahme mit einem Innenraum der Kartusche verbindet,
f) einem Stechdorn, der auf der Rückseite der geschlossenen vorderen Grundfläche der Monomeraufnahme angeordnet ist,
g) einem Monomerflüssigkeitsbehälter mit Monomerflüssigkeit, der mit seiner Bodenseite über dem Stechdorn beabstandet in der Kammer der Monomeraufnahme angeordnet ist,
h) einer verschiebbaren Hülse (beziehungsweise Hohlzylinder), die hinter der Rückseite des Monomerflüssigkeitsbehälters in der hohlzylinderförmigen Monomeraufnahme derart axial verschiebbar angeordnet ist, dass die Hülse über den Rand der hohlzylinderförmigen Monomeraufnahme herausragt,
i) einer einseitig verschlossenen, hohlen Verschlusskappe der hohlzylinderförmigen Monomeraufnahme, wobei ein Innengewinde und ein Anschlag für die hohlzylinderförmige Monomeraufnahme in der hohlen Verschlusskappe angeordnet sind, wobei der Abstand zwischen der unteren Außenkante der Verschlusskappe und dem Anschlag kleiner ist als der Abstand zwischen dem äußeren Ende der Hülse und dem Rand der Schmalseite der Monomeraufnahme aus dem die Hülse herausragt,
j) wenigstens einer Belüftungsöffnung in der Mantelfläche der hohlzylinderförmigen Monomeraufnahme, die durch axiale Verschiebung der Hülse gasdicht verschließbar ist,
k) Zementpulver, das in dem Innenraum der Kartusche angeordnet ist, der durch die Innenwand der Kartusche, den Kartuschendeckel und die geschlossene vordere Grundfläche der Monomeraufnahme begrenzt wird,
l) wobei die hohlzylinderförmige Monomeraufnahme mit ihrem Außengewinde mit dem Innengewinde der Kartusche verschraubt ist.

Die Hülse mit der Verschlusskappe bilden die Öffnungseinrichtung zum Öffnen des Monomerflüssigkeitsbehälters in der Kammer der Monomeraufnahme.

Die beispielhafte erfindungsgemäße Vorrichtung funktioniert in der Weise, dass im Sterilisationszustand- beziehungsweise im Lagerungszustand die kolbenförmige Monomeraufnahme mit dem mindestens einem Außengewinde so in die Kartusche geschraubt ist, dass der Kolben das Zementpulver berührt beziehungsweise sich kurz darüber befindet. Der Kartuschendeckel der Kartusche ist mit der Kartusche gasdicht verbunden. Der Verschlussstopfen ist gasdicht in die Austragsöffnung des Kartuschendeckels eingesetzt beziehungsweise eingesteckt. Die Hülse ist so hinter dem Monomerflüssigkeitsbehälter und dadurch oberhalb des Monomerflüssigkeitsbehälters angeordnet, dass die wenigstens eine Begasungsöffnung für Gase durchgängig ist und freiliegt. Die Hülse ragt über die Monomeraufnahme heraus und wird von der auf das Außengewinde der Monomeraufnahme aufgeschraubten Verschlusskappe umschlossen. Zur Aktivierung der Vorrichtung wird die Verschlusskappe in Richtung des Kartuschendeckels nach unten gedreht. Dabei schiebt die Verschlusskappe die Hülse in Richtung Kartuschendeckel in die Kammer der Monomeraufnahme hinein. Die wenigstens eine Belüftungsöffnung wird dabei durch die Hülse verdeckt und dadurch geschlossen. Die Hülse wird durch die Verschlusskappe weiter in Richtung Kartuschenkopf bewegt und drückt den Monomerflüssigkeitsbehälter gegen den Stechdorn. Der Monomerflüssigkeitsbehälter wird geöffnet und die Monomerflüssigkeit fließt nach unten aus. Dies ist der Grund dafür, dass die Vorrichtung bei der Anwendung mit der Kartusche nach unten gehalten wird. Die Monomerflüssigkeit beginnt nach unten in Richtung des Zementpulvers durch Einwirkung der Schwerkraft zu fließen. Die kolbenförmige Monomeraufnahme wird sofort in der zum Kartuschendeckel entgegengesetzten Rückseite der Kartusche geschraubt. Es entsteht ein Unterdruck im Innenraum der Kartusche mit dem Zementpulver. Dadurch wird die Monomerflüssigkeit in den Innenraum der Kartusche zum Zementpulver gesaugt. Danach wird die Kartusche enthalten die Ausgangskomponenten im Innenraum der Kartusche geschüttelt. Vorteilhaft ist es, wenn frei bewegliche Mischelemente den Mischvorgang unterstützen. Durch Vermischung des Zementpulvers mit der Monomerflüssigkeit entsteht der Knochenzementteig. Danach wird der Verschlussstopfen vom Kartuschendeckel entfernt und der gebildete Knochenzementteig durch Einschrauben der Monomeraufnahme in Richtung des Kartuschendeckels ausgepresst. Zuvor kann durch die gleiche Bewegung der Monomeraufnahme, beziehungsweise des an der Vorderseite der Monomeraufnahme gebildeten Kolbens Gas aus dem Knochenzementteig herausgepresst werden.

Die hohlzylinderförmige Monomeraufnahme hat an ihrer zylinderförmigen vorderen Kopfseite einen Durchmesser gleich oder kleiner dem Innendurchmesser der hohlzylinderförmigen Kartusche und die hohlzylinderförmige Monomeraufnahme kann mit ihrer Kopfseite gasdicht axial in der Kartusche bewegt werden.

Erfindungsgemäß kann vorgesehen sein, dass das Außengewinde der Monomeraufnahme einen kleineren Durchmesser hat als der an der Vorderseite ausgebildete Kolben.

Vorzugsweise ist die Hülse als Hohlzylinder ausgebildet ist, wobei die Vorderseite der Hülse auf dem Monomerflüssigkeitsbehälter aufliegt und wobei die Hülse im Inneren des Hohlraums oder am Ende der Hülse durch eine gasdichte Trennwand verschlossen ist. Wesentlich für die Funktionalität der Vorrichtung ist, dass die Hülse gasdicht axial in der hohlzylinderförmigen Kammer der Monomeraufnahme verschoben werden kann.

Die Innenfläche der Verschlusskappe und die in Richtung des Kartuschendeckels zeigende Fläche der vorderen Grundfläche der Monomeraufnahme (des Kolbens) sind vorzugsweise konkav gewölbt.

Vorteilhaft ist es, wenn ein oder mehrere frei bewegliche Mischkörper in dem Innenraum der Kartusche angeordnet sind, wobei kugelförmige Mischkörper bevorzugt sind und wobei besonders keramische kugelförmige Mischkörper bevorzugt sind.

An der Innenseite des Kartuschendeckels sind vorzugsweise Stege angeordnet, die auf die Austragsöffnung zulaufen.

Ein beispielhaftes erfindungsgemäßes Verfahren zur Vermischung und Applizieren von Polymethylmethacrylat-Knochenzement mit der beispielhaften erfindungsgemäßen Vorrichtung, kann durch folgende nacheinander ablaufende Schritte gekennzeichnet sein:
a) senkrechte Positionierung der Kartusche mit dem Kartuschendeckel nach unten,
b) Schrauben der auf der hohlzylinderförmigen Monomeraufnahme angeschraubten Verschlusskappe in Richtung Kartuschendeckel,
c) Verschieben der Hülse in Richtung Kartuschendeckel durch Einschrauben der Verschlusskappe,
d) Verschließen der wenigstens einen Begasungsöffnung in der hohlzylinderförmigen Monomeraufnahme durch die Hülse,
e) Verschieben des Monomerflüssigkeitsbehälters in Richtung Stechdorn durch axiales Verschieben der Hülse,
f) Zerstörung des Bodens des Monomerflüssigkeitsbehälters durch den Stechdorn,
g) Ausfließen der Monomerflüssigkeit in die Kammer der hohlzylinderförmigen Monomeraufnahme,
h) Hinausschrauben der Monomeraufnahme vom Kartuschendeckel weg und dadurch Ausbilden eines Unterdrucks in dem Innenraum der Kartusche,
i) Fließen und Einsaugen der Monomerflüssigkeit durch den Durchgang in der vorderen Grundfläche der Monomeraufnahme in den Innenraum der Kartusche zum dort angeordneten Polymethylmethacrylat-Zementpulver,
j) Manuelles Schütteln der Kartusche und dadurch Vermischen der Monomerflüssigkeit mit dem Zementpulver,
k) Bilden des Knochenzementteigs aus der mit dem Polymethylmethacrylat-Zementpulver vermischten Monomerflüssigkeit,
l) Entfernen des Verschlussstopfens aus der Austragsöffnung,
m) Schrauben der Monomeraufnahme in Richtung des Kartuschendeckels, und
n) Herauspressen des Polymethylmethacrylat-Knochenzementteigs durch die Bewegung des Kolbens der Monomeraufnahme in Richtung des Kartuschendeckels aus der geöffneten Austragsöffnung.

Im Folgenden werden weitere Ausführungsbeispiele der Erfindung anhand von zwölf schematisch dargestellten Figuren erläutert, ohne jedoch dabei die Erfindung zu beschränken. Dabei zeigt:
Figur 1: eine schematische Querschnittansicht einer beispielhaften ersten erfindungsgemäßen Vorrichtung zum Herstellen eines Knochenzementteigs;
Figur 2: eine schematische perspektivische Außenansicht der ersten erfindungsgemäßen Vorrichtung nach Figur 1;
Figur 3: eine weitere schematische perspektivische Querschnittansicht der ersten erfindungsgemäßen Vorrichtung nach den Figuren 1 und 2 mit einem zweiten Sicherungselement;
Figur 4: eine schematische Querschnittansicht der ersten erfindungsgemäßen Vorrichtung nach den Figuren 1 bis 3 mit eingeschraubter Monomeraufnahme zur Verdeutlichung des Ablaufs eines erfindungsgemäßen Verfahrens;
Figur 5: eine schematische Querschnittansicht der ersten erfindungsgemäßen Vorrichtung nach den Figuren 1 bis 4 mit geöffnetem Monomerflüssigkeitsbehälter zur Verdeutlichung des Ablaufs des erfindungsgemäßen Verfahrens;
Figur 6: eine schematische Querschnittansicht der ersten erfindungsgemäßen Vorrichtung nach den Figuren 1 bis 5 mit herausgeschraubter Monomeraufnahme und eingezogener Monomerflüssigkeit zur Verdeutlichung des Ablaufs des erfindungsgemäßen Verfahrens;
Figur 7: eine schematische Querschnittansicht der ersten erfindungsgemäßen Vorrichtung nach den Figuren 1 bis 6 mit eingeschraubter Monomeraufnahme und herausgedrücktem überschüssigem Gas zur Verdeutlichung des Ablaufs des erfindungsgemäßen Verfahrens;
Figur 8: eine schematische Querschnittansicht der ersten erfindungsgemäßen Vorrichtung nach den Figuren 1 bis 7 beim Austragen des hergestellten Knochenzementteigs zur Verdeutlichung des Ablaufs des erfindungsgemäßen Verfahrens;
Figur 9: eine schematische Querschnittansicht einer beispielhaften zweiten erfindungsgemäßen Vorrichtung zum Herstellen eines Knochenzementteigs;
Figur 10: eine schematische perspektivische Schnittansicht der zweiten erfindungsgemäßen Vorrichtung nach Figur 9 zum Herstellen eines Knochenzementteigs ohne die Ausgangskomponenten;
Figur 11: eine schematische Querschnittansicht einer beispielhaften dritten erfindungsgemäßen Vorrichtung zum Herstellen eines Knochenzementteigs; und
Figur 12: eine schematische Querschnittansicht der dritten erfindungsgemäßen Vorrichtung nach Figur 11 beim Austragen des hergestellten Knochenzementteigs.

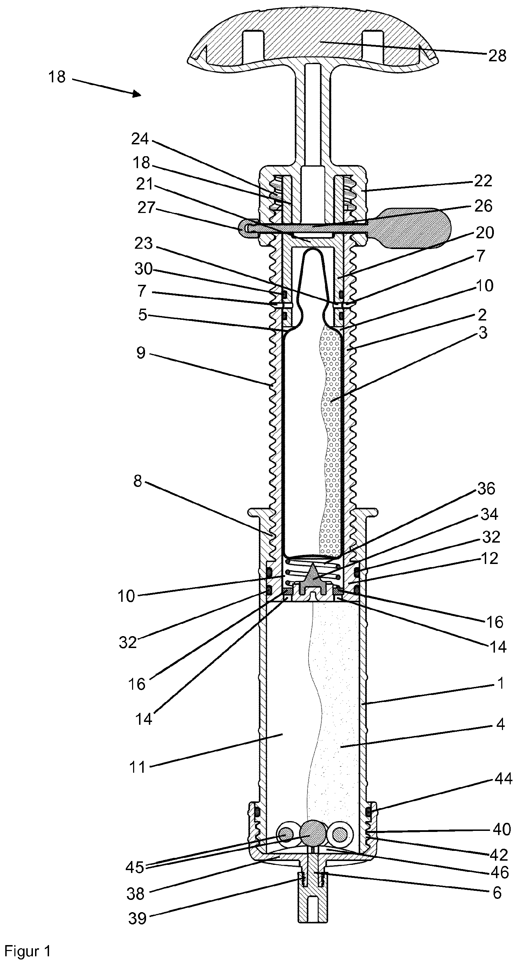

In den Figuren 1 bis 8 sind Abbildungen einer ersten erfindungsgemäßen Vorrichtung zum Lagern der Ausgangskomponenten 3, 4 eines Knochenzementteigs 49 und zum Mischen des Knochenzementteigs 49 gezeigt. Die Figuren 1 und 4 bis 8 zeigen dabei den Ablauf eines erfindungsgemäßen Verfahrens durchgeführt mit der ersten erfindungsgemäßen Vorrichtung, die jeweils als Querschnittansichten dargestellt sind.

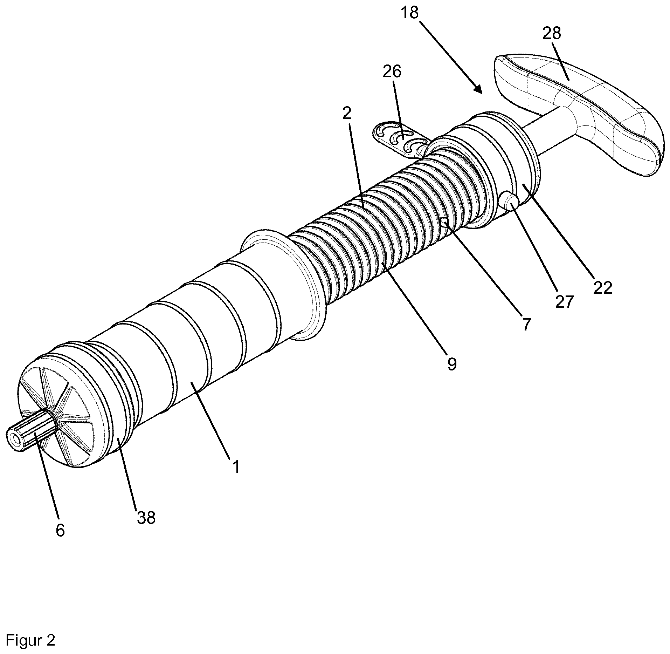

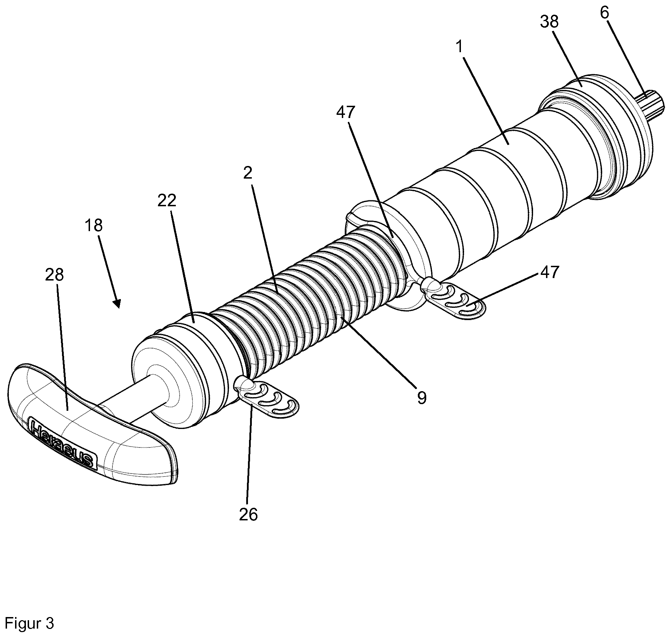

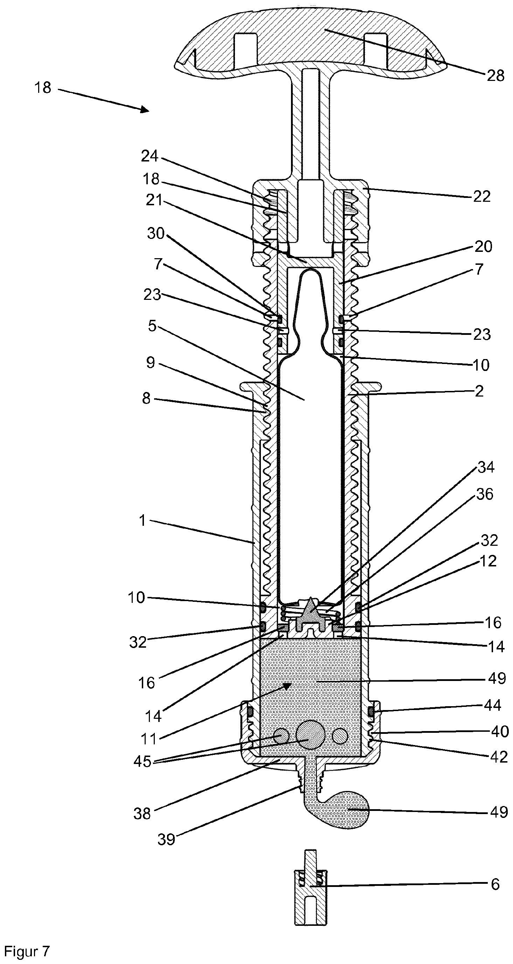

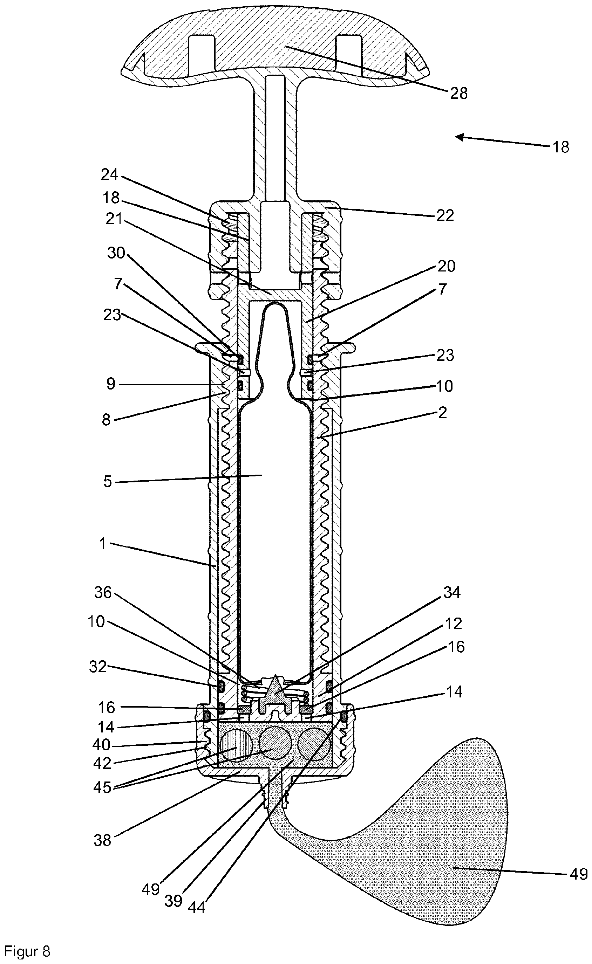

Die erste erfindungsgemäße Vorrichtung weist eine röhrenförmige Kartusche 1 aus Kunststoff auf, die einen vorderen Teil (in den Figuren 1 und 4 bis 8 unten, in Figur 2 rechts oben und in Figur 3 links unten) der Vorrichtung bildet. Ein rückseitiger, hinterer Teil der Vorrichtung wird durch eine Monomeraufnahme 2 gebildet. Die Vorrichtung ist zur Herstellung eines Knochenzementteigs 49 (siehe Figur 7 und 8) vorgesehen, der aus einer Monomerflüssigkeit 3 und aus einem Zementpulver 4 hergestellt wird. Die Monomerflüssigkeit 3 ist dazu in einer aufbrechbaren Ampulle 5 aus Glas oder aus Kunststoff als Monomerflüssigkeitsbehälter für die Monomerflüssigkeit 3 enthalten, wobei die Ampulle 5 in der Monomeraufnahme 2 steckt. Die Kartusche 1 bildet in ihrem Inneren einen zylindrischen Innenraum 11, in dem das Zementpulver 4 enthalten ist.

Die Kartusche 1 weist an ihrer Vorderseite (in den Figuren 1 und 4 bis 8 unten, in Figur 2 rechts oben und in Figur 3 links unten) eine Austragsöffnung auf, die zunächst mit einem herausnehmbaren Verschluss 6 verschlossen ist. In der seitlichen Wandung der Monomeraufnahme 2 befinden sich mehrere Begasungsöffnungen 7, durch die ein Gas aus dem Inneren der Vorrichtung abgesaugt werden kann und durch die ein sterilisierendes Gas wie Ethylenoxid eingefüllt werden kann, um das Innere der Vorrichtung zu sterilisieren.

Am rückseitigen Ende der Kartusche 1 befindet sich ein Innengewinde 8. Die Monomeraufnahme 2 weist an ihrer Außenseite ein zu dem Innengewinde 8 der Kartusche 1 passendes Außengewinde 9 auf. Die Monomeraufnahme 2 ist nach Art eines Gewinderohrs geformt und weist in ihrem Inneren eine zylindrische Kammer 10 auf, in der die Ampulle 5 steckt. Dazu weist die Ampulle 5 einen zylindrischen Ampullenkörper mit passendem Durchmesser auf. Im Inneren der Kartusche 1 bildet die Kartusche 1 einen zylindrischen Innenraum 11. Die Zylindergeometrie des Innenraums 11 und der Kammer 10 entsprechen Zylindern mit kreisförmiger Grundfläche.

Die Monomeraufnahme 2 ist an ihrer Vorderseite durch einen zylindrischen Kolben 12 begrenzt, der die Kammer 10 nach vorne an ihrer kreisförmigen Grundfläche verschließt. Der Kolben 12 weist mehrere Kanäle 14 als Durchgang durch den Kolben 12 auf, die ringförmig in dem Kolben 12 angeordnet sind und die Vorderseite des Kolbens 12 mit der Rückseite des Kolbens 12 und dadurch die Kammer 10 der Monomeraufnahme 2 mit dem Innenraum 11 der Kartusche 1 verbinden. Die Kanäle 14 sind mit einem ringförmigen Porenfilter 16 abgedeckt. Der Porenfilter 16 ist für das Zementpulver 4 aus dem Innenraum 11 der Kartusche 1 undurchlässig und für die Monomerflüssigkeit 3 und Gase durchlässig. Dadurch wird ein Vordringen des Zementpulvers 4 in die Kammer 10 der Monomeraufnahme 2 verhindert. Der Kolben 12 weist einen größeren Außendurchmesser auf, als das Außengewinde 9 der Monomeraufnahme 2. Der Außendurchmesser des zylindrischen Kolbens 12 passt zum Innendurchmesser des Innenraums 11 der Kartusche 1. Beim Zusammenbau der Vorrichtung muss die Monomeraufnahme 2 von vorne in die Kartusche 1 eingesteckt werden und mit dem Außengewinde 9 in das Innengewinde 8 der Kartusche 1 eingeschraubt werden. Der Kolben 12 der Monomeraufnahme 2 dichtet den Innenraum 11 der Kartusche 1 in Richtung der Rückseite (in den Figuren 1 und 4 bis 8 oben, in Figur 2 links unten und in Figur 3 rechts oben) ab.

An der Rückseite der Monomeraufnahme 2 ist eine Öffnungseinrichtung 18 vorgesehen, mit der die Ampulle 5 in Richtung des Kolbens 12 gedrückt werden kann, um die Ampulle 5 zu öffnen und die Monomerflüssigkeit 3 im Inneren der Kammer 10 zu öffnen. Die Öffnungseinrichtung 18 weist hierzu einen Hohlzylinder 20 auf, der nach Art einer Hülse geformt ist. Der Hohlzylinder 20 liegt dabei an der Innenwand der Kammer 10 an und deckt diese im Bereich der Rückseite der Kammer 10 ab. Im Hohlzylinder 20 ist eine geschlossene Wandung 21 senkrecht zur Achse der Zylindergeometrie des Hohlzylinders 20 vorgesehen, so dass der Hohlzylinder 20 mit der geschlossenen Wandung 21 die Kammer 10 an ihrer Rückseite nach außen abschließt. Der Hohlzylinder 20 ist an einer schraubbaren Verschlusskappe 22 befestigt. In der seitlichen Zylinderwandung (der Zylindermantelfläche) des Hohlzylinders 20 sind radiale Bohrungen 23 vorgesehen, die im Ausgangszustand und Lagerungszustand der Vorrichtung (siehe Figur 1) mit den Begasungsöffnungen 7 fluchten. Dadurch ist die Kammer 10 und dadurch über die Kanäle 14 auch der Innenraum 11 der Kartusche 1 und damit das Zementpulver 4 in diesem Zustand gasdurchlässig mit der Umgebung der Vorrichtung verbunden. Die Verschlusskappe 22 weist ein Innengewinde 24 auf, das zu dem Außengewinde 9 des Monomeraufnahme 2 passt.

Die Verschlusskappe 22 beziehungsweise die Öffnungseinrichtung 18 ist ein Stück aber nicht bis zu einem Anschlag auf die Rückseite der Monomeraufnahme 2 aufgeschraubt und so an dieser befestigt. Es ist wichtig, dass die Verschlusskappe 22 noch weiter auf die Monomeraufnahme 2 aufgeschraubt werden kann und dadurch der Hohlzylinder 20 tiefer in die Kammer 10 eingeschoben werden kann.

An der Rückseite der Monomeraufnahme 2 und hinter der geschlossenen Wandung 21 des Hohlzylinders 20 ist eine durchgehende Bohrung vorgesehen, durch die ein Sicherungselement 26 in Form eines Stiftes durchgesteckt und mit einer Halterungskappe 27 vor dem Herausfallen gesichert ist. Das Sicherungselement 26 verhindert ein ungewolltes Schrauben der Verschlusskappe 22 und damit ein ungewolltes Bedienen der Öffnungseinrichtung 18. Das Sicherungselement 26 kann unmittelbar vor einer Anwendung der Vorrichtung gelöst werden, indem die Halterungskappe 27 abgezogen und der Stift herausgezogen wird. Danach kann die Öffnungseinrichtung 18 in die Kammer 10 geschraubt werden.

Beim Einschrauben der Öffnungseinrichtung 18 werden die Bohrungen 23 von den Begasungsöffnungen 7 weggedreht und in Längsrichtung verschoben und durch die Außenwand des Hohlzylinders 20 verschlossen. Dadurch wird die Vorrichtung nach außen abgeschlossen, so dass die in die Kammer 10 austretende Monomerflüssigkeit 3 nicht aus der Kammer 10 durch die Begasungsöffnungen 7 heraustreten kann.

Um eine Drehung der Verschlusskappe 22 in die falsche Richtung und dadurch ein Öffnen der Kammer 10 an deren Rückseite zu verhindern, ist eine Rückdrehsicherung vorgesehen (in den Figuren 1 bis 8 nicht zu sehen). Die Rückdrehsicherung verhindert ein Lösen der Verschlusskappe 22 beziehungsweise ein Lösen der Öffnungseinrichtung 18 von der Monomeraufnahme 2. Die Rückdrehsicherung kann beispielsweise als Schraubensicherung in Form einer Sperrkantscheibe oder durch ein Keilsicherungsscheibenpaar oder ähnliche Maßnahmen realisiert werden. Hiermit wird nicht nur ein Lösen der Öffnungseinrichtung verhindert, sondern es kann bei geeignetem Aufbau der Rückdrehsicherung auch verhindert werden, dass die Begasungsöffnungen 7 durch ein Zurückschrauben der Öffnungseinrichtung 18 wieder geöffnet werden können.

Um die Öffnungseinrichtung 18 bequem mit der Hand drehen zu können, ist deren rückseitiges Ende mit einem Griff 28 ausgestattet. Um die Begasungsöffnung 7 gasdicht und druckdicht schließen zu können und um den Hohlzylinder 20 gegen die Innenwand der Kammer 10 abzudichten, sind zwei umlaufende Dichtungen 30 aus Gummi in umlaufenden Nuten am Außenumfang des Hohlzylinders 20 angeordnet. Die radialen Bohrungen 23 sind zwischen den in Längsrichtung zueinander beabstandeten Dichtungen 30 angeordnet. Die rückseitige der beiden Dichtungen 30 ist dicht neben den Bohrungen 23 angeordnet, so dass die Begasungsöffnungen 7 bei einer axialen Bewegung des Hohlzylinders 20 in Längsrichtung schnell geschlossen werden. Alternativ können auch Dichtungsringe um die Begasungsöffnungen 7 an der Innenwand der Monomeraufnahme 2 herum oder um die Bohrungen 23 herum an der Außenwand des Hohlzylinders 20 angeordnet sein. Die Begasungsöffnungen 7 werden von der hinteren Dichtung (in Figur 1 oben) abgedichtet, wenn der Hohlzylinder 20 in Richtung des Kolbens 12 geschoben wird (siehe Figur 3).

In gleicher Weise befinden sich am Außenumfang des Kolbens 12 zwei in Längsrichtung voneinander beabstandete Nuten, in denen sich zwei umlaufende Dichtungen 32 aus Gummi befinden. Die Dichtungen 32 dichten den Kolben 12 gegen den Innenraum 11 der Kartusche 1 ab und verschließen die Rückseite des Innenraums 11 der Kartusche 1.

Die Kanäle 14 und der ringförmige Porenfilter 16 sind um einen Dorn 34 zum Aufbrechen der Ampulle 5 herum angeordnet. Der Dorn 34 weist hierzu ins Innere der Kammer 10. Die Ampulle 5 kann hierzu mit dem Hohlzylinder 20 auf den Dorn 34 gedrückt werden, bis der Boden der Ampulle 5 aufbricht. Der Hohlzylinder 20 weist dazu in etwa den gleichen Durchmesser wie der Ampullenkörper der Ampulle 5 auf. Ein Ampullenkopf der Ampulle 5 ist dabei im Inneren des Hohlzylinders 20 angeordnet. Damit wird erreicht, dass die Ampulle 5 nicht im Bereich des Hohlzylinders 20 gebrochen wird, weil der zylindrische Ampullenkörper sehr stabil ist, während der Dorn 34 relativ leicht in den Boden der Ampulle 5 gedrückt werden kann.

Damit die Ampulle 5 nicht schon bei Transport der Vorrichtung geöffnet wird, ist um den Dorn 34 herum eine Feder 36 in der Kammer 10 angeordnet, die die Ampulle 5 von dem Dorn 34 beabstandet. Beim Einschrauben der Öffnungseinrichtung 18 wird die Feder 36 von der Ampulle 5 komprimiert und der Boden der Ampulle 5 am Dorn 34 aufgebrochen.

Die Vorderseite der Kartusche 1 ist mit einem Kartuschendeckel 38 verschlossen. In der Mitte des Kartuschendeckels 38 ist ein Stutzen 39 ausgeformt, der die Austragsöffnung an der Vorderseite begrenzt. An dem Stutzen 39 ist der Stopfen 6 lösbar befestigt, der die Austragsöffnung verschließt. Der Kartuschendeckel 38 ist mit einem Innengewinde 40 auf ein Außengewinde 42 an der Vorderseite der Kartusche 1 geschraubt. Der Kartuschendeckel 38 ist zusätzlich über eine umlaufende Dichtung 44 gegen die Kartusche 1 abgedichtet.

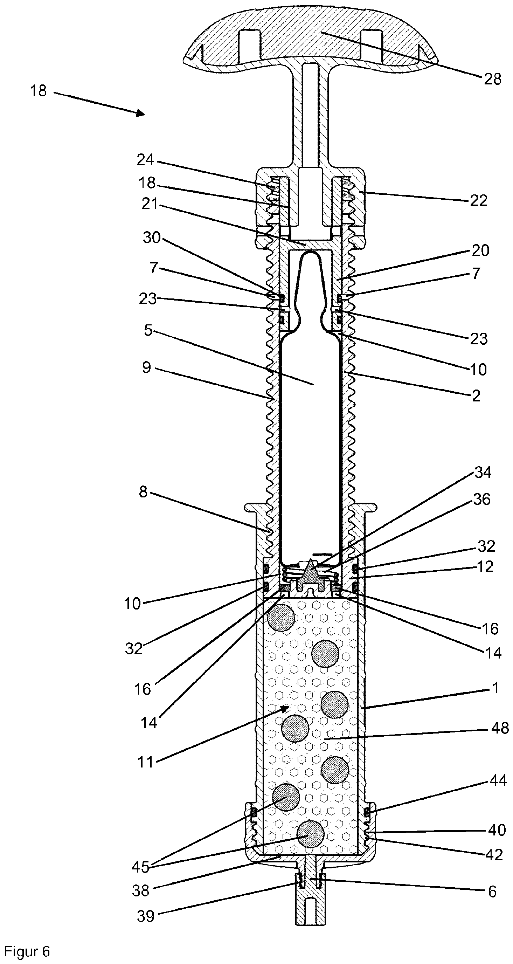

Im Innenraum der Kartusche befinden sich sieben lose Kugeln 45 aus Zirkoniumdioxid-Keramik als Mischelemente, mit denen der Inhalt im Innenraum 11 der Kartusche 1 durch schütteln der Vorrichtung durchmischt werden kann. Dadurch, dass die Kugeln 45 eine größere Dichte haben als der Knochenzementteig 49, können diese in dem Knochenzementteig 49 und noch wesentlich besser in einer Knochenzementteig-Gas-Mischung 48 (siehe Figur 6) durch Schütteln der Vorrichtung gegen den Knochenzementteig 49 beziehungsweise die Knochenzementteig-Gas-Mischung 48 bewegt werden. Die Kugeln 45 fliegen dabei in dem Innenraum 11 der Kartusche 1 umher und durchmischen dabei die Ausgangskomponenten 3, 4.

Damit die Kugeln 45 die Austragsöffnung nicht verschließen können, sind an der Innenseite des Kartuschendeckels 38 mehrere vorspringende Stege 46 vorgesehen, die sich in radialer Richtung vom Rand der Innenwand des Innenraum 11 aus in Richtung der Austragsöffnung erstrecken. Die Stege 46 sind in radialer Richtung nach außen hin abfallend, so dass die Kugeln 45 beim Vortreiben der Kolbens 12 und des Knochenzementteigs 49 nach außen von der Austragsöffnung weg gleiten oder rollen (siehe Figur 7 und 8). Wenn eine Kugel 45 genau mittig vor der Austragsöffnung sitzt, so ist diese auch dann von den Stegen 46 von der Austragsöffnung beabstandet, so dass der Knochenzementteig 49 zwischen der Kugel 45 und den Stegen 46 hindurch in und durch die Austragsöffnung fließen kann.

Ein zweites Sicherungselement 47 in Form einer Spange 47 kann am Übergang von der Monomeraufnahme 2 in die Kartusche 1 angeordnet sein. Mit der Spange 47 kann ein Einschrauben der Monomeraufnahme 2 in die Kartusche 1 hinein verhindert werden. Die Spange 47 wird vor dem Einschrauben der Monomeraufnahme 2 in die Kartusche 1 abgezogen. Die Spange 47 ist nicht besonders wichtig und kann auch weggelassen werden.

Im Folgenden wird der Ablauf eines erfindungsgemäßen Verfahrens anhand der Figuren 1 bis 8 erläutert. Zunächst befindet sich die Vorrichtung im Ausgangszustand (siehe Figuren 1 bis 3). In diesem Zustand ist die Vorrichtung verpackt und mit Ethylenoxid sterilisiert worden. Das Ethylenoxid kann durch die Begasungsöffnungen 7 und die Bohrungen 23 in die Kammer 10 und durch den Porenfilter 16 und die Kanäle 14 in den Innenraum 11 der Kartusche 1 gelangen. Der Gasaustausch erfolgt dabei in einer Vakuumkammer oder in einer Unterdruckkammer. In diesem Zustand (siehe Figur 3) wird die Vorrichtung ausgepackt.

Zunächst wird die Spange 47 abgezogen. Dies kann aber auch später geschehen. Die Vorrichtung befindet sich nun in dem in den Figuren 1 und 2 gezeigten Zustand. Anschließend wird die Monomeraufnahme 2 in die Kartusche 1 hineingeschraubt. Dabei wird der Gasüberstand aus dem Innenraum 11 der Kartusche 1 durch den durch die Kanäle 14 gebildeten Durchgang und durch den Porenfilter 16 in die Kammer 10 gedrückt. Das Gas aus der Kammer entweicht schließlich durch die Bohrungen 23 und die Begasungsöffnungen 7.

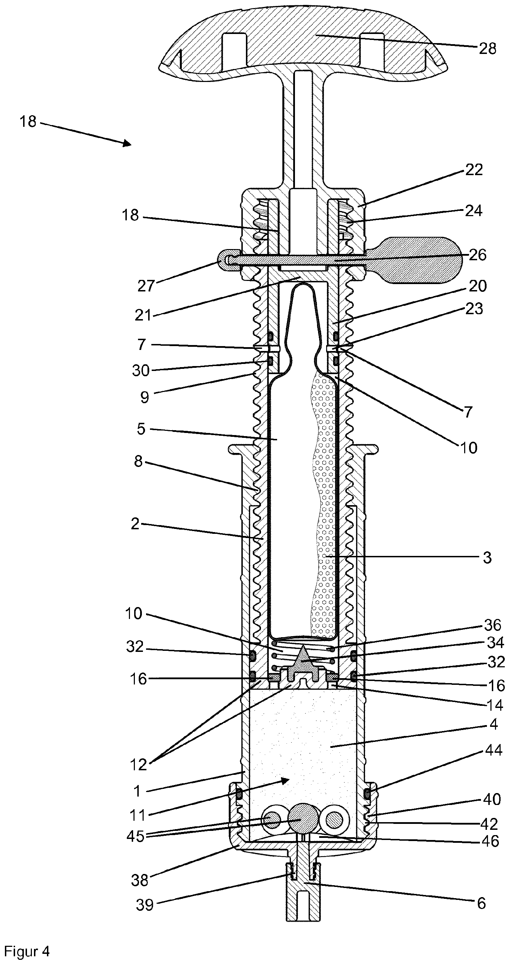

Schließlich ist das Zementpulver 4 in dem Innenraum 11 der Kartusche 1 komprimiert und nur noch zwischen den Pulverpartikeln Gas enthalten. Dieser Zustand ist in Figur 4 gezeigt.

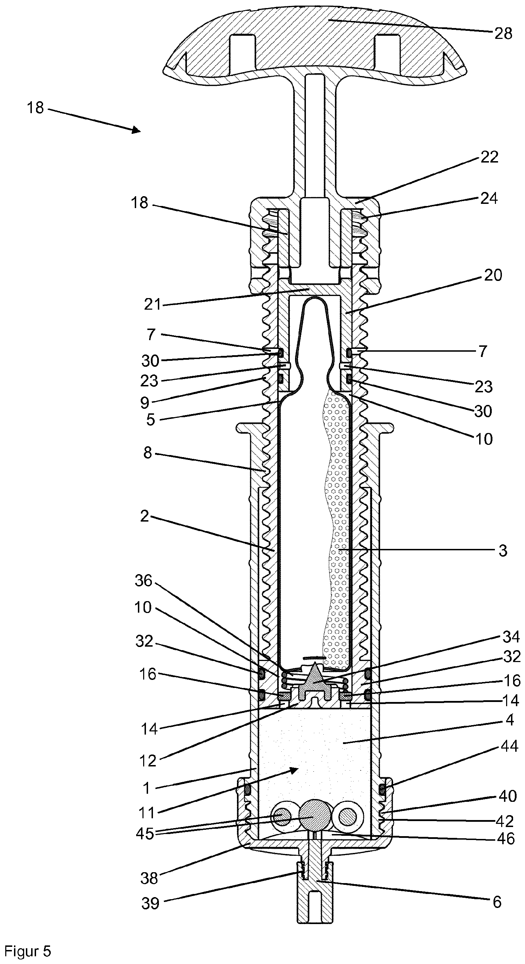

In einem nächsten Schritt wird das Sicherungselement 26 entfernt und die Öffnungseinrichtung 18 in die Kammer 10 geschraubt. Hierbei wird die Vorrichtung vorzugsweise mit dem Kartuschendeckel 38 nach unten gehalten. Dabei drückt der Hohlzylinder die Ampulle 5 an deren Schultern gegen die Kraft der Feder 36 in Richtung des Dorns 34. Die Begasungsöffnungen 7 werden durch die Schraubbewegung von dem Hohlzylinder 20 verschlossen. Anschließend wird die Ampulle 5 mit dem Boden auf den Dorn 34 gedrückt und die Ampulle 5 bricht am Boden auf. Dieser Zustand ist in Figur 5 gezeigt.

Die Monomerflüssigkeit 3 tritt am Boden der Ampulle 5 im Bereich des durch die Kanäle 14 gebildeten Durchgangs aus. Da die Vorrichtung mit dem Kartuschendeckel 38 nach unten gehalten wird, fließt die Monomerflüssigkeit 3 getrieben von der Schwerkraft sofort nach unten durch den Porenfilter 16 und die Kanäle 14 in den Innenraum 11 der Kartusche 1 und verteilt sich in dem Zementpulver 4. Um den Monomertransfer zu beschleunigen wird die Monomeraufnahme 2 wieder aus der Kartusche 1 herausgeschraubt. Da die Begasungsöffnungen 7 gasdicht und druckdicht verschlossen sind und der Innenraum 11 der Kartusche 1 gegen den Kolben 12 abgedichtet und nach außen geschlossen ist, wird durch die Vergrößerung des Volumens des Innenraums 11 der Kartusche 1 ein Unterdruck in dem Innenraum 11 der Kartusche 1 erzeugt, mit dem die Monomerflüssigkeit 3 in den Innenraum 11 der Kartusche 1 gesaugt wird. Auch hierbei wird die Vorrichtung mit dem Kartuschendeckel 38 nach unten gehalten. Zudem wird durch die Vergrößerung des Innenraums 11 der Kartusche 1 der Gasdruck im Inneren der Vorrichtung verringert. Das Gas im Unterdruck steht schließlich auch im Innenraum 11 der Kartusche 1 über den Ausgangskomponenten 3, 4. Das Volumen des überstehenden Gases ist dabei zumindest doppelt so groß wie das Volumen der Monomerflüssigkeit 3.

In diesem Zustand kann der Inhalt des Innenraums 11 der Kartusche 1 nämlich die Monomerflüssigkeit 3 und das Zementpulver 4 durch Schütteln der Vorrichtung gemischt werden. Dabei fliegen die Kugeln 45 in dem Innenraum 11 der Kartusche 1 umher und unterstützen dabei die Vermischung der Komponenten. Im Innenraum 11 der Kartusche 1 befindet sich dann das Knochenzementteig-Gas-Gemisch 48. Dieser Zustand ist in Figur 6 gezeigt. Nach mehrmaligem Schütteln ist der Knochenzementteig 49 gut gemischt.

Die Vorrichtung wird nun umgedreht, so dass der Kartuschendeckel 38 nach oben weist. Der Stopfen 6 wird aus der Austragsöffnung entfernt. Jetzt kann optional ein Schlauch mit einem Trokar (nicht gezeigt) an dem Stutzen 39 befestigt werden, durch die der Knochenzementteig 49 an schwer zugänglichen Orten unter Röntgenkontrolle appliziert werden kann. Da der Knochenzementteig 49 für die Anwendung bei der Spondylodese eher dünnflüssig ist, steigen die Gasblasen nach oben. Die Monomeraufnahme 2 wird wieder in die Kartusche 1 hineingeschraubt und das Gas entweicht nach oben aus der Austragsöffnung. Schließlich tritt der Knochenzementteig 49 aus der Austragsöffnung beziehungsweise durch den Stutzen 39 aus dem Innenraum 11 der Kartusche 1 aus. Dieser Zustand ist in Figur 7 gezeigt.

Durch weiteres Einschrauben der Monomeraufnahme 2 in die Kartusche 1 wird der Knochenzementteig 49 mit dem Kolben 12 aus der Vorrichtung herausgepresst. Schließlich drückt der Kolben 12 die Kugeln 45 gegen den Kartuschendeckel 38. Der Auspressvorgang ist damit beendet. Dieser Zustand ist in Figur 8 gezeigt.

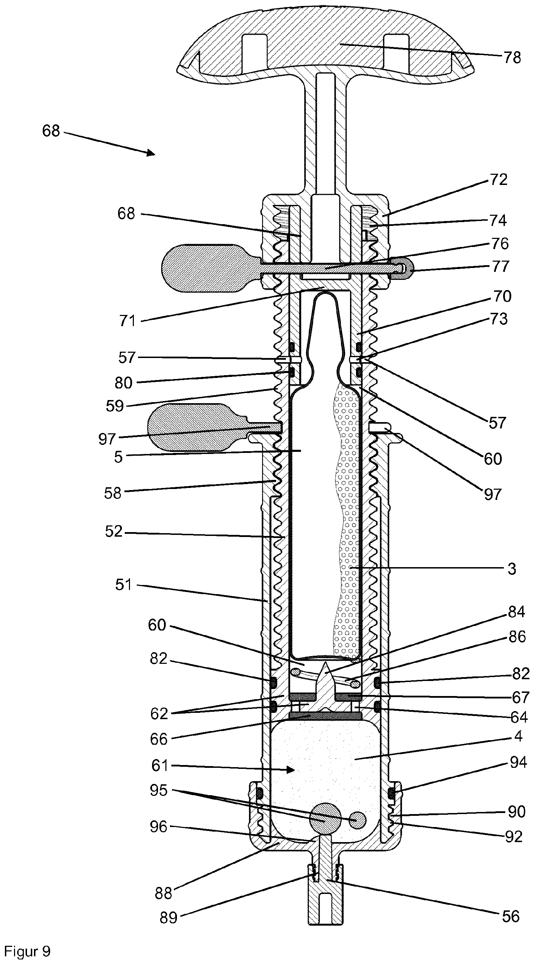

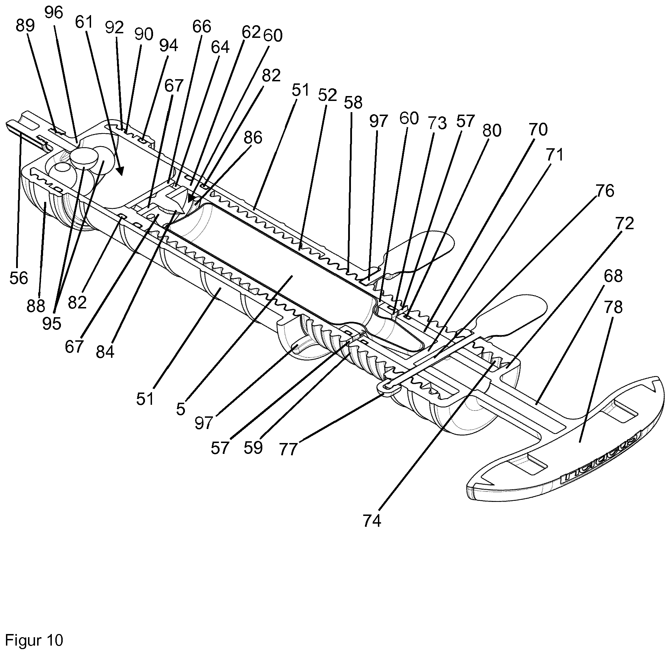

In den Figuren 9 und 10 sind Abbildungen einer zweiten erfindungsgemäßen Vorrichtung zum Lagern der Ausgangskomponenten 3, 4 eines Knochenzementteigs und zum Mischen des Knochenzementteigs gezeigt. Dabei zeigt Figur 9 eine Querschnittansicht im Ausgangszustand und Figur 10 eine perspektivische Querschnittansicht der Vorrichtung ohne die enthaltenen Ausgangskomponenten.

Die zweite erfindungsgemäße Vorrichtung weist eine röhrenförmige Kartusche 51 aus Kunststoff auf, die einen vorderen Teil (in Figur 9 unten und in Figur 10 links oben) der Vorrichtung bildet. Ein rückseitiger, hinterer Teil der Vorrichtung wird durch eine Monomeraufnahme 52 gebildet. Die Vorrichtung ist zur Herstellung eines Knochenzementteigs vorgesehen, der aus einer Monomerflüssigkeit 3 und aus einem Zementpulver 4 hergestellt wird. Die Monomerflüssigkeit 3 ist dazu in einer aufbrechbaren Ampulle 5 aus Glas oder aus Kunststoff als Monomerflüssigkeitsbehälter für die Monomerflüssigkeit 3 enthalten, wobei die Ampulle 5 in der Monomeraufnahme 52 steckt. Die Kartusche 51 bildet in ihrem Inneren einen zylindrischen Innenraum 61, in dem das Zementpulver 4 enthalten ist.

Die Kartusche 51 weist an ihrer Vorderseite (in Figur 9 unten und in Figur 10 links oben) eine Austragsöffnung auf, die zunächst mit einem herausnehmbaren Verschluss 56 verschlossen ist. In der seitlichen Wandung der Monomeraufnahme 52 befinden sich mehrere Begasungsöffnungen 57, durch die ein Gas aus dem Inneren der Vorrichtung abgesaugt werden kann und durch die ein sterilisierendes Gas wie Ethylenoxid eingefüllt werden kann, um das Innere der Vorrichtung zu sterilisieren.

Am rückseitigen Ende der Kartusche 51 befindet sich ein Innengewinde 58. Die Monomeraufnahme 52 weist an ihrer Außenseite ein zu dem Innengewinde 58 der Kartusche 51 passendes Außengewinde 59 auf. Die Monomeraufnahme 52 ist nach Art eines Gewinderohrs geformt und weist in ihrem Inneren eine zylindrische Kammer 60 auf, in der die Ampulle 5 steckt. Dazu weist die Ampulle 5 einen zylindrischen Ampullenkörper mit passendem Durchmesser auf. Im Inneren der Kartusche 51 bildet die Kartusche 51 den zylindrischen Innenraum 61. Die Zylindergeometrie des Innenraums 61 und der Kammer 60 entsprechen Zylindern mit kreisförmiger Grundfläche.

Die Monomeraufnahme 52 ist an ihrer Vorderseite durch einen zylindrischen Kolben 62 begrenzt, der die Kammer 60 nach vorne an ihrer kreisförmigen Grundfläche verschließt. Der Kolben 62 weist mehrere Kanäle 64 als Durchgang durch den Kolben 62 auf, die ringförmig in dem Kolben 62 angeordnet sind und die Vorderseite des Kolbens 62 mit der Rückseite des Kolbens 62 und dadurch die Kammer 60 der Monomeraufnahme 52 mit dem Innenraum 61 der Kartusche 51 verbinden. Die Kanäle 64 sind mit einem kreisscheibenförmigen Porenfilter 66 und mit einem ringförmigen Gitter als Splitterschutz 67 abgedeckt. Der Porenfilter 66 ist für das Zementpulver 4 aus dem Innenraum 61 der Kartusche 51 undurchlässig und für die Monomerflüssigkeit 3 und Gase durchlässig. Dadurch wird ein Vordringen des Zementpulvers 4 in die Kammer 60 der Monomeraufnahme 2 verhindert. Der Splitterschutz 67 kann durch ein Gitter realisiert werden. Mit dem Splitterschutz 67 wird ein Vordringen von Splittern der geöffneten Ampulle 5 in die Kanäle 64 des Durchgangs verhindert. Der Kolben 62 weist einen größeren Außendurchmesser auf, als das Außengewinde 59 der Monomeraufnahme 52. Der Außendurchmesser des zylindrischen Kolbens 62 passt zum Innendurchmesser des Innenraums 61 der Kartusche 51. Beim Zusammenbau der Vorrichtung muss die Monomeraufnahme 52 von vorne in die Kartusche 51 eingesteckt werden und mit dem Außengewinde 59 in das Innengewinde 58 der Kartusche 51 eingeschraubt werden. Der Kolben 62 der Monomeraufnahme 52 dichtet den Innenraum 61 der Kartusche 51 in Richtung der Rückseite (in Figur 9 oben und in Figur 10 rechts unten) ab.

An der Rückseite der Monomeraufnahme 52 ist eine Öffnungseinrichtung 68 vorgesehen, mit der die Ampulle 5 in Richtung des Kolbens 62 gedrückt werden kann, um die Ampulle 5 zu öffnen und die Monomerflüssigkeit 3 im Inneren der Kammer 60 zu öffnen. Die Öffnungseinrichtung 68 weist hierzu einen Hohlzylinder 70 auf, der nach Art einer Hülse geformt ist. Der Hohlzylinder 70 liegt dabei an der Innenwand der Kammer 60 an und deckt diese im Bereich der Rückseite der Kammer 60 ab. Im Hohlzylinder 70 ist eine geschlossene Wandung 71 senkrecht zur Achse der Zylindergeometrie des Hohlzylinders 70 vorgesehen, so dass der Hohlzylinder 70 mit der geschlossenen Wandung 71 die Kammer 60 an ihrer Rückseite nach außen abschließt. Der Hohlzylinder 70 ist an einer schraubbaren Verschlusskappe 72 befestigt. In der seitlichen Zylinderwandung (der Zylindermantelfläche) des Hohlzylinders 70 sind radiale Bohrungen 73 vorgesehen, die im Ausgangszustand und Lagerungszustand der Vorrichtung (siehe Figur 9 und 10) mit den Begasungsöffnungen 57 fluchten. Dadurch ist die Kammer 60 und dadurch über die Kanäle 64 auch der Innenraum 61 der Kartusche 51 und damit das Zementpulver 4 in diesem Zustand gasdurchlässig mit der Umgebung der Vorrichtung verbunden. Die Verschlusskappe 72 weist ein Innengewinde 74 auf, das zu dem Außengewinde 59 des Monomeraufnahme 52 passt.

Die Verschlusskappe 72 beziehungsweise die Öffnungseinrichtung 68 ist ein Stück aber nicht bis zu einem Anschlag auf die Rückseite der Monomeraufnahme 52 aufgeschraubt und so an dieser befestigt. Es ist wichtig, dass die Verschlusskappe 72 noch weiter auf die Monomeraufnahme 52 aufgeschraubt werden kann und dadurch der Hohlzylinder 70 tiefer in die Kammer 60 eingeschoben werden kann.

An der Rückseite der Monomeraufnahme 52 und hinter der geschlossenen Wandung 71 des Hohlzylinders 70 ist eine durchgehende Bohrung vorgesehen, durch die ein Sicherungselement 76 in Form eines Stiftes durchgesteckt und mit einer Halterungskappe 77 vor dem Herausfallen gesichert ist. Das Sicherungselement 76 verhindert ein ungewolltes Schrauben der Verschlusskappe 72 und damit ein ungewolltes Bedienen der Öffnungseinrichtung 68. Das Sicherungselement 76 kann unmittelbar vor einer Anwendung der Vorrichtung gelöst werden, indem die Halterungskappe 77 abgezogen und der Stift herausgezogen wird. Danach kann die Öffnungseinrichtung 68 in die Kammer 60 geschraubt werden.

Beim Einschrauben der Öffnungseinrichtung 68 werden die Bohrungen 73 von den Begasungsöffnungen 57 weggedreht und in Längsrichtung verschoben und durch die Außenwand des Hohlzylinders 70 verschlossen. Dadurch wird die Vorrichtung nach außen abgeschlossen, so dass die in die Kammer 60 austretende Monomerflüssigkeit 3 nicht aus der Kammer 60 durch die Begasungsöffnungen 57 heraustreten kann.

Um eine Drehung der Verschlusskappe 72 in die falsche Richtung und dadurch ein Öffnen der Kammer 60 an deren Rückseite zu verhindern, ist eine Rückdrehsicherung vorgesehen (in den Figuren 9 und 10 nicht zu sehen). Die Rückdrehsicherung verhindert ein Lösen der Verschlusskappe 72 beziehungsweise ein Lösen der Öffnungseinrichtung 68 von der Monomeraufnahme 52. Die Rückdrehsicherung kann beispielsweise als Schraubensicherung in Form einer Sperrkantscheibe oder durch ein Keilsicherungsscheibenpaar oder ähnliche Maßnahmen realisiert werden. Hiermit wird nicht nur ein Lösen der Öffnungseinrichtung verhindert, sondern es kann bei geeignetem Aufbau der Rückdrehsicherung auch verhindert werden, dass die Begasungsöffnungen 57 durch ein Zurückschrauben der Öffnungseinrichtung 68 wieder geöffnet werden können.

Um die Öffnungseinrichtung 68 bequem mit der Hand drehen zu können, ist deren rückseitiges Ende mit einem Griff 78 ausgestattet. Um die Begasungsöffnung 57 gasdicht und druckdicht schließen zu können und um den Hohlzylinder 70 gegen die Innenwand der Kammer 60 abzudichten, sind zwei umlaufende Dichtungen 80 aus Gummi in umlaufenden Nuten am Außenumfang des Hohlzylinders 70 angeordnet. Die radialen Bohrungen 73 sind zwischen den in Längsrichtung zueinander beabstandeten Dichtungen 80 angeordnet. Die rückseitige der beiden Dichtungen 80 ist dicht neben den Bohrungen 73 angeordnet, so dass die Begasungsöffnungen 57 bei einer axialen Bewegung des Hohlzylinders 70 in Längsrichtung schnell geschlossen werden. Alternativ können auch Dichtungsringe um die Begasungsöffnungen 57 an der Innenwand der Monomeraufnahme 52 herum oder um die Bohrungen 73 herum an der Außenwand des Hohlzylinders 70 angeordnet sein. Die Begasungsöffnungen 57 werden von der hinteren Dichtung (in Figur 9 oben) abgedichtet, wenn der Hohlzylinder 70 in Richtung des Kolbens 62 geschoben wird.

In gleicher Weise befinden sich am Außenumfang des Kolbens 62 zwei in Längsrichtung voneinander beabstandete Nuten, in denen sich zwei umlaufende Dichtungen 82 aus Gummi befinden. Die Dichtungen 82 dichten den Kolben 62 gegen den Innenraum 61 der Kartusche 51 ab und verschließen die Rückseite des Innenraums 61 der Kartusche 51.

Die Kanäle 64 und der ringförmige Porenfilter 66 sind um einen Dorn 84 oder eine Schneidkante 84 zum Aufbrechen der Ampulle 5 herum angeordnet. Der Dorn 84 oder die Schneidkante 84 weist hierzu ins Innere der Kammer 60. Die Ampulle 5 kann hierzu mit dem Hohlzylinder 70 auf den Dorn 84 oder die Schneidkante 84 gedrückt werden, bis der Boden der Ampulle 5 aufbricht. Der Hohlzylinder 70 weist dazu in etwa den gleichen Durchmesser wie der Ampullenkörper der Ampulle 5 auf. Ein Ampullenkopf der Ampulle 5 ist dabei im Inneren des Hohlzylinders 70 angeordnet. Damit wird erreicht, dass die Ampulle 5 nicht im Bereich des Hohlzylinders 70 gebrochen wird, weil der zylindrische Ampullenkörper sehr stabil ist, während der Dorn 84 oder die Schneidkante 84 relativ leicht in den Boden der Ampulle 5 gedrückt werden kann.

Damit die Ampulle 5 nicht schon bei Transport der Vorrichtung geöffnet wird, ist um den Dorn 84 oder die Schneidkante 84 herum eine Feder 86 in der Kammer 60 angeordnet, die die Ampulle 5 von dem Dorn 84 oder der Schneidkante 84 beabstandet. Beim Einschrauben der Öffnungseinrichtung 68 wird die Feder 86 von der Ampulle 5 komprimiert und der Boden der Ampulle 5 am Dorn 84 oder an der Schneidkante 84 aufgebrochen.

Die Vorderseite der Kartusche 51 ist mit einem Kartuschendeckel 88 verschlossen. In der Mitte des Kartuschendeckels 88 ist ein Stutzen 89 ausgeformt, der die Austragsöffnung an der Vorderseite begrenzt. An dem Stutzen 89 ist der Stopfen 56 lösbar befestigt, der die Austragsöffnung verschließt. Der Kartuschendeckel 88 ist mit einem Innengewinde 90 auf ein Außengewinde 92 an der Vorderseite der Kartusche 51 geschraubt. Der Kartuschendeckel 88 ist zusätzlich über eine umlaufende Dichtung 94 gegen die Kartusche 51 abgedichtet.

Im Innenraum 61 der Kartusche 51 befinden sich mehrere lose Kugeln 95 aus Zirkoniumdioxid-Keramik als Mischelemente, mit denen der Inhalt im Innenraum 61 der Kartusche 51 durch schütteln der Vorrichtung durchmischt werden kann. Dadurch, dass die Kugeln 95 eine größere Dichte haben als der Knochenzementteig, können diese in dem Knochenzementteig und noch wesentlich besser in einer Knochenzementteig-Gas-Mischung durch Schütteln der Vorrichtung gegen den Knochenzementteig beziehungsweise die Knochenzementteig-Gas-Mischung bewegt werden. Die Kugeln 95 fliegen dabei in dem Innenraum 61 der Kartusche 51 umher und durchmischen dabei die Ausgangskomponenten 3, 4.

Damit die Kugeln 95 die Austragsöffnung nicht verschließen können, ist an der Innenseite des Kartuschendeckels 88 ein vorspringender Steg 96 vorgesehen, der sich unmittelbar neben der Austragsöffnung erhebt. Wenn eine Kugel 95 mittig vor der Austragsöffnung sitzt, so wird sie von dem Steg 96 zur Seite gedrückt und kann nicht bündig an der Austragsöffnung anliegen, so dass der Knochenzementteig zwischen den Kugeln 95 und dem Steg 96 hindurch in und durch die Austragsöffnung fließen kann.

Die Vorderseite des Kolbens 62 und die Rückseite des Kartuschendeckels 88, die die Stirnseiten des Innenraums 61 der Kartusche 51 begrenzen, weisen zur Seitenwand des Innenraums 61 der Kartusche 51 hin ansteigende Flanken auf, deren Krümmungsradius größer ist als der Radius der Kugeln 95. Dadurch können die Kugeln 95 beim Schütteln der Vorrichtung jeden Bereich des Innenraums 61 erreichen. Hiermit wird verhindert, dass Kanten im Innenraum 61 vorhanden sind, in denen das Zementpulver 4 nicht von den Kugeln 95 erreicht wird und das dadurch nicht in den Knochenzementteig gemischt werden kann.

Ein zweites Sicherungselement 97 in Form einer Spange 97 kann am Übergang von der Monomeraufnahme 52 in die Kartusche 51 angeordnet sein. Mit der Spange 97 kann ein Einschrauben der Monomeraufnahme 52 in die Kartusche 51 hinein verhindert werden. Die Spange 97 wird vor dem Einschrauben der Monomeraufnahme 52 in die Kartusche 51 abgezogen. Die Spange 97 ist nicht besonders wichtig und kann auch weggelassen werden.

Im Folgenden wird der Ablauf eines erfindungsgemäßen Verfahrens erläutert. Zunächst befindet sich die Vorrichtung im Ausgangszustand (siehe Figur 9 und 10). In diesem Zustand ist die Vorrichtung verpackt und mit Ethylenoxid sterilisiert worden. Das Ethylenoxid kann durch die Begasungsöffnungen 57 und die Bohrungen 73 in die Kammer 60 und durch den Porenfilter 66, den Splitterschutz 67 und die Kanäle 64 in den Innenraum 61 der Kartusche 51 gelangen. Der Gasaustausch erfolgt dabei in einer Vakuumkammer oder in einer Unterdruckkammer. In diesem Zustand (siehe Figur 9 und 10) wird die Vorrichtung ausgepackt.

Zunächst wird die Spange 97 abgezogen. Dies kann aber auch später geschehen. Die Vorrichtung befindet sich nun in dem in Figur 9 gezeigten Zustand. Bei der zweiten erfindungsgemäßen Vorrichtung muss die Monomeraufnahme 52 nicht mehr zunächst in die Kartusche 51 hineingeschraubt werden, da die Vorrichtung bereits in dem maximal eingeschraubten Zustand vorliegt (siehe Figur 9 und 10). Ab hier verläuft das Verfahren sehr weitgehend analog dem zu dem ersten Ausführungsbeispiel nach den Figuren 1 bis 8 beschriebenen Verfahren.

In einem nächsten Schritt wird das Sicherungselement 76 entfernt und die Öffnungseinrichtung 68 in die Kammer 60 geschraubt. Hierbei wird die Vorrichtung vorzugsweise mit dem Kartuschendeckel 88 nach unten gehalten. Dabei drückt der Hohlzylinder die Ampulle 5 an deren Schultern gegen die Kraft der Feder 86 in Richtung des Dorns 84 oder der Schneidkante 84. Die Begasungsöffnungen 57 werden durch die Schraubbewegung von dem Hohlzylinder 70 verschlossen. Anschließend wird die Ampulle 5 mit dem Boden auf den Dorn 84 oder die Schneidkante 84 gedrückt und die Ampulle 5 bricht am Boden auf.

Die Monomerflüssigkeit 3 tritt am Boden der Ampulle 5 im Bereich des durch die Kanäle 64 gebildeten Durchgangs aus. Da die Vorrichtung mit dem Kartuschendeckel 88 nach unten gehalten wird, fließt die Monomerflüssigkeit 3 getrieben von der Schwerkraft sofort nach unten durch den Splitterschutz 67, die Kanäle 64 und den Porenfilter 66 in den Innenraum 61 der Kartusche 51 und verteilt sich in dem Zementpulver 4. Splitter der Ampulle 5 werden gegebenenfalls von dem Splitterschutz 67 zurückgehalten. Um den Monomertransfer zu beschleunigen wird die Monomeraufnahme 52 aus der Kartusche 51 herausgeschraubt. Da die Begasungsöffnungen 57 gasdicht und druckdicht verschlossen sind und der Innenraum 61 der Kartusche 51 gegen den Kolben 62 abgedichtet und nach außen geschlossen ist, wird durch die Vergrößerung des Volumens des Innenraums 61 der Kartusche 51 ein Unterdruck in dem Innenraum 61 der Kartusche 51 erzeugt, mit dem die Monomerflüssigkeit 3 in den Innenraum 61 der Kartusche 51 gesaugt wird. Auch hierbei wird die Vorrichtung mit dem Kartuschendeckel 88 nach unten gehalten. Zudem wird durch die Vergrößerung des Innenraums 61 der Kartusche 51 der Gasdruck im Inneren der Vorrichtung verringert. Das Gas im Unterdruck steht schließlich auch im Innenraum 61 der Kartusche 51 über den Ausgangskomponenten 3, 4. Das Volumen des überstehenden Gases ist dabei zumindest doppelt so groß wie das Volumen der Monomerflüssigkeit 3.

In diesem Zustand kann der Inhalt des Innenraums 61 der Kartusche 51 nämlich die Monomerflüssigkeit 3 und das Zementpulver 4 durch Schütteln der Vorrichtung gemischt werden. Dabei fliegen die Kugeln 95 in dem Innenraum 61 der Kartusche 51 umher und unterstützen dabei die Vermischung der Komponenten, wobei aufgrund der abgerundeten Form der Begrenzung des Innenraums 61 der Kartusche 51 alle Bereiche erreicht werden, so dass eine vollständige Durchmischung erreicht wird. Im Innenraum 61 der Kartusche 51 befindet sich dann ein Knochenzementteig-Gas-Gemisch. Nach mehrmaligem Schütteln ist der Knochenzementteig gut gemischt.

Die Vorrichtung wird nun umgedreht, so dass der Kartuschendeckel 88 nach oben weist. Der Stopfen 56 wird aus der Austragsöffnung entfernt. Jetzt kann optional ein Schlauch mit einem Trokar (nicht gezeigt) an dem Stutzen 89 befestigt werden, durch die der Knochenzementteig an schwer zugänglichen Orten unter Röntgenkontrolle appliziert werden kann. Da der Knochenzementteig für die Anwendung bei der Spondylodese eher dünnflüssig ist, steigen die Gasblasen nach oben. Die Monomeraufnahme 52 wird wieder in die Kartusche 51 hineingeschraubt und das Gas entweicht nach oben aus der Austragsöffnung. Schließlich tritt der Knochenzementteig aus der Austragsöffnung beziehungsweise durch den Stutzen 89 aus dem Innenraum 61 der Kartusche 51 aus.

Durch weiteres Einschrauben der Monomeraufnahme 52 in die Kartusche 51 wird der Knochenzementteig mit dem Kolben 62 aus der Vorrichtung herausgepresst. Schließlich drückt der Kolben 62 die Kugeln 95 gegen den Kartuschendeckel 88. Der Auspressvorgang ist damit beendet.

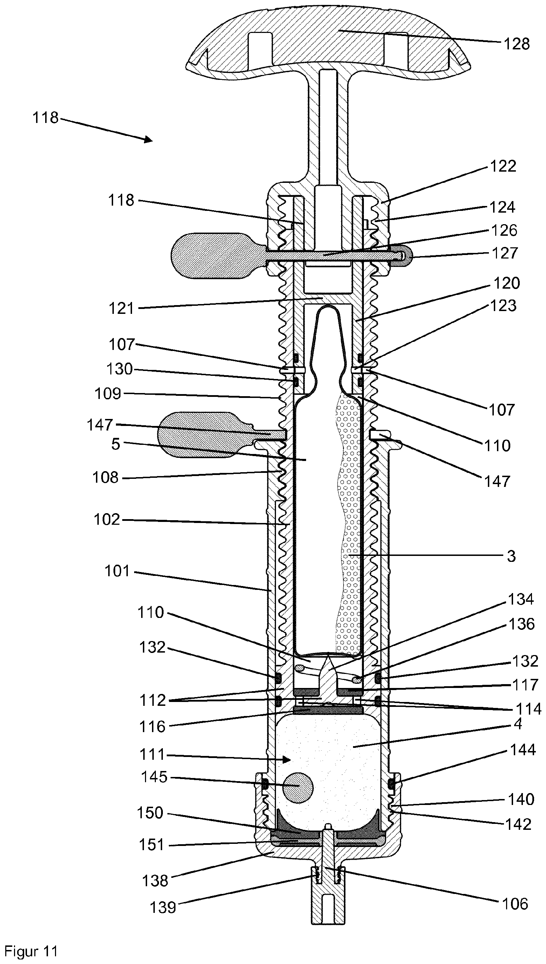

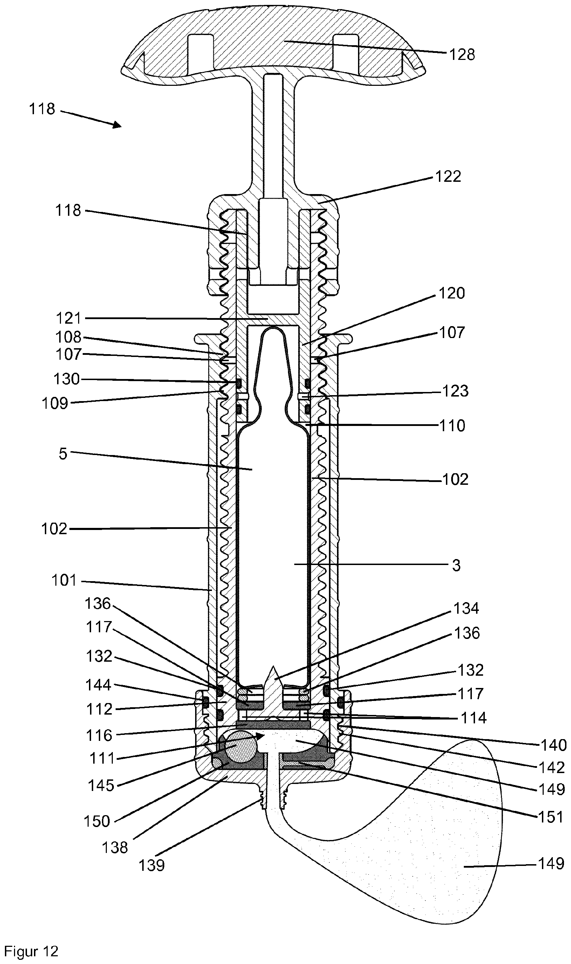

In den Figuren 11 und 12 sind Abbildungen einer dritten erfindungsgemäßen Vorrichtung zum Lagern der Ausgangskomponenten 3, 4 eines Knochenzementteigs 149 und zum Mischen des Knochenzementteigs 149 gezeigt. Dabei zeigt Figur 11 eine Querschnittansicht im Ausgangszustand und Figur 10 eine Querschnittansicht im Endzustand nach dem Auspressen des fertig gemischten Knochenzementteigs 149.

Die dritte erfindungsgemäße Vorrichtung weist eine röhrenförmige Kartusche 101 aus Kunststoff auf, die einen vorderen Teil (in Figur 11 und 12 unten) der Vorrichtung bildet. Ein rückseitiger, hinterer Teil der Vorrichtung wird durch eine Monomeraufnahme 102 gebildet. Die Vorrichtung ist zur Herstellung eines Knochenzementteigs 149 vorgesehen, der aus einer Monomerflüssigkeit 3 und aus einem Zementpulver 4 hergestellt wird. Die Monomerflüssigkeit 3 ist dazu in einer aufbrechbaren Ampulle 5 aus Glas oder aus Kunststoff als Monomerflüssigkeitsbehälter für die Monomerflüssigkeit 3 enthalten, wobei die Ampulle 5 in der Monomeraufnahme 102 steckt. Die Kartusche 101 bildet in ihrem Inneren einen zylindrischen Innenraum 111, in dem das Zementpulver 4 enthalten ist.

Die Kartusche 101 weist an ihrer Vorderseite (in Figur 11 und 12 unten) eine Austragsöffnung auf, die zunächst mit einem herausnehmbaren Verschluss 106 verschlossen ist. In der seitlichen Wandung der Monomeraufnahme 102 befinden sich mehrere Begasungsöffnungen 107, durch die ein Gas aus dem Inneren der Vorrichtung abgesaugt werden kann und durch die ein sterilisierendes Gas wie Ethylenoxid eingefüllt werden kann, um das Innere der Vorrichtung zu sterilisieren.

Am rückseitigen Ende der Kartusche 101 befindet sich ein Innengewinde 108. Die Monomeraufnahme 102 weist an ihrer Außenseite ein zu dem Innengewinde 108 der Kartusche 101 passendes Außengewinde 109 auf. Die Monomeraufnahme 102 ist nach Art eines Gewinderohrs geformt und weist in ihrem Inneren eine zylindrische Kammer 110 auf, in der die Ampulle 5 steckt. Dazu weist die Ampulle 5 einen zylindrischen Ampullenkörper mit passendem Durchmesser auf. Im Inneren der Kartusche 101 bildet die Kartusche 101 den zylindrischen Innenraum 111. Die Zylindergeometrie des Innenraums 111 und der Kammer 110 entsprechen Zylindern mit kreisförmiger Grundfläche.

Die Monomeraufnahme 102 ist an ihrer Vorderseite durch einen zylindrischen Kolben 112 begrenzt, der die Kammer 110 nach vorne an ihrer kreisförmigen Grundfläche verschließt. Der Kolben 112 weist mehrere Kanäle 114 als Durchgang durch den Kolben 112 auf, die ringförmig in dem Kolben 112 angeordnet sind und die Vorderseite des Kolbens 112 mit der Rückseite des Kolbens 112 und dadurch die Kammer 110 der Monomeraufnahme 102 mit dem Innenraum 111 der Kartusche 101 verbinden. Die Kanäle 114 sind mit einem kreisscheibenförmigen Porenfilter 116 und mit einem ringförmigen Gitter als Splitterschutz 117 abgedeckt. Der Porenfilter 116 ist für das Zementpulver 4 aus dem Innenraum 111 der Kartusche 101 undurchlässig und für die Monomerflüssigkeit 3 und Gase durchlässig. Dadurch wird ein Vordringen des Zementpulvers 4 in die Kammer 110 der Monomeraufnahme 2 verhindert. Der Splitterschutz 117 kann durch ein Gitter realisiert werden. Mit dem Splitterschutz 117 wird ein Vordringen von Splittern der geöffneten Ampulle 5 in die Kanäle 114 des Durchgangs verhindert. Der Kolben 112 weist einen größeren Außendurchmesser auf, als das Außengewinde 109 der Monomeraufnahme 102. Der Außendurchmesser des zylindrischen Kolbens 112 passt zum Innendurchmesser des Innenraums 111 der Kartusche 101. Beim Zusammenbau der Vorrichtung muss die Monomeraufnahme 102 von vorne in die Kartusche 101 eingesteckt werden und mit dem Außengewinde 109 in das Innengewinde 108 der Kartusche 101 eingeschraubt werden. Der Kolben 112 der Monomeraufnahme 102 dichtet den Innenraum 111 der Kartusche 101 in Richtung der Rückseite (in Figur 11 und 12 oben) ab.

An der Rückseite der Monomeraufnahme 102 ist eine Öffnungseinrichtung 118 vorgesehen, mit der die Ampulle 5 in Richtung des Kolbens 112 gedrückt werden kann, um die Ampulle 5 zu öffnen und die Monomerflüssigkeit 3 im Inneren der Kammer 110 zu öffnen. Die Öffnungseinrichtung 118 weist hierzu einen Hohlzylinder 120 auf, der nach Art einer Hülse geformt ist. Der Hohlzylinder 120 liegt dabei an der Innenwand der Kammer 110 an und deckt diese im Bereich der Rückseite der Kammer 110 ab. Im Hohlzylinder 120 ist eine geschlossene Wandung 121 senkrecht zur Achse der Zylindergeometrie des Hohlzylinders 120 vorgesehen, so dass der Hohlzylinder 120 mit der geschlossenen Wandung 121 die Kammer 110 an ihrer Rückseite nach außen abschließt. Der Hohlzylinder 120 ist an einer schraubbaren Verschlusskappe 122 befestigt. In der seitlichen Zylinderwandung (der Zylindermantelfläche) des Hohlzylinders 120 sind radiale Bohrungen 123 vorgesehen, die im Ausgangszustand und Lagerungszustand der Vorrichtung (siehe Figur 11) mit den Begasungsöffnungen 107 fluchten. Dadurch ist die Kammer 110 und dadurch über die Kanäle 114 auch der Innenraum 111 der Kartusche 101 und damit das Zementpulver 4 in diesem Zustand gasdurchlässig mit der Umgebung der Vorrichtung verbunden. Die Verschlusskappe 122 weist ein Innengewinde 124 auf, das zu dem Außengewinde 109 des Monomeraufnahme 102 passt.

Die Verschlusskappe 122 beziehungsweise die Öffnungseinrichtung 118 ist ein Stück aber nicht bis zu einem Anschlag auf die Rückseite der Monomeraufnahme 102 aufgeschraubt und so an dieser befestigt. Es ist wichtig, dass die Verschlusskappe 122 noch weiter auf die Monomeraufnahme 102 aufgeschraubt werden kann und dadurch der Hohlzylinder 120 tiefer in die Kammer 110 eingeschoben werden kann.

An der Rückseite der Monomeraufnahme 102 und hinter der geschlossenen Wandung 121 des Hohlzylinders 120 ist eine durchgehende Bohrung vorgesehen, durch die ein Sicherungselement 126 in Form eines Stiftes durchgesteckt und mit einer Halterungskappe 127 vor dem Herausfallen gesichert ist. Das Sicherungselement 126 verhindert ein ungewolltes Schrauben der Verschlusskappe 122 und damit ein ungewolltes Bedienen der Öffnungseinrichtung 118. Das Sicherungselement 126 kann unmittelbar vor einer Anwendung der Vorrichtung gelöst werden, indem die Halterungskappe 127 abgezogen und der Stift herausgezogen wird. Danach kann die Öffnungseinrichtung 118 in die Kammer 110 geschraubt werden.

Beim Einschrauben der Öffnungseinrichtung 118 werden die Bohrungen 123 von den Begasungsöffnungen 107 weggedreht und in Längsrichtung verschoben und durch die Außenwand des Hohlzylinders 120 verschlossen. Dadurch wird die Vorrichtung nach außen abgeschlossen, so dass die in die Kammer 110 austretende Monomerflüssigkeit 3 nicht aus der Kammer 110 durch die Begasungsöffnungen 107 heraustreten kann.

Um eine Drehung der Verschlusskappe 122 in die falsche Richtung und dadurch ein Öffnen der Kammer 110 an deren Rückseite zu verhindern, ist eine Rückdrehsicherung vorgesehen (in den Figuren 11 und 12 nicht zu sehen). Die Rückdrehsicherung verhindert ein Lösen der Verschlusskappe 122 beziehungsweise ein Lösen der Öffnungseinrichtung 118 von der Monomeraufnahme 102. Die Rückdrehsicherung kann beispielsweise als Schraubensicherung in Form einer Sperrkantscheibe oder durch ein Keilsicherungsscheibenpaar oder ähnliche Maßnahmen realisiert werden. Hiermit wird nicht nur ein Lösen der Öffnungseinrichtung verhindert, sondern es kann bei geeignetem Aufbau der Rückdrehsicherung auch verhindert werden, dass die Begasungsöffnungen 107 durch ein Zurückschrauben der Öffnungseinrichtung 118 wieder geöffnet werden können.

Um die Öffnungseinrichtung 118 bequem mit der Hand drehen zu können, ist deren rückseitiges Ende mit einem Griff 128 ausgestattet. Um die Begasungsöffnung 107 gasdicht und druckdicht schließen zu können und um den Hohlzylinder 120 gegen die Innenwand der Kammer 110 abzudichten, sind zwei umlaufende Dichtungen 130 aus Gummi in umlaufenden Nuten am Außenumfang des Hohlzylinders 120 angeordnet. Die radialen Bohrungen 123 sind zwischen den in Längsrichtung zueinander beabstandeten Dichtungen 130 angeordnet. Die rückseitige der beiden Dichtungen 130 ist dicht neben den Bohrungen 123 angeordnet, so dass die Begasungsöffnungen 107 bei einer axialen Bewegung des Hohlzylinders 120 in Längsrichtung schnell geschlossen werden. Alternativ können auch Dichtungsringe um die Begasungsöffnungen 107 an der Innenwand der Monomeraufnahme 102 herum oder um die Bohrungen 123 herum an der Außenwand des Hohlzylinders 120 angeordnet sein. Die Begasungsöffnungen 107 werden von der hinteren Dichtung (in Figur 11 und 12 oben) abgedichtet, wenn der Hohlzylinder 120 in Richtung des Kolbens 112 geschoben wird.

In gleicher Weise befinden sich am Außenumfang des Kolbens 112 zwei in Längsrichtung voneinander beabstandete Nuten, in denen sich zwei umlaufende Dichtungen 132 aus Gummi befinden. Die Dichtungen 132 dichten den Kolben 112 gegen den Innenraum 111 der Kartusche 101 ab und verschließen die Rückseite des Innenraums 111 der Kartusche 101.

Die Kanäle 114 und der ringförmige Porenfilter 116 sind um einen Dorn 134 oder eine Schneidkante 134 zum Aufbrechen der Ampulle 5 herum angeordnet. Der Dorn 134 oder die Schneidkante 134 weist hierzu ins Innere der Kammer 110. Die Ampulle 5 kann hierzu mit dem Hohlzylinder 120 auf den Dorn 134 oder die Schneidkante 134 gedrückt werden, bis der Boden der Ampulle 5 aufbricht. Der Hohlzylinder 120 weist dazu in etwa den gleichen Durchmesser wie der Ampullenkörper der Ampulle 5 auf. Ein Ampullenkopf der Ampulle 5 ist dabei im Inneren des Hohlzylinders 120 angeordnet. Damit wird erreicht, dass die Ampulle 5 nicht im Bereich des Hohlzylinders 120 gebrochen wird, weil der zylindrische Ampullenkörper sehr stabil ist, während der Dorn 134 oder die Schneidkante 134 relativ leicht in den Boden der Ampulle 5 gedrückt werden kann.

Damit die Ampulle 5 nicht schon bei Transport der Vorrichtung geöffnet wird, ist um den Dorn 134 oder die Schneidkante 134 herum eine Feder 136 in der Kammer 110 angeordnet, die die Ampulle 5 von dem Dorn 134 oder der Schneidkante 134 beabstandet. Alternativ zu der Feder 136 kann auch ein elastischer zusammenpressbarer geschlossen-poriger Schaumstoff oder ein komprimierbarer hohler Gummikörper um den Dorn 134 oder die Schneidkante 134 herum angeordnet sein. Beim Einschrauben der Öffnungseinrichtung 118 wird die Feder 136 oder der Schaumstoff von der Ampulle 5 komprimiert und der Boden der Ampulle 5 am Dorn 134 oder an der Schneidkante 134 aufgebrochen.

Die Vorderseite der Kartusche 101 ist mit einem Kartuschendeckel 138 verschlossen. In der Mitte des Kartuschendeckels 138 ist ein Stutzen 139 ausgeformt, der die Austragsöffnung an der Vorderseite begrenzt. An dem Stutzen 139 ist der Stopfen 106 lösbar befestigt, der die Austragsöffnung verschließt. Der Kartuschendeckel 138 ist mit einem Innengewinde 140 auf ein Außengewinde 142 an der Vorderseite der Kartusche 101 geschraubt. Der Kartuschendeckel 138 ist zusätzlich über eine umlaufende Dichtung 144 gegen die Kartusche 101 abgedichtet.

Im Innenraum 111 der Kartusche 101 befindet sich eine lose Kugel 145 aus Zirkoniumdioxid-Keramik als Mischelement, mit der der Inhalt im Innenraum 111 der Kartusche 101 durch schütteln der Vorrichtung durchmischt werden kann. Dadurch, dass die Kugel 145 eine größere Dichte hat als der Knochenzementteig 149, können diese in dem Knochenzementteig 149 und noch wesentlich besser in einer Knochenzementteig-Gas-Mischung durch Schütteln der Vorrichtung gegen den Knochenzementteig 149 beziehungsweise gegen die Knochenzementteig-Gas-Mischung bewegt werden. Die Kugel 145 fliegt dabei in dem Innenraum 111 der Kartusche 101 umher und durchmischt dabei die Ausgangskomponenten 3, 4.

An der Vorderseite des Innenraums 11 der Kartusche 101 befindet sich ein verformbares Aufnahmeelement 150 in Form eines hohlen Gummikörpers oder eines geschlossen-porigen Schaumstoffs, in den die Kugel 145 hineingedrückt werden kann. In dem Aufnahmeelement 150 ist ein Kunststoffkörper in Form eines Rads mit Speichen 151 vorgesehen, zwischen denen die Kugel 145 Platz findet. Im Bereich der "Radnabe" bildet der Kunststoffkörper eine Hülse, von der die Kugel 145 abgleitet. Hierzu kann die Hülse des Kunststoffkörpers asymmetrisch aufgebaut sein.

Die Vorderseite des Kolbens 112 und die durch das Aufnahmeelement 150 geformte Rückseite, die die Stirnseiten des Innenraums 111 der Kartusche 101 begrenzen, weisen zur Seitenwand des Innenraums 111 der Kartusche 101 hin ansteigende Flanken auf, deren Krümmungsradius größer ist als der Radius der Kugel 145. Dadurch kann die Kugel 145 beim Schütteln der Vorrichtung jeden Bereich des Innenraums 111 erreichen. Hiermit wird verhindert, dass Kanten im Innenraum 111 vorhanden sind, in denen das Zementpulver 4 nicht von der Kugel 145 erreicht wird und das dadurch nicht in den Knochenzementteig 149 gemischt werden kann.

Ein zweites Sicherungselement 147 in Form einer Spange 147 kann am Übergang von der Monomeraufnahme 102 in die Kartusche 101 angeordnet sein. Mit der Spange 147 kann ein Einschrauben der Monomeraufnahme 102 in die Kartusche 101 hinein verhindert werden. Die Spange 147 wird vor dem Einschrauben der Monomeraufnahme 102 in die Kartusche 101 abgezogen. Die Spange 147 ist nicht besonders wichtig und kann auch weggelassen werden.

Im Folgenden wird der Ablauf eines erfindungsgemäßen Verfahrens erläutert. Zunächst befindet sich die Vorrichtung im Ausgangszustand (siehe Figur 11). In diesem Zustand ist die Vorrichtung verpackt und mit Ethylenoxid sterilisiert worden. Das Ethylenoxid kann durch die Begasungsöffnungen 107 und die Bohrungen 123 in die Kammer 110 und durch den Porenfilter 116, den Splitterschutz 117 und die Kanäle 114 in den Innenraum 111 der Kartusche 101 gelangen. Der Gasaustausch erfolgt dabei in einer Vakuumkammer oder in einer Unterdruckkammer. In diesem Zustand (siehe Figur 11) wird die Vorrichtung ausgepackt.

Bei der dritten erfindungsgemäßen Vorrichtung muss die Monomeraufnahme 102 nicht mehr zunächst in die Kartusche 101 hineingeschraubt werden, da die Vorrichtung bereits in dem maximal eingeschraubten Zustand vorliegt (siehe Figur 11). Ab hier verläuft das Verfahren sehr weitgehend analog den zu dem ersten Ausführungsbeispiel nach den Figuren 1 bis 8 und zu dem zweiten Ausführungsbeispiel nach den Figuren 9 und 10 beschriebenen Verfahren.

In einem nächsten Schritt wird das Sicherungselement 126 entfernt und die Öffnungseinrichtung 118 in die Kammer 110 geschraubt. Hierbei wird die Vorrichtung vorzugsweise mit dem Kartuschendeckel 138 nach unten gehalten. Dabei drückt der Hohlzylinder die Ampulle 5 an deren Schultern gegen die Kraft der Feder 136 in Richtung des Dorns 134 oder der Schneidkante 134. Die Begasungsöffnungen 107 werden durch die Schraubbewegung von dem Hohlzylinder 120 verschlossen. Anschließend wird die Ampulle 5 mit dem Boden auf den Dorn 134 oder die Schneidkante 134 gedrückt und die Ampulle 5 bricht am Boden auf.

Die Monomerflüssigkeit 3 tritt am Boden der Ampulle 5 im Bereich des durch die Kanäle 114 gebildeten Durchgangs aus. Da die Vorrichtung mit dem Kartuschendeckel 138 nach unten gehalten wird, fließt die Monomerflüssigkeit 3 getrieben von der Schwerkraft sofort nach unten durch den Splitterschutz 117, die Kanäle 114 und den Porenfilter 116 in den Innenraum 111 der Kartusche 101 und verteilt sich in dem Zementpulver 4. Splitter der Ampulle 5 werden gegebenenfalls von dem Splitterschutz 117 zurückgehalten. Um den Monomertransfer zu beschleunigen wird die Monomeraufnahme 102 aus der Kartusche 101 herausgeschraubt. Hierzu wird zuvor die Sicherungsklammer 147 entfernt. Da die Begasungsöffnungen 107 gasdicht und druckdicht verschlossen sind und der Innenraum 111 der Kartusche 101 gegen den Kolben 112 abgedichtet und nach außen geschlossen ist, wird durch die Vergrößerung des Volumens des Innenraums 111 der Kartusche 101 ein Unterdruck in dem Innenraum 111 der Kartusche 101 erzeugt, mit dem die Monomerflüssigkeit 3 in den Innenraum 111 der Kartusche 101 gesaugt wird. Auch hierbei wird die Vorrichtung mit dem Kartuschendeckel 138 nach unten gehalten. Zudem wird durch die Vergrößerung des Innenraums 111 der Kartusche 101 der Gasdruck im Inneren der Vorrichtung verringert. Das Gas im Unterdruck steht schließlich auch im Innenraum 111 der Kartusche 101 über den Ausgangskomponenten 3, 4. Das Volumen des überstehenden Gases ist dabei zumindest doppelt so groß wie das Volumen der Monomerflüssigkeit 3.

In diesem Zustand kann der Inhalt des Innenraums 111 der Kartusche 101 nämlich die Monomerflüssigkeit 3 und das Zementpulver 4 durch Schütteln der Vorrichtung gemischt werden. Dabei fliegt die Kugel 145 in dem Innenraum 111 der Kartusche 101 umher und unterstützt dabei die Vermischung der Komponenten, wobei aufgrund der abgerundeten Form der Begrenzung des Innenraums 111 der Kartusche 101 alle Bereiche erreicht werden, so dass eine vollständige Durchmischung erreicht wird. Im Innenraum 111 der Kartusche 101 befindet sich dann ein Knochenzementteig-Gas-Gemisch. Nach mehrmaligem Schütteln ist der Knochenzementteig 149 gut gemischt.

Die Vorrichtung wird nun umgedreht, so dass der Kartuschendeckel 138 nach oben weist. Der Stopfen 106 wird aus der Austragsöffnung entfernt. Jetzt kann optional ein Schlauch mit einem Trokar (nicht gezeigt) an dem Stutzen 139 befestigt werden, durch die der Knochenzementteig 149 an schwer zugänglichen Orten unter Röntgenkontrolle appliziert werden kann. Da der Knochenzementteig 149 für die Anwendung bei der Spondylodese eher dünnflüssig ist, steigen die Gasblasen nach oben. Die Monomeraufnahme 102 wird wieder in die Kartusche 101 hineingeschraubt und das Gas entweicht nach oben aus der Austragsöffnung. Schließlich tritt der Knochenzementteig 149 aus der Austragsöffnung beziehungsweise durch den Stutzen 139 aus dem Innenraum 111 der Kartusche 101 aus.

Durch weiteres Einschrauben der Monomeraufnahme 102 in die Kartusche 101 wird der Knochenzementteig 149 mit dem Kolben 112 aus der Vorrichtung herausgepresst. Schließlich drückt der Kolben 112 die Kugel 145 in das Aufnahmeelement 150 zwischen die Speichen 151 gegen den Kartuschendeckel 138. Die Speichen 151 können dabei verformt werden. Der Auspressvorgang ist damit beendet. Dieser Zustand ist in Figur 12 gezeigt.

Praktisch alle Teile der drei mit den Figuren 1 bis 12 beschriebenen Vorrichtungen (bis auf die Ausgangskomponenten 3, 4) können bevorzugt aus Kunststoff bestehen und können durch Spitzguss kostengünstig hergestellt werden. Die Ampulle 5 besteht vorzugsweise aus Glas.

### Bezugszeichenliste

- 1, 51, 101: Kartusche
- 2, 52, 102: Monomeraufnahme
- 3: Monomerflüssigkeit
- 4: Zementpulver
- 5: Ampulle
- 6, 56, 106: Stopfen
- 7, 57, 107: Begasungsöffnung
- 8, 58, 108: Innengewinde
- 9, 59, 109: Außengewinde
- 10, 60, 110: Kammer
- 11, 61, 111: Innenraum
- 12, 62, 112: Kolben
- 14, 64, 114: Durchgang / Kanal
- 16, 66, 116: Porenfilter
- 18, 68, 118: Öffnungseinrichtung
- 20, 70, 120: Hohlzylinder / Hülse
- 21, 71, 121: Geschlossene Wandung
- 22, 72, 122: Verschlusskappe
- 23, 73, 123: Bohrung
- 24, 74, 124: Innengewinde
- 26, 76, 126: Sicherungselement / Sicherungsstift
- 27, 77, 127: Halterungskappe
- 28, 78, 128: Griff
- 30, 80, 130: Dichtung
- 32, 82, 132: Dichtung
- 34, 84, 134: Dorn / Schneidkante
- 36, 86, 136: Feder
- 38, 88, 138: Kartuschendeckel
- 39, 89, 139: Stutzen
- 40, 90, 140: Innengewinde
- 42, 92, 142: Außengewinde
- 44, 94, 144: Dichtung
- 45, 95, 145: Lose Mischkugel
- 46, 96: Steg
- 47, 97, 147: Sicherungselement / Sicherungsklammer
- 48: Knochenzementteig-Gas-Mischung
- 49, 149: Knochenzementteig
- 67, 117: Splitterschutz / Sieb
- 150: Aufnahmeelement / Gummi-Hohlkörper
- 151: Speiche

## Patentansprüche

1. Vorrichtung zum Herstellen eines Knochenzementteigs (49, 149) aus einer Monomerflüssigkeit (3) und einem Zementpulver (4) als Ausgangskomponenten des Knochenzementteigs (49, 149) und zum Austragen des gemischten Knochenzementteigs (49, 149), die Vorrichtung aufweisend
eine Kartusche (1, 51, 101) mit einem zylindrischen Innenraum (11, 61, 111), wobei das Zementpulver (4) im Innenraum (11, 61, 111) der Kartusche (1, 51, 101) angeordnet ist, die Kartusche (1, 51, 101) an einer geschlossenen Vorderseite eine Austragsöffnung aufweist, die Austragsöffnung durch einen entfernbaren Verschluss (6, 56, 106) verschlossen ist und wobei die Kartusche (1, 51, 101) an einer Rückseite ein Gewinde (8, 58, 108) aufweist,
eine Monomeraufnahme (2, 52, 102) aufweisend ein zum Gewinde (8, 58, 108) an der Rückseite der Kartusche (1, 51, 101) passendes Gegengewinde (9, 59, 109), wobei die Monomeraufnahme (2, 52, 102) in ihrem Inneren eine Kammer (10, 60, 110) bildet, die Monomeraufnahme (2, 52, 102) über ihr Gegengewinde (9, 59, 109) an das Gewinde (8, 58, 108) der Kartusche (1, 51, 101) geschraubt ist, die Monomeraufnahme (2, 52, 102) gegen die Kartusche (1, 51, 101) in Längsrichtung schraubbar beweglich ist und wobei die Monomeraufnahme (2, 52, 102) an einer Vorderseite einen zylindrischen Kolben (12, 62, 112) bildet, wobei in dem Kolben (12, 62, 112) ein für Gase und die Monomerflüssigkeit (3) durchlässiger aber für das Zementpulver (4) undurchlässiger Durchgang (14, 64, 114) vorgesehen ist, wobei der Durchgang (14, 64, 114) den Innenraum (11, 61, 111) der Kartusche (1, 51, 101) mit der Kammer (10, 60, 110) der Monomeraufnahme (2, 52, 102) verbindet, wobei der Kolben (12, 62, 112) den Innenraum (11, 61, 111) der Kartusche (1, 51, 101) an seiner Rückseite bis auf den Durchgang (14, 64, 114) dicht verschließt und wobei in der Wandung der Monomeraufnahme (2, 52, 102) wenigstens eine Begasungsöffnung (7, 57, 107) angeordnet ist, die die Kammer (10, 60, 110) mit der Umgebung der Vorrichtung verbindet,
einen Monomerflüssigkeitsbehälter (5) enthaltend die Monomerflüssigkeit (3), wobei der Monomerflüssigkeitsbehälter (5) in der Kammer (10, 60, 110) der Monomeraufnahme (2, 52, 102) angeordnet ist, und
eine Öffnungseinrichtung (18, 68, 118) zum Öffnen des Monomerflüssigkeitsbehälters (5) innerhalb der Kammer (10, 60, 110) der Monomeraufnahme (2, 52, 102), wobei die wenigstens eine Begasungsöffnung (7, 57, 107) durch die Bewegung der Öffnungseinrichtung (18, 68, 118) oder durch eine mit der Bedienung der Öffnungseinrichtung (18, 68, 118) synchronisierte Bewegung eines Verschlussmittels (20, 70, 120) verschließbar ist, bevor der Monomerflüssigkeitsbehälter (5) durch die Öffnungseinrichtung (18, 68, 118) geöffnet ist.

2. Vorrichtung nach Anspruch 1, **dadurch gekennzeichnet, dass**
die Öffnungseinrichtung (18, 68, 118) eine Sicherungseinrichtung aufweist, die ein erneutes Öffnen der wenigstens einen Begasungsöffnung (7, 57, 107) nach deren Verschließen verhindert, wobei vorzugsweise die Öffnungseinrichtung (18, 68, 118) über ein Gewinde mit der Monomeraufnahme (2, 52, 102) verbunden ist und beim Einschrauben der Öffnungseinrichtung (18, 68, 118) in die Kammer (10, 60, 110) die wenigstens eine Begasungsöffnung (7, 57, 107) verschließbar ist, wobei die Sicherungseinrichtung eine Rückdrehsicherung ist.

3. Vorrichtung nach Anspruch 1 oder 2, **dadurch gekennzeichnet, dass**
ein erstes lösbares Sicherungselement (26, 76, 126) vorgesehen ist, das eine Bedienung der Öffnungseinrichtung (18, 68, 118) verhindert, und/oder ein zweites lösbares Sicherungselement (47, 97, 147) vorgesehen ist, das ein Schrauben der Monomeraufnahme (2, 52, 102) in die Kartusche (1, 51, 101) verhindert.

4. Vorrichtung nach einem der vorangehenden Ansprüche, **dadurch gekennzeichnet, dass**
an der in die Kammer (10, 60, 110) der Monomeraufnahme (2, 52, 102) weisenden Seite des Kolbens (12, 62, 112) ein Dorn (34, 84, 134) oder eine Schneidkante (84, 134) zum Aufbrechen des Monomerflüssigkeitsbehälters (5) angeordnet ist, wobei vorzugsweise zwischen dem Kolben (12, 62, 112) und dem Monomerflüssigkeitsbehälter (5) ein zusammenpressbares Lagerungselement (36, 86, 136), insbesondere eine Feder (36, 86, 136) oder ein Schaumstoff, angeordnet ist, wobei das Lagerungselement (36, 86, 136) den Monomerflüssigkeitsbehälter (5) von dem Dorn (34, 84, 134) oder der Schneidkante (84, 134) beabstandet.

5. Vorrichtung nach einem der vorangehenden Ansprüche, **dadurch gekennzeichnet, dass**
zumindest ein loses Mischelement (45, 95, 145), insbesondere zumindest eine lose Kugel (45, 95, 145), frei beweglich im Innenraum (11, 61, 111) der Kartusche (1, 51, 101) angeordnet ist.

6. Vorrichtung nach Anspruch 5, **dadurch gekennzeichnet, dass**
an der in den Innenraum (11, 61, 111) der Kartusche (1, 51, 101) weisenden Vorderseite der Kartusche (1, 51, 101), die den Innenraum (11, 61, 111) der Kartusche (1, 51, 101) an der Vorderseite begrenzt, wenigstens ein Vorsprung (96) neben der Austragsöffnung und/oder wenigstens ein bis zur Austragsöffnung laufenden Steg (46, 96) angeordnet ist, der sich in den Innenraum (11, 61, 111) der Kartusche (1, 51, 101) erstreckt.

7. Vorrichtung nach einem der Ansprüche 5 oder 6, **dadurch gekennzeichnet, dass**
im Innenraum (11, 61, 111) der Kartusche (1, 51, 101) an dessen Vorderseite ein verformbares Aufnahmeelement (150) angeordnet ist, insbesondere eine vorformbare Ringscheibe (150) angeordnet ist, wobei vorzugsweise die Höhe des verformbaren Aufnahmeelements (150) in radialer Richtung nach außen zur Seitenwand des Innenraums (11, 61, 111) hin zunimmt.

8. Vorrichtung nach einem der Ansprüche 5 bis 7, **dadurch gekennzeichnet, dass**
das zumindest eine lose Mischelement (45, 95, 145) eine größere Dichte aufweist als Polymethylmethacrylat, vorzugsweise eine zumindest doppelt so hohe Dichte oder eine zumindest dreimal so hohe Dichte als Polymethylmethacrylat aufweist, wobei bevorzugt das zumindest eine Mischelement (45, 95, 145) aus einem Korund, aus α-Korund, aus einem Zirkonoxid, aus tetragonalem ZrO₂ oder aus mit Y₂O₃ kubisch stabilisiertem ZrO₂ besteht.

9. Vorrichtung nach einem der vorangehenden Ansprüche, **dadurch gekennzeichnet, dass**
der Monomerflüssigkeitsbehälter (5) eine Ampulle (5) aus Glas oder Kunststoff ist, wobei die Ampulle (5) einen Ampullenkörper mit einer zylindrischen Wandung aufweist und wobei die Öffnungseinrichtung (18, 68, 118) einen in der Monomeraufnahme (2, 52, 102) in einer Längsrichtung der zylindrischen Kammer (10, 60, 110) der Monomeraufnahme (2, 52, 102) beweglichen Hohlzylinder (20, 70, 120) aufweist, der mit der zylindrischen Wandung der Ampulle (5) fluchtet, so dass die Ampulle (5) mit dem Hohlzylinder (20, 70, 120) in Richtung des Innenraums (11, 61, 111) der Kartusche (1, 51, 101) drückbar ist.

10. Vorrichtung nach Anspruch 9, **dadurch gekennzeichnet, dass**
die wenigstens eine Begasungsöffnung (7, 57, 107) neben dem Hohlzylinder (20, 70, 120) in die Kammer (10, 60, 110) der Monomeraufnahme (2, 52, 102) mündet, so dass eine Bewegung des Hohlzylinders (20, 70, 120) in die Kammer (10, 60, 110) hinein die wenigstens eine Begasungsöffnung (7, 57, 107) mit einer seitlichen Wandung des Hohlzylinders (20, 70, 120) flüssigkeitsdicht oder gasdicht verschließt, wobei bevorzugt der Hohlzylinder (20, 70, 120) hierzu zumindest einen umlaufenden Dichtungsring (30, 80, 130) aufweist, besonders bevorzugt zwei umlaufende Dichtungsringe (30, 80, 130) aufweist, der oder die die wenigstens eine Begasungsöffnung (7, 57, 107) bei einer Bewegung des Hohlzylinders (20, 70, 120) in die Kammer (10, 60, 110) hinein überfahren und gegen die Kammer (10, 60, 110) abdichten.

11. Vorrichtung nach einem der vorangehenden Ansprüche, **dadurch gekennzeichnet, dass**
die den Innenraum (11, 61, 111) begrenzende Vorderseite des Kolbens (12, 62, 112) und/oder die die Vorderseite des Innenraums (11, 61, 111) begrenzende Oberfläche sich in radialer Richtung zunehmend in den Innenraum (11, 61, 111) der Kartusche (1, 51, 101) hinein erstreckt, so dass eine vordere Grundfläche und/oder eine rückseitige Grundfläche des Innenraums (11, 61, 111) keine Kante oder keine Kante mit einem Winkel von weniger als 60° aufweist, wobei vorzugsweise die vordere Grundfläche und/oder die rückseitige Grundfläche des Innenraums (11, 61, 111) eine abgerundete Form aufweisen.

12. Vorrichtung nach Anspruch 11, **dadurch gekennzeichnet, dass**
zumindest eine Kugel (45, 95, 145) als Mischelement (45, 95, 145) frei beweglich im Innenraum (11, 61, 111) der Kartusche (1, 51, 101) angeordnet ist und der Radius der zumindest einen Kugel (45, 95, 145) genauso groß oder kleiner ist als der Krümmungsradius der vorderen Grundfläche und/oder der rückseitigen Grundfläche des Innenraums (11, 61, 111) der Kartusche (1, 51, 101).

13. Vorrichtung nach einem der vorangehenden Ansprüche, **dadurch gekennzeichnet, dass**
die Öffnungseinrichtung (18, 68, 118) mit der Monomeraufnahme (2, 52, 102) über ein Gewinde (24, 74, 124) und ein Gegengewinde (9, 59, 109) verbunden sind, so dass die Öffnungseinrichtung (18, 68, 118) in die Kammer (10, 60, 110) der Monomeraufnahme (2, 52, 102) schraubbar ist und der Monomerflüssigkeitsbehälter (5) durch Hineinschrauben der Öffnungseinrichtung (18, 68, 118) in die Kammer (10, 60, 110) der Monomeraufnahme (2, 52, 102) aufbrechbar, aufschneidbar oder aufstechbar ist.

14. Vorrichtung nach einem der vorangehenden Ansprüche, **dadurch gekennzeichnet, dass**
das Gewinde (8, 58, 108) an der Rückseite der Kartusche (1, 51, 101) ein Innengewinde (8, 58, 108) ist und das Gegengewinde (9, 59, 109) der Monomeraufnahme (2, 52, 102) ein an den seitlichen Außenflächen vorgesehenes Außengewinde (9, 59, 109).

15. Vorrichtung nach Anspruch 14, **dadurch gekennzeichnet, dass**
der Kolben (12, 62, 112) einen größeren Durchmesser hat als das Innengewinde (8, 58, 108) an der Rückseite der Kartusche (1, 51, 101).

16. Vorrichtung nach einem der vorangehenden Ansprüche, **dadurch gekennzeichnet, dass**
die Vorderseite der Kartusche (1, 51, 101) mit einem Kartuschendeckel (38, 88, 138) verschlossen ist, wobei die Austragsöffnung in dem Kartuschendeckel (38, 88, 138) angeordnet ist und der Kartuschendeckel (38, 88, 138) gasdicht und flüssigkeitsdicht mit den seitlichen Wandungen der Kartusche (1, 51, 101) verbunden ist, wobei bevorzugt der Kartuschendeckel (38, 88, 138) auf ein Außengewinde (42, 92, 142) an der Vorderseite der Kartusche (1, 51, 101) aufgeschraubt ist.

17. Vorrichtung nach einem der vorangehenden Ansprüche, **dadurch gekennzeichnet, dass**
die Öffnungseinrichtung (18, 68, 118) eine auf die Rückseite der Monomeraufnahme (2, 52, 102) schraubbare Verschlusskappe (22, 72, 122) aufweist, die die Kammer (10, 60, 110) an der Rückseite gasdicht verschließt, wobei vorzugsweise ein Anschlag vorgesehen ist, der ein weiteres Aufschrauben der Verschlusskappe (22, 72, 122) auf die Monomeraufnahme (2, 52, 102) verhindert.

18. Vorrichtung nach Anspruch 17, **dadurch gekennzeichnet, dass**
an der Verschlusskappe (22, 72, 122) eine Hülse (20, 70, 120) angeordnet ist, die derart ins Innere der Kammer (10, 60, 110) steckbar oder schraubbar ist, dass sie die Kammer (10, 60, 110) an deren Rückseite gasdicht verschließt.

19. Vorrichtung nach einem der vorangehenden Ansprüche, **dadurch gekennzeichnet, dass**
in einem Zustand der Vorrichtung, bei dem die Monomeraufnahme (2, 52, 102) bis zu einem Anschlag maximal aus dem Innenraum (11, 61, 111) der Kartusche (1, 51, 101) herausgeschraubt ist, in dem derart maximal vergrößerten Innenraum (11, 61, 111) der Kartusche (1, 51, 101) ein freies Volumen über dem Zementpulver (4) vorhanden ist, das mindestens genausgroß ist wie das Volumen der Monomerflüssigkeit (3) in dem Monomerflüssigkeitsbehälter (5), vorzugsweise mindestens doppelt so groß ist wie das Volumen der Monomerflüssigkeit (3) in dem Monomerflüssigkeitsbehälter (5).

20. Verfahren zur Herstellung eines Knochenzementteigs (49, 149), insbesondere eines niedrigviskosen Polymethylmethacrylat-Knochenzementteigs (49, 149) zur Augmentation von Wirbelkörpern, wobei der Knochenzementteig (49, 149) aus einem Zementpulver (4) und einer Monomerflüssigkeit (3) hergestellt wird und das Verfahren mit einer Vorrichtung zum Herstellen des Knochenzementteigs (49, 149) und zum Austragen des gemischten Knochenzementteigs (49, 149) durchgeführt wird, **gekennzeichnet durch** die folgenden Schritte:
A) Bedienen einer Öffnungseinrichtung (18, 68, 118) der Vorrichtung, wobei durch das Bedienen der Öffnungseinrichtung (18, 68, 118) wenigstens eine Begasungsöffnung (7, 57, 107) in einer Wandung einer Monomeraufnahme (2, 52, 102) der Vorrichtung, die die Umgebung der Vorrichtung mit einer Kammer (10, 60, 110) im Inneren der Monomeraufnahme (2, 52, 102) gasdurchlässig verbindet, gasdicht verschlossen wird und anschließend ein Monomerflüssigkeitsbehälter (5) enthaltend die Monomerflüssigkeit (3) innerhalb der Kammer (10, 60, 110) der Monomeraufnahme (2, 52, 102) geöffnet wird,
B) Überführen der Monomerflüssigkeit (3) aus der Monomeraufnahme (2, 52, 102) durch einen für Gase und die Monomerflüssigkeit (3) durchlässigen aber für das Zementpulver (4) undurchlässigen Durchgang (14, 64, 114), der in einem Kolben (12, 62, 112) angeordnet ist, welcher an der Vorderseite der Monomeraufnahme (2, 52, 102) ausgebildet ist, in einen Innenraum (11, 61, 111) einer Kartusche (1, 51, 101), in dem das Zementpulver (4) enthalten ist,
C) Mischen der Monomerflüssigkeit (3) und des Zementpulvers (4) in dem Innenraum (11, 61, 111) der Kartusche (1, 51, 101) zu einem Knochenzementteig (49, 149),
D) Herausdrücken von Gas, das in dem Knochenzementteig (49, 149) verteilt ist, aus dem Innenraum (11, 61, 111) der Kartusche (1, 51, 101), durch Hineinschrauben der Monomeraufnahme (2, 52, 102) mit dem Kolben (12, 62, 112) an der Vorderseite der Monomeraufnahme (2, 52, 102) von der Rückseite des Innenraums (11, 61, 111) der Kartusche (1, 51, 101) in die Kartusche (1, 51, 101), und
E) Auspressen des fertig gemischten Knochenzementteigs (49, 149) aus dem Innenraum (11, 61, 111) der Kartusche (1, 51, 101) durch eine dem Kolben (12, 62, 112) gegenüberliegende Austragsöffnung der Kartusche (1, 51, 101) durch weiteres Hineinschrauben der Monomeraufnahme (2, 52, 102) in die Kartusche (1, 51, 101).

21. Verfahren nach Anspruch 20, **dadurch gekennzeichnet, dass**
das Verfahren mit einer Vorrichtung nach einem der Ansprüche 1 bis 19 durchgeführt wird.

22. Verfahren nach Anspruch 20 oder 21, **dadurch gekennzeichnet, dass**
in Schritt C) das Mischen der Monomerflüssigkeit (3) und des Zementpulvers (4) durch ein Schütteln der Kartusche (1, 51, 101) erfolgt, wobei in dem Innenraum (11, 61, 111) der Kartusche (1, 51, 101) zusätzlich zum Zementpulver (4) und der Monomerflüssigkeit (3) ein Gas und zumindest ein loses und im Innenraum (11, 61, 111) der Kartusche (1, 51, 101) frei bewegliches Mischelement (45, 95, 145), insbesondere zumindest eine lose Kugel (45, 95, 145), enthalten ist, wobei das zumindest eine Mischelement (45, 95, 145) in dem Innenraum (11, 61, 111) der Kartusche (1, 51, 101) durch das Schütteln geschleudert wird und dadurch eine Durchmischung des Zementpulvers (4) mit der Monomerflüssigkeit (3) erfolgt.

23. Verfahren nach einem der Ansprüche 20 bis 22, **dadurch gekennzeichnet, dass**
die Öffnungseinrichtung (18, 68, 118) in die Monomeraufnahme (2, 52, 102) hineingeschraubt wird und durch die Bewegung der Öffnungseinrichtung (18, 68, 118) der Monomerflüssigkeitsbehälter (5) geöffnet und die wenigstens eine Begasungsöffnung (7, 57, 107), die in einer Wandung der Monomeraufnahme (2, 52, 102) angeordnet ist, zuvor verschlossen wird.

24. Verfahren nach einem der Ansprüche 20 bis 23, **dadurch gekennzeichnet, dass**
der Monomerflüssigkeitsbehälter (5) eine Ampulle (5) aus Glas oder aus einem Kunststoff ist und beim Öffnen der Ampulle (5) in Schritt A) die Ampulle (5) auf einen Dorn (34, 84, 134) oder eine Schneidkante (84, 134) an der in die Kammer (10, 60, 110) der Monomeraufnahme (2, 52, 102) weisenden Seite des Kolbens (12, 62, 112) gedrückt wird, so dass die Ampulle (5) an dieser Seite aufgebrochen wird und die Monomerflüssigkeit (3) im Bereich des Durchgangs (14, 64, 114) aus der geöffneten Ampulle (5) austritt.

25. Verfahren nach einem der Ansprüche 20 bis 24, **dadurch gekennzeichnet, dass**
während des Schritts B), bevorzugt während der Schritte A) und B), die Monomeraufnahme (2, 52, 102) oberhalb der Kartusche (1, 51, 101) gehalten wird, so dass die Monomerflüssigkeit (3) angetrieben von der Schwerkraft aus der Kammer (10, 60, 110) durch den Durchgang (14, 64, 114) in den Innenraum (11, 61, 111) der Kartusche (1, 51, 101) fließt.

26. Verfahren nach einem der Ansprüche 20 bis 25, **dadurch gekennzeichnet, dass**
vor Schritt A) ein Schritt erfolgt, bei dem ein erstes Sicherungselement (26, 76, 126) gelöst wird, das eine Bedienung der Öffnungseinrichtung (18, 68, 118) verhindert, und/oder ein zweites Sicherungselement (47, 97, 147) gelöst wird, das ein Schrauben der Monomeraufnahme (2, 52, 102) in der Kartusche (1, 51, 101) verhindert.

27. Verfahren nach einem der Ansprüche 20 bis 26, **dadurch gekennzeichnet, dass**
zum Überführen der Monomerflüssigkeit (3) von der Kammer (10, 60, 110) in den Innenraum (11, 61, 111) der Kartusche (1, 51, 101) in Schritt B) die Monomeraufnahme (2, 52, 102) aus dem Innenraum (11, 61, 111) der Kartusche (1, 51, 101) herausgeschraubt wird, so dass sich der Innenraum (11, 61, 111) der Kartusche (1, 51, 101) vergrößert und in dem Innenraum (11, 61, 111) der Kartusche (1, 51, 101) ein Unterdruck entsteht, mit dem die Monomerflüssigkeit (3) aus der Kammer (10, 60, 110) in den Innenraum (11, 61, 111) der Kartusche (1, 51, 101) gesaugt wird.

## Claims

1. A device for producing a bone cement dough (49, 149) from a monomer liquid (3) and a cement powder (4) as starting components of the bone cement dough (49, 149), and for dispensing the bone cement dough (49, 149), the device comprising
a cartridge (1, 51, 101) with a cylindrical internal space (11, 61, 111), whereby the cement powder (4) is arranged in the internal space (11, 61, 111) of the cartridge (1, 51, 101), the cartridge comprises a closed dispensing opening on a closed front side, the dispensing opening is closed by a removable closure (6, 56, 106), and whereby the cartridge (1, 51, 101) comprises a thread (8, 58, 108) on a rear side,
a monomer receptacle (2, 52, 102) comprising a counter-thread (9, 59, 109) fitting the thread (8, 58, 108) on the rear side of the cartridge (1, 51, 101), whereby the monomer receptacle (2, 52, 102) forms on its inside a chamber (10, 60, 110), the monomer receptacle (2, 52, 102) is screwed by its counter-thread (9, 59, 109) to the thread (8, 58, 108) of the cartridge (1, 51, 101), the monomer receptacle (2, 52, 102) is movable in longitudinal direction against the cartridge (1, 51, 101) in a screw-like fashion, and whereby the monomer receptacle (2, 52, 102) forms on a front side a cylindrical plunger (12, 62, 112), whereby a passage (14, 64, 114) that is permeable to gases and the monomer liquid (3), but impermeable to the cement powder (4) is provided in the plunger (12, 62, 112), whereby the passage (14, 64, 114) connects the internal space (11, 61, 111) of the cartridge (1, 51, 101) to the chamber (10, 60, 110) of the monomer receptacle (2, 52, 102), whereby the plunger (12, 62, 112) tightly closes the internal space (11, 61, 111) of the cartridge (1, 51, 101) on its rear side except for the passage (14, 64, 114), and whereby at least one fumigation opening (7, 57, 107) connecting the chamber (10, 60, 110) to the surroundings of the device is arranged in the wall of the monomer receptacle (2, 52, 102),
a monomer liquid container (5) containing the monomer liquid (3), whereby the monomer liquid container (5) is arranged in the chamber (10, 60, 110) of the monomer receptacle (2, 52, 102), and
an opening facility (18, 68, 118) for opening of the monomer liquid container (5) within the chamber (10, 60, 110) of the monomer receptacle (2, 52, 102), whereby the at least one fumigation opening (7, 57, 107) can be closed by the motion of the opening facility (18, 68, 118) or by a motion of a closing means (20, 70, 120) that is synchronised to the operation of the opening facility (18, 68, 118), before the monomer liquid container (5) is opened by the opening facility (18, 68, 118).

2. The device according to claim 1, **characterised in that**
the opening facility (18, 68, 118) comprises a securing facility that prevents the at least one fumigation opening (7, 57, 107) from being re-opened after being closed, whereby the opening facility (18, 68, 118) preferably is connected to the monomer receptacle (2, 52, 102) via a thread, and the at least one fumigation opening (7, 57, 107) can be closed while the opening facility (18, 68, 118) is being screwed into the chamber (10, 60, 110), whereby the securing facility is a reverse rotation lock.

3. The device according to claim 1 or 2, **characterised in that**
a first releasable securing element (26, 76, 126) preventing the opening facility (18, 68, 118) from being operated is provided and/or a second releasable securing element (47, 97, 147) preventing the monomer receptacle (2, 52, 102) from being screwed into the cartridge (1, 51, 101) is provided.

4. The device according to any one of the preceding claims, **characterised in that**
a mandrel (34, 84, 134) or a cutting edge (84, 134) for breaking open the monomer liquid container (5) is provided on the side of the plunger (12, 62, 112) pointing into the chamber (10, 60, 110) of the monomer receptacle (2, 52, 102), whereby, preferably, a compressible positioning element (36, 86, 136), in particular a spring (36, 86, 136) or a foam, is arranged between the plunger (12, 62, 112) and the monomer liquid container (5), whereby the positioning element (36, 86, 136) keeps the monomer liquid container (5) at a distance from the mandrel (34, 84, 134) or the cutting edge (84, 134).

5. The device according to any one of the preceding claims, **characterised in that** at least one unattached mixing element (45, 95, 145), in particular at least one ball (45, 95, 145), is arranged such as to be freely mobile in the internal space (11, 61, 111) of the cartridge (1, 51, 101).

6. The device according to claim 5, **characterised in that**
the front side of the cartridge (1, 51, 101) that points into the internal space (11, 61, 111) of the cartridge (1, 51, 101) and borders the internal space (11, 61, 111) of the cartridge (1, 51, 101) on the front side has arranged on it at least one projection (96) next to the dispensing opening and/or at least one fin (46, 96) that runs to the dispensing opening and extends into the internal space (11, 61, 111) of the cartridge (1, 51, 101).

7. The device according to any one of the claims 5 or 6, **characterised in that** a deformable receiving element (150), in particular a deformable annular disc (150), is arranged in the internal space (11, 61, 111) of the cartridge (1, 51, 101), whereby the height of the deformable receiving element (150) preferably increases in radial direction outwards towards the side wall of the internal space (11, 61, 111).

8. The device according to any one of the claims 5 to 7, **characterised in that** the at least one unattached mixing element (45, 95, 145) comprises a density higher than that of polymethylmethacrylate, preferably a density that is at least two times or at least three times the density of polymethylmethacrylate, whereby the at least one mixing element (45, 95, 145) preferably consists of a corundom, of alpha-corundom, of a zirconium oxide, of tetragonal ZrO₂, or of ZrO₂ cubically stabilised with Y₂O₃

9. The device according to any one of the preceding claims, **characterised in that** the monomer liquid container (5) is an ampoule (5) made of glass or plastics, whereby the ampoule (5) comprises an ampoule body with a cylindrical wall, and whereby the opening facility (18, 68, 118) comprises a hollow cylinder (20, 70, 120) that is mobile in the monomer receptacle (2, 52, 102) in a longitudinal direction of the cylindrical chamber (10, 60, 110) of the monomer receptacle (2, 52, 102) and is flush with the cylindrical wall of the ampoule (5) such that the ampoule (5) can be pushed with the hollow cylinder (20, 70, 120) in the direction of the internal space (11, 61, 111) of the cartridge (1, 51, 101).

10. The device according to claim 9, **characterised in that**
the at least one fumigation opening (7, 57, 107) merges next to the hollow cylinder (20, 70, 120) into the chamber (10, 60, 110) of the monomer receptacle (2, 52, 102) such that a motion of the hollow cylinder (20, 70, 120) into the chamber (10, 60, 110) closes the at least one fumigation opening (7, 57, 107) by means of a lateral wall of the hollow cylinder (20, 70, 120) in liquid-tight or gas-tight manner, whereby the hollow cylinder (20, 70, 120) preferably comprises at least one circumferential sealing ring (30, 80, 130) for this purpose, particularly preferably comprises two circumferential sealing rings (30, 80, 130) for this purpose, which travels or travel over the at least one fumigation opening (7, 57, 107) upon a motion of the hollow cylinder (20, 70, 120) into the chamber (10, 60, 110), and seals or seal it with respect to the chamber (10, 60, 110).

11. The device according to any one of the preceding claims, **characterised in that** the front side of the plunger (12, 62, 112) bordering the internal space (11, 61, 111) and/or the surface bordering the front side of the internal space (11, 61, 111) extends, progressively in radial direction, into the internal space (11, 61, 111) of the cartridge (1, 51, 101) such that a frontal base area and/or a rear-side base area of the internal space (11, 61, 111) comprises no edge or no edge with an angle of less than 60°, whereby the front base area and/or the rear-side base area of the internal space (11, 61, 111) preferably comprise a rounded shape.

12. The device according to claim 11, **characterised in that**
at least one ball (45, 95, 145) as mixing element (45, 95, 145) is arranged such as to be freely mobile in the internal space (11, 61, 111) of the cartridge (1, 51, 101) and the radius of the at least one ball (45, 95, 145) is equal to or smaller than the radius of curvature of the front base area and/or rear-side base area of the internal space (11, 61, 111) of the cartridge (1, 51, 101).

13. The device according to any one of the preceding claims, **characterised in that** the opening facility (18, 68, 118) is connected to the monomer receptacle (2, 52, 102) by means of a thread (24, 74, 124) and a counter-thread (9, 59, 109) such that the opening facility (18, 68, 118) can be screwed into the chamber (10, 60, 110) of the monomer receptacle (2, 52, 102) and such that the monomer liquid container (5) can be broken, cut or punctured such as to be open by screwing the opening facility (18, 68, 118) into the chamber (10, 60, 110) of the monomer receptacle (2, 52, 102).

14. The device according to any one of the preceding claims, **characterised in that** the thread (8, 58, 108) on the rear side of the cartridge (1, 51, 101) is an internal thread (8, 58, 108), and the counter-thread (9, 59, 109) of the monomer receptacle (2, 52, 102) is an external thread (9, 59, 109) provided on the lateral external surfaces.

15. The device according to claim 14, **characterised in that**
the plunger (12, 62, 112) has a larger diameter than the internal thread (8, 58, 108) on the rear side of the cartridge (1, 51, 101).

16. The device according to any one of the preceding claims, **characterised in that** the front side of the cartridge (1, 51, 101) is closed by means of a cartridge lid (38, 88, 138), whereby the dispensing opening is arranged in said cartridge lid (38, 88, 138) and the cartridge lid (38, 88, 138) is connected in gas-tight and liquid-tight manner to the lateral walls of the cartridge (1, 51, 101), whereby the cartridge lid (38, 88, 138) preferably is screwed onto an external thread (42, 92, 142) on the front side of the cartridge (1, 51, 101).

17. The device according to any one of the preceding claims, **characterised in that** the opening facility (18, 68, 118) comprises a closure cap (22, 72, 122) that can be screwed onto the rear side of the monomer receptacle (2, 52, 102) and closes the chamber (10, 60, 110) on its rear side in gas-tight manner, whereby it is preferred to provide a limit stop that prevents the closure cap (22, 72, 122) from being screwed further onto the monomer receptacle (2, 52, 102).

18. The device according to claim 17, **characterised in that**
a sleeve (20, 70, 120) is arranged on the closure cap (22, 72, 122) and can be appropriately plugged or screwed into the inside of the chamber (10, 60, 110) such that it closes the chamber (10, 60, 110) on its rear side in gas-tight manner.

19. The device according to any one of the preceding claims, **characterised in that**,
in a state of the device, in which the monomer receptacle (2, 52, 102) is screwed out maximally up to a limit stop from the internal space (11, 61, 111) of the cartridge (1, 51, 101), a free volume is present above the cement powder (4) in the thus maximally enlarged internal space (11, 61, 111) of the cartridge (1, 51, 101), with the free volume being at least equal to the volume of the monomer liquid (3) in the monomer liquid container (5), preferably being at least twice the volume of the monomer (3) in the monomer liquid container (5).

20. A method for producing a bone cement dough (49, 149), in particular a low viscosity polymethylmethacrylate bone cement dough (49, 149) for augmentation of vertebral bodies, whereby the bone cement dough (49, 149) is produced from a cement powder (4) and a monomer liquid (3), and the method is implemented with a device for producing the bone cement dough (49, 149), and for dispensing the mixed bone cement dough (49, 149), **characterised by** the following steps of:
A) Operating an opening facility (18, 68, 118) of the device, whereby the operation of the opening facility (18, 68, 118) closes, in gas-tight manner, at least one fumigation opening (7, 57, 107) in a wall of the monomer receptacle (2, 52, 102) of the device that connects the surroundings of the device to a chamber (10, 60, 110) on the inside of the monomer receptacle (2, 52, 102) in gas-permeable manner, and subsequently opening a monomer liquid container (5) containing the monomer liquid (3) within the chamber (10, 60, 110) of the monomer receptacle (2, 52, 102);
B) transferring the monomer liquid (3) from the monomer receptacle (2, 52, 102) through a passage (14, 64, 114) that is permeable to gases and the monomer liquid (3), but is impermeable to the cement powder (4), and is arranged in a plunger (12, 62, 112) provided on the front side of the monomer receptacle (2, 52, 102), into an internal space (11, 61, 111) of a cartridge (1, 51, 101) containing the cement powder (4);
C) mixing the monomer liquid (3) and the cement powder (4) in the internal space (11, 61, 111) of the cartridge (1, 51, 101) to form a bone cement dough (49, 149);
D) expelling gas distributed throughout the bone cement dough (49, 149) from the internal space (11, 61, 111) of the cartridge (1, 51, 101) by screwing the monomer receptacle (2, 52, 102) with the plunger (12, 62, 112) on the front side of the monomer receptacle (2, 52, 102) from the rear side of the internal space (11, 61, 111) of the cartridge (1, 51, 101) into the cartridge (1, 51, 101); and
E) extruding the ready-mixed bone cement dough (49, 149) from the internal space (11, 61, 111) of the cartridge (1, 51, 101) through a dispensing opening of the cartridge (1, 51, 101) that is situated opposite from the plunger (12, 62, 112) by screwing the monomer receptacle (2, 52, 102) further into the cartridge (1, 51, 101).

21. The method according to claim 20, **characterised in that**
the method is implemented with a device according to any one of the claims 1 to 19.

22. The method according to claim 20 or 21, **characterised in that**
the mixing of the monomer liquid (3) and the cement powder (4) in step C) takes place through shaking the cartridge (1, 51, 101), whereby the internal space (11, 61, 111) of the cartridge (1, 51, 101) contains, in addition to the cement powder (4) and the monomer liquid (3), a gas and at least one mixing element (45, 95, 145) that is unattached and freely mobile in the internal space (11, 61, 111) of the cartridge (1, 51, 101), in particular at least one unattached ball (45, 95, 145), whereby the at least one mixing element (45, 95, 145) is hurled around in the internal space (11, 61, 111) of the cartridge (1, 51, 101) due to the shaking, and a mixing of the cement powder (4) and the monomer liquid (3) thus takes place.

23. The method according to any one of the claims 20 to 22, **characterised in that** the opening facility (18, 68, 118) is being screwed into the monomer receptacle (2, 52, 102), and the motion of the opening facility (18, 68, 118) opens the monomer liquid container (5), and the at least one fumigation opening (7, 57, 107), arranged in a wall of the monomer receptacle (2, 52, 102), is being closed beforehand.

24. The method according to any one of the claims 20 to 23, **characterised in that** the monomer liquid container (5) is an ampoule (5) made of glass or a plastics, and **in that**, when the ampoule (5) is being opened in step A), the ampoule (5) is being pushed onto a mandrel (34, 84, 134) or a cutting edge (84, 134) on the side of the plunger (12, 62, 112) pointing into the chamber (10, 60, 110) of the monomer receptacle (2, 52, 102), such that the ampoule (5) is broken open on said side and the monomer liquid (3) leaks from the opened ampoule (5) in the area of the passage (14, 64, 114).

25. The method according to any one of the claims 20 to 24, **characterised in that**, during step B), preferably during steps A) and B), the monomer receptacle (2, 52, 102) is being held above the cartridge (1, 51, 101) such that the monomer liquid (3), driven by gravity, flows from the chamber (10, 60, 110) through the passage (14, 64, 114) into the internal space (11, 61, 111) of the cartridge (1, 51, 101).

26. The method according to any one of the claims 20 to 25, **characterised in that** step A) is preceded by a step, in which a first securing element (26, 76, 126) preventing the opening facility (18, 68, 118) from being operated is released, and/or a second securing element (47, 97, 147) preventing the monomer receptacle (2, 52, 102) from being screwed in or out within the cartridge (1, 51, 101) is released.

27. The method according to any one of the claims 20 to 26, **characterised in that**, in order to transfer the monomer liquid (3) from the chamber (10, 60, 110) into the internal space (11, 61, 111) of the cartridge (1, 51, 101) in step B), the monomer receptacle (2, 52, 102) is screwed out from the internal space (11, 61, 111) of the cartridge (1, 51, 101) such that the internal space (11, 61, 111) of the cartridge (1, 51, 101) becomes enlarged and a negative pressure is generated in the internal space (11, 61, 111) of the cartridge (1, 51, 101) by means of which the monomer liquid (3) is aspirated from the chamber (10, 60, 110) into the internal space (11, 61, 111) of the cartridge (1, 51, 101).

## Revendications

1. Dispositif pour la fabrication d'une pâte de ciment osseux (49, 149) à partir d'un liquide monomère (3) et d'une poudre de ciment (4) comme composants de départ de la pâte de ciment osseux (49, 149) et pour le déversement de la pâte de ciment osseux (49, 149) mélangée, le dispositif présentant une cartouche (1, 51, 101) avec un espace intérieur (11, 61, 111) cylindrique, la poudre de ciment (4) étant disposée dans l'espace intérieur (11, 61, 111) de la cartouche (1, 51, 101), la cartouche (1, 51, 101) présentant, sur une face frontale fermée, un orifice de sortie, l'orifice de sortie étant obturé par un bouchon (6, 56, 106) amovible et la cartouche (1, 51, 101) présentant, sur une face arrière, un filetage (8, 58, 108),
une entrée de monomère (2, 52, 102) présentant un contre-filetage (9, 59, 109) adapté au filetage (8, 58, 108) sur la face arrière de la cartouche (1, 51, 101), l'entrée de monomère (2, 52, 102) formant, dans son intérieur, un compartiment (10, 60, 110), l'entrée de monomère (2, 52, 102) étant vissée sur le filetage (8, 58, 108) de la cartouche (1, 51, 101) par son contre-filetage (9, 59, 109), l'entrée de monomère (2, 52, 102) pouvant être déplacée en étant vissée dans le sens longitudinal contre la cartouche (1, 51, 101) et l'entrée de monomère (2, 52, 102) formant, sur une face frontale, un piston (12, 62, 112) cylindrique, un passage (14, 64, 114) perméable aux gaz et au liquide monomère (3) mais imperméable à la poudre de ciment (4) étant prévu dans le piston (12, 62, 112), le passage (14, 64, 114) reliant l'espace intérieur (11, 61, 111) de la cartouche (1, 51, 101) avec le compartiment (10, 60, 110) de l'entrée de monomère (2, 52, 102), le piston (12, 62, 112) fermant hermétiquement l'espace intérieur (11, 61, 111) de la cartouche (1, 51, 101) sur sa face arrière jusqu'au passage (14, 64, 114) et au moins un orifice d'injection de gaz (7, 57, 107), lequel relie le compartiment (10, 60, 110) à l'environnement du dispositif, étant disposé dans la paroi de l'entrée de monomère (2, 52, 102),
un réservoir de liquide monomère (5) contenant le liquide monomère (3), le réservoir de liquide monomère (5) étant disposé dans le compartiment (10, 60, 110) de l'entrée de monomère (2, 52, 102) et
un système d'ouverture (18, 68, 118) pour l'ouverture du réservoir de liquide monomère (5) à l'intérieur du compartiment (10, 60, 110) de l'entrée de monomère (2, 52, 102), au moins un orifice d'injection de gaz (7, 57, 107) pouvant être obturé par le déplacement du système d'ouverture (18, 68, 118) ou par un déplacement d'un moyen de fermeture (20, 70, 120) synchronisé avec l'utilisation du système d'ouverture (18, 68, 118) avant que le réservoir de liquide monomère (5) ne soit ouvert par le système d'ouverture (18, 68, 118).

2. Dispositif conformément à la revendication n°1, **caractérisé en ce que**
le système d'ouverture (18, 68, 118) présente un dispositif de sécurité qui empêche une nouvelle ouverture d'au moins un orifice d'injection de gaz (7, 57, 107) après son obturation, le système d'ouverture (18, 68, 118) étant préférablement relié à l'entrée de monomère (2, 52, 102) par un filetage et au moins un orifice d'injection de gaz (7, 57, 107) pouvant être obturé lors du vissage du système d'ouverture (18, 68, 118) dans le compartiment (10, 60, 110), le dispositif de sécurité étant une protection contre la rotation en sens inverse.

3. Dispositif conformément à la revendication n°1 ou n°2, **caractérisé en ce que** un premier élément de sécurité (26, 76, 126) amovible, empêchant une utilisation du système d'ouverture (18, 68, 118), et/ou un deuxième élément de sécurité (47, 97, 147) amovible, empêchant un vissage de l'entrée de monomère (2, 52, 102) dans la cartouche (1, 51, 101), est prévu.

4. Dispositif conformément à l'une des revendications précédentes, **caractérisé en ce que**,
sur la face du piston (12, 62, 112) tournée vers le compartiment (10, 60, 110) de l'entrée de monomère (2, 52, 102), un mandrin (34, 84, 134) ou un bord coupant (84, 134) est disposé pour ouvrir par rupture le réservoir de liquide monomère (5), un élément de suspension (36, 86, 136) compressible, en particulier un ressort (36, 86, 136) ou une mousse synthétique, est disposé préférablement entre le piston (12, 62, 112) et le réservoir de liquide monomère (5), l'élément de suspension (36, 86, 136) espaçant le réservoir de liquide monomère (5) du mandrin (34, 84, 134) ou du bord coupant (84, 134).

5. Dispositif conformément à l'une des revendications précédentes, **caractérisé en ce que**
au moins un élément de mélange (45, 95, 145) libre, en particulier au moins une bille (45, 95, 145) libre, est disposé de façon mobile dans l'espace intérieur (11, 61, 111) de la cartouche (1, 51, 101).

6. Dispositif conformément à la revendication n°5, **caractérisé en ce que**
au moins une saillie (96) à côté de l'orifice de sortie et/ou au moins une entretoise (46, 96) allant jusqu'à l'orifice de sortie, laquelle s'étend dans l'espace intérieur (11, 61, 111) de la cartouche (1, 51, 101), est disposée sur la face frontale de la cartouche (1, 51, 101) tournée vers l'espace intérieur (11, 61, 111) de la cartouche (1, 51, 101), laquelle limite l'espace intérieur (11, 61, 111) de la cartouche (1, 51, 101) sur la face frontale.

7. Dispositif conformément à l'une des revendications n°5 ou n°6, **caractérisé en ce que**
un élément d'entrée (150) malléable, en particulier une rondelle annulaire (150) préformable, est disposé dans l'espace intérieur (11, 61, 111) de la cartouche (1, 51, 101) sur sa face frontale, la hauteur de l'élément d'entrée (150) malléable augmentant préférablement vers l'extérieur par rapport à la paroi latérale de l'espace intérieur (11, 61, 111) dans le sens radial.

8. Dispositif conformément à l'une des revendications n°5 à n°7, **caractérisé en ce que**
au moins un élément de mélange (45, 95, 145) libre présente une plus grande densité que le méthacrylate de polyméthyle, préférablement une densité au moins deux fois plus élevée ou une densité au moins trois fois plus élevée que celle du méthacrylate de polyméthyle, préférablement au moins un élément de mélange (45, 95, 145) se composant d'un corindon, de corindon a, d'un oxyde de zirconium, de ZrO₂ tétragonal ou de ZrO₂ stabilisé cubiquement avec Y₂O₃.

9. Dispositif conformément à l'une des revendications précédentes, **caractérisé en ce que**
le réservoir de liquide monomère (5) est une ampoule (5) en verre ou en matière plastique, l'ampoule (5) présentant un corps d'ampoule avec une paroi cylindrique et le système d'ouverture (18, 68, 118) présentant un cylindre creux (20, 70, 120) mobile dans un sens longitudinal du compartiment cylindrique (10, 60, 110) de l'entrée de monomère (2, 52, 102) dans l'entrée de monomère (2, 52, 102), lequel est aligné avec la paroi cylindrique de l'ampoule (5) de telle manière à ce que l'ampoule (5) puisse être poussée avec le cylindre creux (20, 70, 120) vers l'espace intérieur (11, 61, 111) de la cartouche (1, 51, 101).

10. Dispositif conformément à la revendication n°9, **caractérisé en ce que**
au moins un orifice d'injection de gaz (7, 57, 107) à côté du cylindre creux (20, 70, 120) débouche dans le compartiment (10, 60, 110) de l'entrée de monomère (2, 52, 102) de telle manière à ce qu'un déplacement du cylindre creux (20, 70, 120) dans le compartiment (10, 60, 110) obture hermétiquement aux liquides ou aux gaz au moins un orifice d'injection de gaz (7, 57, 107) avec une paroi latérale du cylindre creux (20, 70, 120), le cylindre creux (20, 70, 120) présentant préférablement au moins un joint d'étanchéité circulaire (30, 80, 130), en particulier préférablement deux joints d'étanchéité circulaires (30, 80, 130), lequel ou lesquels écrasent au moins un orifice d'injection de gaz (7, 57, 107) lors d'un déplacement du cylindre creux (20, 70, 120) dans le compartiment (10, 60, 110) et l'étanchéifient contre le compartiment (10, 60, 110).

11. Dispositif conformément à l'une des revendications précédentes, **caractérisé en ce que**
la face frontale du piston (12, 62, 112) limitant l'espace intérieur (11, 61, 111) et/ou la surface limitant la face frontale de l'espace intérieur (11, 61, 111) s'étend dans le sens radial en augmentant dans l'espace intérieur (11, 61, 111) de la cartouche (1, 51, 101) de telle manière à ce qu'une surface de base frontale et/ou une surface de base arrière de l'espace intérieur (11, 61, 111) ne présente pas de bord ou pas de bord avec un angle inférieur à 60°, la surface de base frontale et/ou la surface de base arrière de l'espace intérieur (11, 61, 111) présentant préférablement une forme arrondie.

12. Dispositif conformément à la revendication n°11, **caractérisé en ce que**
au moins une bille (45, 95, 145) est disposée comme élément de mélange (45, 95, 145) de façon mobile dans l'espace intérieur (11, 61, 111) de la cartouche (1, 51, 101) et que le rayon d'au moins une bille (45, 95, 145) est aussi grand ou plus petit que le rayon de courbure de la surface de base frontale et/ou de la surface de base arrière de l'espace intérieur (11, 61, 111) de la cartouche (1, 51, 101).

13. Dispositif conformément à l'une des revendications précédentes, **caractérisé en ce que**
le système d'ouverture (18, 68, 118) est relié à l'entrée de monomère (2, 52, 102) par un filetage (24, 74, 124) et un contre-filetage (9, 59, 109) de telle manière à ce que le système d'ouverture (18, 68, 118) puisse être vissé dans le compartiment (10, 60, 110) de l'entrée de monomère (2, 52, 102) et à ce que le réservoir de liquide monomère (5) puisse être ouvert en étant rompu, coupé ou percé par vissage du système d'ouverture (18, 68, 118) dans le compartiment (10, 60, 110) de l'entrée de monomère (2, 52, 102).

14. Dispositif conformément à l'une des revendications précédentes, **caractérisé en ce que**
le filetage (8, 58, 108) sur la face arrière de la cartouche (1, 51, 101) est un filetage intérieur (8, 58, 108) et que le contre-filetage (9, 59, 109) de l'entrée de monomère (2, 52, 102) est un filetage extérieur (9, 59, 109) prévu sur les faces extérieures latérales.

15. Dispositif conformément à la revendication n°14, **caractérisé en ce que**
le piston (12, 62,112) présente un plus grand diamètre que le filetage intérieur (8, 58, 108) sur la face arrière de la cartouche (1, 51, 101).

16. Dispositif conformément à l'une des revendications précédentes, **caractérisé en ce que**
la face frontale de la cartouche (1, 51, 101) est obturée avec un couvercle de cartouche (38, 88, 138), l'orifice de sortie étant disposé dans le couvercle de cartouche (38, 88, 138) et le couvercle de cartouche (38, 88, 138) étant relié hermétiquement aux gaz et aux liquides avec les parois latérales de la cartouche (1, 51, 101), le couvercle de cartouche (38, 88, 138) étant préférablement vissé sur un filetage extérieur (42, 92, 142) sur la face frontale de la cartouche (1, 51, 101).

17. Dispositif conformément à l'une des revendications précédentes, **caractérisé en ce que**
le système d'ouverture (18, 68, 118) présente un capuchon de fermeture (22, 72, 122) vissable sur la face arrière de l'entrée de monomère (2, 52, 102), lequel obture hermétiquement aux gaz le compartiment (10, 60, 110) sur la face arrière, une butée empêchant un vissage supplémentaire du capuchon de fermeture (22, 72, 122) sur l'entrée de monomère (2, 52, 102), étant préférablement prévue.

18. Dispositif conformément à la revendication n°17, **caractérisé en ce que**
une gaine (20, 70, 120) qui peut être enfichée ou vissée à l'intérieur du compartiment (10, 60, 110) de façon à obturer hermétiquement aux gaz le compartiment (10, 60, 110) sur sa face arrière, est disposée sur le capuchon de fermeture (22, 72, 122).

19. Dispositif conformément à l'une des revendications précédentes, **caractérisé en ce que**,
dans un état du dispositif dans lequel l'entrée de monomère (2, 52, 102) est dévissée jusqu'à une butée maximum de l'espace intérieur (11, 61, 111) de la cartouche (1, 51, 101), dans l'espace intérieur (11, 61, 111) ainsi agrandi au maximum de la cartouche (1, 51, 101), un volume libre au-dessus de la poudre de ciment (4) est présent, lequel est au moins aussi grand que le volume du liquide monomère (3) dans le réservoir de liquide monomère (5), préférablement au moins deux fois plus grand que le volume du liquide monomère (3) dans le réservoir de liquide monomère (5).

20. Procédé pour la fabrication d'une pâte de ciment osseux (49, 149), en particulier d'une pâte de ciment osseux (49, 149) de méthacrylate de polyméthyle à faible viscosité pour l'augmentation de corps vertébraux, la pâte de ciment osseux (49, 149) étant fabriquée à partir d'une poudre de ciment (4) et d'un liquide monomère (3) et le procédé étant réalisé avec un dispositif pour la fabrication de la pâte de ciment osseux (49, 149) et pour le déversement de la pâte de ciment osseux (49, 149) mélangée, **caractérisé par** les étapes suivantes :
A) Utilisation d'un système d'ouverture (18, 68, 118) du dispositif, au moins un orifice d'injection de gaz (7, 57, 107) dans une paroi d'une entrée de monomère (2, 52, 102) du dispositif qui relie de manière perméable aux gaz l'environnement du dispositif à un compartiment (10, 60, 110) à l'intérieur de l'entrée de monomère (2, 52, 102) étant obturé hermétiquement aux gaz par l'utilisation d'un système d'ouverture (18, 68, 118), puis un réservoir de liquide monomère (5) contenant le liquide monomère (3) à l'intérieur du compartiment (10, 60, 110) de l'entrée de monomère (2, 52, 102) étant ouvert,
B) Transfert du liquide monomère (3) depuis l'entrée de monomère (2, 52, 102) par un passage (14, 64, 114) perméable aux gaz et au liquide monomère (3) mais imperméable à la poudre de ciment (4), lequel est disposé dans un piston (12, 62, 112) qui est formé sur la face frontale de l'entrée de monomère (2, 52, 102), dans un espace intérieur (11, 61, 111) d'une cartouche (1, 51, 101) dans lequel est contenue la poudre de ciment (4),
C) Mélange du liquide monomère (3) et de la poudre de ciment (4) dans l'espace intérieur (11, 61, 111) de la cartouche (1, 51, 101) en une pâte de ciment osseux (49, 149),
D) Extraction de gaz réparti dans la pâte de ciment osseux (49, 149) depuis l'espace intérieur (11, 61, 111) de la cartouche (1, 51, 101) par vissage de l'entrée de monomère (2, 52, 102) avec le piston (12, 62, 112) sur la face frontale de l'entrée de monomère (2, 52, 102) depuis la face arrière de l'espace intérieur (11, 61, 111) de la cartouche (1, 51, 101) dans la cartouche (1, 51, 101) et
E) Pressage de la pâte de ciment osseux (49, 149) mélangée depuis l'espace intérieur (11, 61, 111) de la cartouche (1, 51, 101) par un orifice de sortie de la cartouche (1, 51, 101), opposé au piston (12, 62, 112) par un vissage supplémentaire de l'entrée de monomère (2, 52, 102) dans la cartouche (1, 51, 101).

21. Procédé conformément à la revendication n°20, **caractérisé en ce que**
le procédé est réalisé avec un dispositif conformément à l'une des revendications n°1 à n°19.

22. Procédé conformément à la revendication n°20 ou n°21, **caractérisé en ce que** dans l'étape C), le mélange du liquide monomère (3) et de la poudre de ciment (4) s'opère par une agitation de la cartouche (1, 51, 101), un gaz et au moins un élément de mélange (45, 95, 145) libre et mobile dans l'espace intérieur (11, 61, 111) de la cartouche (1, 51, 101), en particulier au moins une bille (45, 95, 145) libre, sont contenus dans l'espace intérieur (11, 61, 111) de la cartouche (1, 51, 101), en plus de la poudre de ciment (4) et du liquide monomère (3), au moins un élément de mélange (45, 95, 145) étant centrifugé par l'agitation dans l'espace intérieur (11, 61, 111) de la cartouche (1, 51, 101) et un mélange de la poudre de ciment (4) avec le liquide monomère (3) étant ainsi effectué.

23. Procédé conformément à l'une des revendications n°20 à n°22, **caractérisé en ce que**
le système d'ouverture (18, 68, 118) est vissé dans l'entrée de monomère (2, 52, 102), est ouvert par le déplacement du système d'ouverture (18, 68, 118) du réservoir de liquide monomère (5) et qu'au moins un orifice d'injection de gaz (7, 57, 107) qui est disposé dans une paroi de l'entrée de monomère (2, 52, 102), est préalablement obturé.

24. Procédé conformément à l'une des revendications n°20 à n°23, **caractérisé en ce que**
le réservoir de liquide monomère (5) est une ampoule (5) en verre ou en matière plastique et que, lors de l'ouverture de l'ampoule (5) dans l'étape A), l'ampoule (5) est poussée sur un mandrin (34, 84, 134) ou un bord coupant (84, 134) sur la face du piston (12, 62, 112) tournée vers le compartiment (10, 60, 110) de l'entrée de monomère (2, 52, 102) de telle manière à ce que l'ampoule (5) soit ouverte par rupture à cet endroit et que le liquide monomère (3) dans la zone du passage (14, 64, 114) sorte de l'ampoule (5) ouverte.

25. Procédé conformément à l'une des revendications n°20 à n°24, **caractérisé en ce que**,
pendant l'étape B), préférablement pendant les étapes A) et B), l'entrée de monomère (2, 52, 102) est maintenue au-dessus de la cartouche (1, 51, 101) de telle sorte que le liquide monomère (3) entraîné par la pesanteur du compartiment (10, 60, 110) coule dans l'espace intérieur (11, 61, 111) de la cartouche (1, 51, 101) par le passage (14, 64, 114).

26. Procédé conformément à l'une des revendications n°20 à n°25, **caractérisé en ce que**
une étape pendant laquelle un premier élément de sécurité (26, 76, 126) empêchant une utilisation du système d'ouverture (18, 68, 118) est desserré et/ou un deuxième élément de sécurité (47, 97, 147) empêchant un vissage de l'entrée de monomère (2, 52, 102) dans la cartouche (1, 51, 101) est desserré, est réalisée avant l'étape A).

27. Procédé conformément à l'une des revendications n°20 à n°26, **caractérisé en ce que**,
pour le transfert du liquide monomère (3) du compartiment (10, 60, 110) dans l'espace intérieur (11, 61, 111) de la cartouche (1, 51, 101) dans l'étape B), l'entrée de monomère (2, 52, 102) est dévissée de l'espace intérieur (11, 61, 111) de la cartouche (1, 51, 101) de telle sorte que l'espace intérieur (11, 61, 111) de la cartouche (1, 51, 101) s'agrandisse et qu'une dépression par laquelle le liquide monomère (3) est aspiré du compartiment (10, 60, 110) dans l'espace intérieur (11, 61, 111) de la cartouche (1, 51, 101), se forme dans l'espace intérieur (11, 61, 111) de la cartouche (1, 51, 101).
